# EUROPEAN PATENT SPECIFICATION

(11) **EP 1 706 427 B1**
(45) Date of publication and mention of the grant of the patent: **07.12.2011**
(21) Application number: 04804973.8
(22) Date of filing: 21.12.2004
(51) Int. Cl.: C07K 16/28, A61K 39/395

(54) **HUMAN BINDING MOLECULE AGAINST CD1A**
HUMANES BINDUNGSMOLEKÜL GEGEN CD1A
MOLECULE DE LIAISON HUMAINE CONTRE CD1A

(30) Priority: 23.12.2003 WO PCT/EP03/51096; 09.09.2004 WO PCT/EP2004/052110
(43) Date of publication of application: 04.10.2006
(73) Proprietor: Crucell Holland B.V., 2333 CN Leiden (NL); Johns Hopkins University, Baltimore MD 21218 (US)
(72) Inventor: THROSBY, Mark, NL-3581 RB Utrecht (NL); VAN MEIJER, Marja, NL-1066 WS Amsterdam (NL); GERMERAAD, Wilfred Thomas Vincent, NL-6247 JA Gronsveld (NL); ARCECI, Robert John, Baltimore, MD 21212 (US); KRUISBEEK, Ada Maria, NL-1066 EH Amsterdam (NL)
(74) Representative: Verhage, Richard Abraham
(86) International application number: PCT/EP2004/053639
(87) International publication number: WO 2005/063819

(56) References cited:
- WO-A-02/06347
- US-A1- 2001 051 156
- AMIOT M ET AL: "HLA CLASS I MOLECULES ARE ASSOCIATED WITH CD1A HEAVY CHAINS ON NORMAL HUMAN THYMUS CELLS" PROCEEDINGS OF THE NATIONAL ACADEMY OF SCIENCES OF USA, NATIONAL ACADEMY OF SCIENCE. WASHINGTON, US, vol. 85, no. 12, June 1988 (1988-06), pages 4451-4454, XP001146191 ISSN: 0027-8424
- PRITCHARD K K ET AL: "CD1 antibody immunolocalisation in Langerhans' cell histiocytosis." LANCET. 7 AUG 1993, vol. 342, no. 8867, 7 August 1993 (1993-08-07), pages 367-368, XP002340733 ISSN: 0140-6736
- ARCECI ROBERT J ET AL: "Atypical cellular disorders." HEMATOLOGY / THE EDUCATION PROGRAM OF THE AMERICAN SOCIETY OF HEMATOLOGY. AMERICAN SOCIETY OF HEMATOLOGY. EDUCATION PROGRAM. 2002, 2002, pages 297-314, XP002340734 ISSN: 1520-4391
- FURUE M ET AL: "EPITOPES FOR CD1A, CD1B, AND CD1C ANTIGENS ARE DIFFERENTIALLY MAPPED ON LANGERHANS CELLS, DERMAL DENDRITIC CELLS, KERATINOCYTES, AND BASEMENT MEMBRANE ZONE IN HUMAN SKIN" JOURNAL OF THE AMERICAN ACADEMY OF DERMATOLOGY, C.V. MOSBY, ST. LOUIS, MO, US, vol. 27, no. 3, 1992, pages 419-426, XP009007440 ISSN: 0190-9622
- VAUGHAN T J ET AL: "HUMAN ANTIBODIES BY DESIGN" NATURE BIOTECHNOLOGY, NATURE PUB. CO, NEW YORK, NY, US, vol. 16, no. 6, June 1998 (1998-06), pages 535-539, XP000941675 ISSN: 1087-0156
- MOULON C: "A potential role for CD1a molecules on human epidermal Langerhans cells in allogeneic T-cell activation" J INVEST DERMATOL., vol. 97, - September 1991 (1991-09) pages 524-528,
- DEZUTTER-DAMBUYANT C ET AL: "DMC1: a monoclonal antibody produced from histiocytosis X cells which reacts with the native CD1a molecule of human epidermal Langerhans cells" HYBRIDOMA, April 1089 (1089-04), pages 199-208, XP008081981
- MURRAY S ET AL: "Diagnostic and Therapeutic Evaluation of an Anti-Langerhans Cell Histiocytosis Monoclonal Antibody (NA1/34) in a New Xenograft Model" J INVEST DERMATOL., vol. 114, - January 2001 (2001-01) pages 127-134,

### SEQUENCE LISTING

<110> Crucell Holland BV
   The Johns Hopkins University
   Throsby, Mark
   Van Meijer, Marja
   Germeraad, Wilfred T.V.
   Arceci, Robert J.
   Kruisbeek, Ada M.
<120> HUMAN BINDING MOLECULES AGAINST CD1A
<130> 0097 WO 00 ORD
<160> 65
<170> PatentIn version 3.1
<210> 1
   <211> 9
   <212> PRT
   <213> Artificial sequence
<220>
   <223> HCDR3 of SC02 -113
<400> 1
   Ala Pro Tyr Met Met Tyr Phe Asp Ser
   1               5
<210> 2
   <211> 9
   <212> PRT
   <213> Artificial sequence
<220>
   <223> BCDR3 of Sac02-114
<400> 2
   Glu Thr Trp Trp Gln Ser Phe Asp Tyr
   1 5
<210> 3
   <211> 9
   <212> PRT
   <213> Artificial sequence
<220>
   <223> HCDR3 of SC02 -115
   <400> 3
   Ser Gln Met Pro Ser Tyr Phe Asp Tyr
   1 5
<210> 4
   <211> 9
   <212> PRT
   <213> Artificial sequence
<220>
   <223> HCDR3 of SC02 -116
   <400> 4
   Asp Ala Leu Trp Leu Ala Phe Asp Tyr
   1 5
<210> 5
   <211> 9
   <212> PRT
   <213> Artificial sequence
<220>
   <223> HCDR3 of SC02 -117
   <400> 5
   Ser Thr Pro Trp Phe Ser Phe Asp Tyr
   1 5
<210> 6
   <211> 9
   <212> PRT
   <213> Artificial sequence
<220>
   <223> HCDR3 of SC02 -118
   <400> 6
   Ser Ala Trp Trp Leu Ser Phe Asp Ser
   1 5
<210> 7
   <211> 351
   <212> DNA
   <213> Artificial sequence
<220>
   <223> Variable region of heavy chain of 02 -113
   <220>

   <221> CDS
   <222> (1)..(351)
   <223>
<400> 7
<210> 8
   <211> 117
   <212> PRT
   <213> Artificial sequence
<220>
   <223> Variable region of heavy chain of 02 -113
   <400> 8
   Gln Val Gln Leu Gln Glu Ser Gly Ala Gly Leu Leu Lys Pro Ser Glu
   1               5                   10                  15
   Thr Leu Ser Leu Thr Cys Ala Val Tyr Gly Gly Ser Phe Ser Gly Tyr
               20                  25                  30
   Tyr Trp Ser Trp Ile Arg Gln Pro Pro Gly Lys Gly Leu Glu Trp Ile
           35                  40                  45
   Gly Tyr Ile Tyr Tyr Ser Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Lys Ala Pro Tyr Met Met Tyr Phe Asp Ser Trp Gly Gln Gly Thr Leu
               100                 105                 110
   Val Thr Val Ser Ser
           115
<210> 9
   <211> 354
   <212> DNA
   <213> Artificial sequence
<220>
   <223> Variable region of heavy chain of 02 -114
   <220>
   <221> CDS
   <222> (1)..(354)
   <223>
<400> 9
<210> 10
   <211> 118
   <212> PRT
   <213> Artificial sequence
<220>
   <223> Variable region of heavy chain of 02 -114
   <400> 10
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ser Met Asn Trp Val Arg Gln Ala Pro Gly Lys Gly Leu Glu Trp Val
           35                  40                  45
   Ser Tyr Ile Ser Ser Ser Ser Ser Thr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Asp Glu Asp Thr Ala Val Tyr Tyr Cys
                   85                  90                  95
   Ala Lys Glu Thr Trp Trp Gln Ser Phe Asp Tyr Trp Gly Gln Gly Thr
               100                 105                 110
   Leu Val Thr Val Ser Ser
           115
<210> 11
   <211> 354
   <212> DNA
   <213> Artificial sequence
<220>
   <223> Variable region of heavy chain of 02 -115
<220>
   <221> CDS
   <222> (1)..(354)
   <223>
<400> 11
<210> 12
   <211> 118
   <212> PRT
   <213> Artificial sequence
<220>
   <223> Variable region of heavy chain of 02 -115
   <400> 12
   Gln Val Gln Leu Val Gln Ser Gly Ala Glu Ala Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Ser Ala Tyr Asn Gly Asn Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Ser Gln Met Pro Ser Tyr Phe Asp Tyr Trp Gly Gln Gly Thr
               100                 105                 110
   Leu Val Thr Val Ser Ser
           115
<210> 13
   <211> 354
   <212> DNA
   <213> Artificial sequence
<220>
   <223> Variable region of heavy chain of 02 -116
   <220>
   <221> CDS
   <222> (1)..(354)
   <223>
<400> 13 <210> 14
   <211> 118
   <212> PRT
   <213> Artificial sequence
<220>
   <223> Variable region of heavy chain of 02 -116
   <400> 14
<210> 15
   <211> 351
   <212> DNA
   <213> Artificial sequence
<220>
   <223> Variable region of heavy chain of 02 -117
   <220>

   <221> CDS
   <222> (1)..(351)
   <223>
<400> 15
   gag gtg cag ctg gtg gag tct ggg gga ggc ttg gta cat cct ggg ggg      48
   Glu Val Gln Leu Val Glu Ser Gly Gly Gly Leu Val His Pro Gly Gly
   1               5                   10                  15
   tcc ctg aga ctc tcc tgt gca ggc tct gga ttc acc ttc agt agc tat      96
   Ser Leu Arg Leu Ser Cys Ala Gly Ser Gly Phe Thr Phe Ser Ser Tyr
               20                  25                  30
   gct atg cac tgg gtt cgc cag gct cca gga aaa ggt ctg gag tgg gta     144
   Ala Met His Trp Val Arg Gln Ala Pro Gly Lys Gly Leu Glu Trp Val
           35                  40                  45
   tca gct att ggt act ggt ggt ggc aca tac tat gca gac tcc gtg aag     192
   Ser Ala Ile Gly Thr Gly Gly Gly Thr Tyr Tyr Ala Asp Ser Val Lys
       50                  55                  60
   ggc cga ttc acc atc tcc aga gac aat gcc aag aac tcc ttg tat ctt     240
   Gly Arg Phe Thr Ile Ser Arg Asp Asn Ala Lys Asn Ser Leu Tyr Leu
   65                  70                  75                  80
   caa atg aac agc ctg aga gcc gag gac acg gcc gtg tat tac tgt gca     288
   Gln Met Asn Ser Leu Arg Ala Glu Asp Thr Ala Val Tyr Tyr Cys Ala
                   85                  90                  95
   agg tct acg cct tgg ttt tcc ttt gac tac tgg ggc cag ggc acc ctg     336
   Arg Ser Thr Pro Trp Phe Ser Phe Asp Tyr Trp Gly Gln Gly Thr Leu
               100                 105                 110
   gtg acc gtc tcc agc                                                 351
   Val Thr Val Ser Ser
           115
<210> 16
   <211> 117
   <212> PRT
   <213> Artificial sequence
<220>
   <223> Variable region of heavy chain of 02 -117
   <400> 16
   Glu Val Gln Leu Val Glu Ser Gly Gly Gly Leu Val His Pro Gly Gly
   1               5                   10                  15
   Ser Leu Arg Leu Ser Cys Ala Gly Ser Gly Phe Thr Phe Ser Ser Tyr
               20                  25                  30
   Ala Met His Trp Val Arg Gln Ala Pro Gly Lys Gly Leu Glu Trp Val
           35                  40                  45
   Ser Ala Ile Gly Thr Gly Gly Gly Thr Tyr Tyr Ala Asp Ser Val Lys
       50                  55                  60
   Gly Arg Phe Thr Ile Ser Arg Asp Asn Ala Lys Asn Ser Leu Tyr Leu
   65                  70                  75                  80
   Gln Met Asn Ser Leu Arg Ala Glu Asp Thr Ala Val Tyr Tyr Cys Ala
                   85                  90                  95
   Arg Ser Thr Pro Trp Phe Ser Phe Asp Tyr Trp Gly Gln Gly Thr Leu
               100                 105                 110
   Val Thr Val Ser Ser
           115
<210> 17
   <211> 351
   <212> DNA
   <213> Artificial sequence
<220>
   <223> Variable region of heavy chain of 02 -118
   <220>
   <221> CDS
   <222> (1)..(351)
   <223>
<400> 17
<210> 18
   <211> 117
   <212> PRT
   <213> Artificial sequence
<220>
   <223> Variable region of heavy chain of 02 -118
   <400> 18
<210> 19
   <211> 330
   <212> DNA
   <213> Artificial sequence
<220>
   <223> Variable region of light chain of 02 -113, 02-114, 02-116, 02-117 and 02 -118
<220>
   <221> CDS
   <222> (1)..(330)
   <223>
<400> 19
   gac att cag atg acc cag tct cca tcc tcc ctg tct gca tct gta gga      48
   Asp Ile Gln Met Thr Gln Ser Pro Ser Ser Leu Ser Ala Ser Val Gly
   1               5                   10                  15
   gac aga gtc acc atc act tgc cgg gca agt cag agc att agc agc tac      96
   Asp Arg Val Thr Ile Thr Cys Arg Ala Ser Gln Ser Ile Ser Ser Tyr
               20                  25                  30
   tta aat tgg tat cag cag aaa cca ggg aaa gcc cct aag ctc ctg atc       144
   Leu Asn Trp Tyr Gln Gln Lys Pro Gly Lys Ala Pro Lys Leu Leu Ile
           35                  40                  45
   tat gct gca tcc agt ttg caa agt ggg gtc cca tca agg ttc agt ggc       192
   Tyr Ala Ala Ser Ser Leu Gln Ser Gly Val Pro Ser Arg Phe Ser Gly
       50                  55                  60
   agt gga tct ggg aca gat ttc act ctc acc atc agc agt ctg caa cct     240
   Ser Gly Ser Gly Thr Asp Phe Thr Leu Thr Ile Ser Ser Leu Gln Pro
   65                  70                  75                  80
   gaa gat ttt gca act tac tac tgt caa cag agt tac agt acc cct cca     288
   Glu Asp Phe Ala Thr Tyr Tyr Cys Gln Gln Ser Tyr Ser Thr Pro Pro
                   85                  90                  95
   acg ttc ggc caa ggg acc aag gtg gag atc aaa cgg acc gtg             330
   Thr Phe Gly Gln Gly Thr Lys Val Glu Ile Lys Arg Thr Val
               100                 105                 110
<210> 20
   <211> 110
   <212> PRT
   <213> Artificial sequence
<220>
   <223> Variable region of light chain of 02 -113, 02-114, 02-116, 02-117 and 02-118
   <400> 20
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Pro
                   85                  90                  95
   Thr Phe Gly Gln Gly Thr Lys Val Glu Ile Lys Arg Thr Val
               100                 105                 110
<210> 21
   <211> 354
   <212> DNA
   <213> Artificial sequence
<220>
   <223> Variable region of light chain of 02 -115
   <220>
   <221> CDS
   <222> (1)..(354)
   <223>
<400> 21
<210> 22
   <211> 118
   <212> PRT
   <213> Artificial sequence
<220>
   <223> Variable region of light chain of 02 -115
   <400> 22
<210> 23
   <211> 16
   <212> DNA
   <213> Artificial sequence
<220>
   <223> Primer M13rev
<400> 23
   aacagctatg accatg 16
<210> 24
   <211> 17
   <212> DNA
   <213> Artificial sequence
<220>
   <223> Primer fdSeq
   <400> 24
   gaattttctg tatgagg 17
<210> 25
   <211> 720
   <212> DNA
   <213> Artificial sequence
<220>
   <223> Sequence of scFv SC02 -113
   <220>
   <221> CDS
   <222> (1)..(720)
   <223>
<400> 25
<210> 26
   <211> 240
   <212> PRT
   <213> Artificial sequence
<220>
   <223> Sequence of scFv SC02 -113
   <400> 26
   Gln Val Gln Leu Gln Glu Ser Gly Ala Gly Leu Leu Lys Pro Ser Glu
   1 5 10 15
   Thr Leu Ser Leu Thr Cys Ala Val Tyr Gly Gly Ser Phe Ser Gly Tyr
   20 25 30
   Tyr Trp Ser Trp Ile Arg Gln Pro Pro Gly Lys Gly Leu Glu Trp Ile
   35 40 45
   Gly Tyr Ile Tyr Tyr Ser Gly Ser Thr Asn Tyr Asn Pro Ser Leu Lys
   50 55 60
   Ser Arg Val Thr Ile Ser Val Asp Thr Ser Lys Asn Gln Phe Ser Leu
   65 70 75 80
   Lys Leu Ser Ser Val Thr Ala Ala Asp Thr Ala Val Tyr Tyr Cys Ala
   85 90 95
   Lys Ala Pro Tyr Met Met Tyr Phe Asp Ser Trp Gly Gln Gly Thr Leu
   100 105 110
   Val Thr Val Ser Ser Gly Gly Gly Gly Ser Gly Gly Gly Gly Ser Gly
   115 120 125
   Gly Gly Gly Ser Glu Ile Glu Leu Thr Gln Ser Pro Ser Ser Leu Ser
   130 135 140
   Ala Ser Val Gly Asp Arg Val Thr Ile Thr Cys Arg Ala Ser Gln Ser
   145 150 155 160
   Ile Ser Ser Tyr Leu Asn Trp Tyr Gln Gln Lys Pro Gly Lys Ala Pro
   165 170 175
   Lys Leu Leu Ile Tyr Ala Ala Ser Ser Leu Gln Ser Gly Val Pro Ser
   180 185 190
   Arg Phe Ser Gly Ser Gly Ser Gly Thr Asp Phe Thr Leu Thr Ile Ser
   195 200 205
   Ser Leu Gln Pro Glu Asp Phe Ala Thr Tyr Tyr Cys Gln Gln Ser Tyr
   210 215 220
   Ser Thr Pro Pro Thr Phe Gly Gln Gly Thr Lys Val Glu Ile Lys Arg
   225 230 235 240
<210> 27
   <211> 723
<212> DNA
   <213> Artificial sequence
<220>
   <223> Sequence of scFv SC02 -114
   <220>
   <221> CDS
   <222> (1)..(723)
   <223>
<400> 27
   gag gtg cag ctg gtg gag tct ggg gga ggc ttg gta  cag cct ggg ggg      48
   Glu Val Gln Leu Val Glu Ser Gly Gly Gly Leu Val Gln Pro Gly Gly
   1               5                   10                  15
   tcc ctg aga ctc tcc tgt gca gcc tct gga ttc acc ttc agt agc tat      96
   Ser Leu Arg Leu Ser Cys Ala Ala Ser Gly Phe Thr Phe Ser Ser Tyr
               20                  25                  30
   agc atg aac tgg gtc cgc cag gct cca ggg aag ggg ctg gag tgg gtt      144
   Ser Met Asn Trp Val Arg Gln Ala Pro Gly Lys Gly Leu Glu Trp Val
           35                  40                  45
   tca tac att agt agt agt agt agt acc ata tac tac gca gac tct gtg 192
   Ser Tyr Ile Ser Ser Ser Ser Ser Thr Ile Tyr Tyr Ala Asp Ser Val
   50 55 60
   aag ggc cga ttc acc atc tcc aga gac aat gcc aag aac tca ctg tat      240
   Lys Gly Arg Phe Thr Ile Ser Arg Asp Asn Ala Lys Asn Ser Leu Tyr
   65                  70                  75                  80
   ctg caa atg aac agc ctg aga gac gag gac acg gcc gtg tat tac tgt      288
   Leu Gln Met Asn Ser Leu Arg Asp Glu Asp Thr Ala Val Tyr Tyr Cys
                   85                  90                  95
   gca aag gag act tgg tgg cag tcc ttt gac tac tgg ggc caa ggt acc      336
   Ala Lys Glu Thr Trp Trp Gln Ser Phe Asp Tyr Trp Gly Gln Gly Thr
               100                 105                 110
   ctg gtc acc gtc tcg agt ggt gga ggc ggt tca  ggc gga ggt ggc tct      384
   Leu Val Thr Val Ser Ser Gly Gly Gly Gly Ser Gly Gly Gly Gly Ser
           115                 120                 125
   ggc ggt ggc gga tcg gaa att gag ctc acc cag tct cca tcc tcc ctg      432
   Gly Gly Gly Gly Ser Glu Ile Glu Leu Thr Gln Ser Pro Ser Ser Leu
       130                 135                 140
   tct gca tct gta gga gac aga gtc acc atc act tgc cgg gca agt cag      480
   Ser Ala Ser Val Gly Asp Arg Val Thr Ile Thr Cys Arg Ala Ser Gln
   145                 150                 155                 160
   agc att agc agc tac tta aat tgg tat cag cag aaa cca ggg aaa gcc      528
   Ser Ile Ser Ser Tyr Leu Asn Trp Tyr Gln Gln Lys Pro Gly Lys Ala
                   165                 170                 175
   cct aag ctc ctg atc tat gct gca tcc agt ttg caa agt ggg gtc cca      576
   Pro Lys Leu Leu Ile Tyr Ala Ala Ser Ser Leu Gln Ser Gly Val Pro
               180                 185                 190
   tca agg ttc agt ggc agt gga tct ggg aca gat ttc act ctc acc atc      624
   Ser Arg Phe Ser Gly Ser Gly Ser Gly Thr Asp Phe Thr Leu Thr Ile
           195                 200                 205
   agc agt ctg caa cct gaa gat ttt gca act tac tac tgt caa cag agt      672
   Ser Ser Leu Gln Pro Glu Asp Phe Ala Thr Tyr Tyr Cys Gln Gln Ser
       210                 215                 220
   tac agt acc cct cca acg ttc ggc caa ggg  acc aag gtg gag atc aaa      720
   Tyr Ser Thr Pro Pro Thr Phe Gly Gln Gly Thr Lys Val Glu Ile Lys
   225                 230                 235                 240
   cgt                                                                   723
   Arg
<210> 28
   <211> 241
   <212> PRT
   <213> Artificial sequence
<220>
   <223> Sequence of scfv iSC02 -114
   <400> 28
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ser Met Asn Trp Val Arg Gln Ala Pro Gly Lys Gly Leu Glu Trp Val
           35                  40                  45
   Ser Tyr Ile Ser Ser Ser Ser Ser Thr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Asp Glu Asp Thr Ala Val Tyr Tyr Cys
                   85                  90                  95
   Ala Lys Glu Thr Trp Trp Gln Ser Phe Asp Tyr Trp Gly Gln Gly Thr
               100                 105                 110
   Leu Val Thr Val Ser Ser Gly Gly Gly Gly Ser Gly Gly Gly Gly Ser
           115                 120                 125
   Gly Gly Gly Gly Ser Glu Ile Glu Leu Thr Gln Ser Pro Ser Ser Leu
       130                 135                 140
   Ser Ala Ser Val Gly Asp Arg Val Thr Ile Thr Cys Arg Ala Ser Gln
   145                 150                 155                 160
   Ser Ile Ser Ser Tyr Leu Asn Trp Tyr Gln Gln Lys Pro Gly Lys Ala
                   165                 170                 175
   Pro Lys Leu Leu Ile Tyr Ala Ala Ser Ser Leu Gln Ser Gly Val Pro
               180                 185                 190
   Ser Arg Phe Ser Gly Ser Gly Ser Gly Thr Asp Phe Thr Leu Thr Ile
           195                 200                 205
   Ser Ser Leu Gln Pro Glu Asp Phe Ala Thr Tyr Tyr Cys Gln Gln Ser
       210                 215                 220
   Tyr Ser Thr Pro Pro Thr Phe Gly Gln Gly Thr Lys Val Glu Ile Lys
   225                 230                 235                 240
   Arg
<210> 29
   <211> 723
   <212> DNA
   <213> Artificial sequence
<220>
   <223> Sequence of scFv SC02 -115
   <400> 29
<210> 30
   <211> 241
   <212> PRT
   <213> Artificial sequence
<220>
   <223> Sequence of scFv SC02 -115
   <400> 30
   Gln Val Gln Leu Val Gln Ser Gly Ala Glu Ala Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Ser Ala Tyr Asn Gly Asn Thr Asn Tyr Ala Gln Lys Leu
   50 55 60
   Gln Gly Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Ser Gln Met Pro Ser Tyr Phe Asp Tyr Trp Gly Gln Gly Thr
   100 105 110
   Leu Val Thr Val Ser Arg Gly Gly Gly Gly Ser Gly Gly Gly Gly Ser
           115                 120                 125
   Gly Gly Gly Gly Ser Ser Glu Leu Thr Gln Asp Pro Ala Val Ser Val
       130                 135                 140
   Ala Leu Gly Gln Thr Val Arg Ile Thr Cys Gln Gly Asp Ser Leu Arg
   145                 150                 155                 160
   Ser Tyr Tyr Ala Ser Trp Tyr Gln Gln Lys Pro Gly Gln Ala Pro Val
                   165                 170                 175
   Leu Val Ile Tyr Gly Lys Asn Asn Arg Pro Ser Gly Ile Pro Asp Arg
               180                 185                 190
   Phe Ser Gly Ser Ser Ser Gly Asn Thr Ala Ser Leu Thr Ile Thr Gly
           195                 200                 205
   Ala Gln Ala Glu Asp Glu Ala Asp Tyr Tyr Cys Asn Ser Arg Asp Ser
       210                 215                 220
   Ser Gly Asn His Val Val Phe Gly Gly Gly Thr Lys Leu Thr Val Leu
   225                 230                 235                 240
   Gly
<210> 31
   <211> 723
   <212> DNA
   <213> Artificial sequence
<220>
   <223> Sequence of scFv SC02 -116
   <220>

   <221> CDS
   <222> (1)..(723)
   <223>
<400> 31
   gag gtg cag ctg gtg gag tct ggg gga ggc ttg gta cag cct ggg ggg      48
   Glu Val Gln Leu Val Glu Ser Gly Gly Gly Leu Val Gln Pro Gly Gly
   1 5 10 15
   tcc ctg aga ctc tcc tgt gca gcc tct gga ttc acc ttt agc agc tat 96
   Ser Leu Arg Leu Ser Cys Ala Ala Ser Gly Phe Thr Phe Ser Ser Tyr
   20 25 30
   gcc atg agc tgg gtc cgc cag gct cca ggg aag ggg ctg gag tgg gtc 144
   Ala Met Ser Trp Val Arg Gln Ala Pro Gly Lys Gly Leu Glu Trp Val
   35 40 45
   tca gct att agt ggt agt ggt ggt agc aca tac tac gca gac tcc gtg 192
   Ser Ala Ile Ser Gly Ser Gly Gly Ser Thr Tyr Tyr Ala Asp Ser Val
   50 55 60
   aag ggc cgg ttc acc atc tcc aga gac aat tcc aag aac acg ctg tat 240
   Lys Gly Arg Phe Thr Ile Ser Arg Asp Asn Ser Lys Asn Thr Leu Tyr
   65 70 75 80
   ctg caa atg aac agc cta aga gcc gag gac acg gcc gtg tat tac tgt 288
   Leu Gln Met Asn Ser Leu Arg Ala Glu Asp Thr Ala Val Tyr Tyr Cys
   85 90 95
   gca aag gac gct ctt tgg ctg gct ttt gac tac tgg ggc caa ggt acc 336
   Ala Lys Asp Ala Leu Trp Leu Ala Phe Asp Tyr Trp Gly Gln Gly Thr
   100 105 110
   ctg gtc acc gtc tcg agt ggt gga ggc ggt tca ggc gga ggt ggc tct 384
   Leu Val Thr Val Ser Ser Gly Gly Gly Gly Ser Gly Gly Gly Gly Ser
   115 120 125
   ggc ggt ggc gga tcg gaa att gag ctc acc cag tct cca tcc tcc ctg 432
   Gly Gly Gly Gly Ser Glu Ile Glu Leu Thr Gln Ser Pro Ser Ser Leu
   130 135 140
   tct gca tct gta gga gac aga gtc acc atc act tgc cgg gca agt cag 480
   Ser Ala Ser Val Gly Asp Arg Val Thr Ile Thr Cys Arg Ala Ser Gln
   145 150 155 160
   agc att agc agc tat tta aat tgg tat cag cag aaa cca ggg aaa gcc 528
   Ser Ile Ser Ser Tyr Leu Asn Trp Tyr Gln Gln Lys Pro Gly Lys Ala
   165 170 175
   cct aag ctc ctg atc tat gct gca tcc agt ttg caa agt ggg gtc cca 576
   Pro Lys Leu Leu Ile Tyr Ala Ala Ser Ser Leu Gln Ser Gly Val Pro
   180 185 190
   tca agg ttc agt ggc agt gga tct ggg aca gat ttc act ctc acc atc 624
   Ser Arg Phe Ser Gly Ser Gly Ser Gly Thr Asp Phe Thr Leu Thr Ile
   195 200 205
   agc agt ctg caa cct gaa gat ttt gca act tac tac tgt gct cag agg 672
   Ser Ser Leu Gln Pro Glu Asp Phe Ala Thr Tyr Tyr Cys Ala Gln Arg
   210 215 220
   agt tat ccg cct cct aag ttc ggc caa ggg acc aag gtg gat atc aaa 720
   Ser Tyr Pro Pro Pro Lys Phe Gly Gln Gly Thr Lys Val Asp Ile Lys
   225 230 235 240
   cgt 723
   Arg
<210> 32
   <211> 241
   <212> PRT
   <213> Artificial sequence
<220>
   <223> Sequence of scFv SC02 -116
   <400> 32
<210> 33
   <211> 720
   <212> DNA
   <213> Artificial sequence
<220>
   <223> Sequence of scFv SC02 -117
   <220>
   <221> CDS
   <222> (1)..(720)
   <223>
<400> 33
   gag gtg cag ctg gtg gag tct ggg gga ggc ttg gta cat cct ggg ggg      48
   Glu Val Gln Leu Val Glu Ser Gly Gly Gly Leu  Val His Pro Gly Gly
   1               5                   10                  15
   tcc ctg aga ctc tcc tgt gca ggc tct gga ttc acc ttc agt agc tat      96
   Ser Leu Arg Leu Ser Cys Ala Gly Ser Gly Phe Thr Phe Ser Ser Tyr
               20                  25                  30
   gct atg cac tgg gtt cgc cag gct cca gga aaa ggt ctg gag tgg gta     144
   Ala Met His Trp Val Arg Gln Ala Pro Gly Lys Gly Leu Glu Trp Val
           35                  40                  45
   tca gct att ggt act ggt ggt ggc aca tac tat gca gac tcc gtg aag     192
   Ser Ala Ile Gly Thr Gly Gly Gly Thr Tyr Tyr Ala Asp Ser Val Lys
       50                  55                  60
   ggc cga ttc acc atc tcc aga gac aat gcc aag aac tcc ttg tat ctt     240
   Gly Arg Phe Thr Ile Ser Arg Asp Asn Ala Lys Asn Ser Leu Tyr Leu
   65                  70                  75                  80
   caa atg aac agc ctg aga gcc gag gac acg g cc gtg tat tac tgt gca     288
   Gln Met Asn Ser Leu Arg Ala Glu Asp Thr Ala Val Tyr Tyr Cys Ala
                   85                  90                  95
   agg tct acg cct tgg ttt tcc ttt gac tac tgg ggc caa ggt acc ctg     336
   Arg Ser Thr Pro Trp Phe Ser Phe Asp Tyr Trp Gly Gln Gly Thr Leu
               100                 105                 110
   gtc acc gtc tcg agt ggt gga ggc ggt tca ggc gga ggt ggc tct ggc     384
   Val Thr Val Ser Ser Gly Gly Gly Gly Ser  Gly Gly Gly Gly Ser Gly
           115                 120                 125
   ggt ggc gga tcg gaa att gag ctc acc cag tct cca tcc tcc ctg tct     432
   Gly Gly Gly Ser Glu Ile Glu Leu Thr Gln Ser Pro Ser Ser Leu Ser
       130                 135                 140
   gca tct gta gga gac aga gtc acc atc act tgc cgg gca agt cag agc     480
   Ala Ser Val Gly Asp Arg Val Thr Ile Thr Cys Arg Ala Ser Gln Ser
   145                 150                 155                 160
   att agc agc tac tta aat tgg tat cag cag aaa cca ggg aaa gcc cct     528
   Ile Ser Ser Tyr Leu Asn Trp Tyr Gln Gln Lys Pro Gly Lys Ala Pro
                   165                 170                 175
   aag ctc ctg atc tat gct gca tcc agt ttg caa agt ggg gtc cca tca     576
   Lys Leu Leu Ile Tyr Ala Ala Ser Ser Leu Gln Ser Gly Val Pro Ser
               180                 185                 190
   agg ttc agt ggc agt gga tct ggg aca g at ttc act ctc acc atc agc     624
   Arg Phe Ser Gly Ser Gly Ser Gly Thr Asp Phe Thr Leu Thr Ile Ser
           195                 200                 205
   agt ctg caa cct gaa gat ttt gca act tac tac tgt caa cag agt tac     672
   Ser Leu Gln Pro Glu Asp Phe Ala Thr Tyr Tyr Cys Gln Gln Ser Tyr
       210                 215                 220
   agt acc cct cca acg ttc ggc caa ggg acc aag gtg gag atc aaa cgt     720
   Ser Thr Pro Pro Thr Phe Gly Gln Gly  Thr Lys Val Glu Ile Lys Arg
   225                 230                 235                 240
<210> 34
   <211> 240
   <212> PRT
   <213> Artificial sequence
<220>
   <223> Sequence of scFv SC02 -117
   <400> 34
   Glu Val Gln Leu Val Glu Ser Gly Gly Gly Leu Val His Pro Gly Gly
   1 5 10 15
   Ser Leu Arg Leu Ser Cys Ala Gly Ser Gly Phe Thr Phe Ser Ser Tyr
   20 25 30
   Ala Met His Trp Val Arg Gln Ala Pro Gly Lys Gly Leu Glu Trp Val
   35 40 45
   Ser Ala Ile Gly Thr Gly Gly Gly Thr Tyr Tyr Ala Asp Ser Val Lys
   50 55 60
   Gly Arg Phe Thr Ile Ser Arg Asp Asn Ala Lys Asn Ser Leu Tyr Leu
   65 70 75 80
   Gln Met Asn Ser Leu Arg Ala Glu Asp Thr Ala Val Tyr Tyr Cys Ala
   85 90 95
   Arg Ser Thr Pro Trp Phe Ser Phe Asp Tyr Trp Gly Gln Gly Thr Leu
   100 105 110
   Val Thr Val Ser Ser Gly Gly Gly Gly Ser Gly Gly Gly Gly Ser Gly
   115 120 125
   Gly Gly Gly Ser Glu Ile Glu Leu Thr Gln Ser Pro Ser Ser Leu Ser
   130 135 140
   Ala Ser Val Gly Asp Arg Val Thr Ile Thr Cys Arg Ala Ser Gln Ser
   145 150 155 160
   Ile Ser Ser Tyr Leu Asn Trp Tyr Gln Gln Lys Pro Gly Lys Ala Pro
   165 170 175
   Lys Leu Leu Ile Tyr Ala Ala Ser Ser Leu Gln Ser Gly Val Pro Ser
   180 185 190
   Arg Phe Ser Gly Ser Gly Ser Gly Thr Asp Phe Thr Leu Thr Ile Ser
   195 200 205
   Ser Leu Gln Pro Glu Asp Phe Ala Thr Tyr Tyr Cys Gln Gln Ser Tyr
   210 215 220
   Ser Thr Pro Pro Thr Phe Gly Gln Gly Thr Lys Val Glu Ile Lys Arg
   225 230 235 240
<210> 35
   <211> 720
   <212> DNA
   <213> Artificial sequence
<220>
   <223> Sequence of scFv SC02 -118
   <400> 35
<210> 36
   <211> 240
   <212> PRT
   <213> Artificial sequence
<220>
   <223> Sequence of scFv SC02 -118
   <400> 3 6
<210> 37
   <211> 6778
   <212> DNA
   <213> Artificial sequence
<220>
   <223> pSyn-C03-HCgamma1
   <400> 37
<210> 38
   <211> 6267
   <212> DNA
   <213> Artificial sequence
<220>
   <223> pSyn -C05-Cκappa
   <400> 38
<210> 39
   <211> 6283
   <212> DNA
   <213> Artificial sequence
<220>
   <223> pSyn-C04-Clambda
   <400> 39
<210> 40
   <211> 54
   <212> DNA
   <213> Artificial sequence
<220>
   <223> Oligonucleotide 5k-I
   <400> 40
   acctgtctcg agtttccat ggctgacatc cagatgaccc ag tctccatc ctcc 54
<210> 41
   <211> 42
   <212> DNA
   <213> Artificial sequence
<220>
   <223> Oligonucleotide sy3K -C
   <400> 41
   gggaccaagg tggagatcaa acggaccgtg gccgccccca gc 42
<210> 42
   <211> 52
   <212> DNA
   <213> Artificial sequence
<220>
   <223> Oligonucleotide sy5L-A
   <400> 42
   acctgtctcg agttttccat ggcttcctcc gagctgaccc aggaccctgc tg 52
<210> 43
   <211> 44
   <212> DNA
   <213> Artificial sequence
<220>
   <223> Oligonucleotide 3L -B
<400> 43
   ttttccttag cggccgcgac tcacctagga cggtcagctt ggtc 44
<210> 44
   <211> 49
   <212> DNA
   <213> Artificial sequence
<220>
   <223> Oligonucleotide 5H -Fshort
   <400> 44
   acctgtcttg aattctccat ggcccaggtg cagctgcagg agtccggcc 49
<210> 45
   <211> 47
   <212> DNA
   <213> Artificial sequence
<220>
   <223> Oligonucleotide sy3H -A
   <400> 45
   gcccttggtg ctagcgctgg agacggtcac cagggtgccc tggcccc 47
<210> 46
   <211> 46
   <212> DNA
   <213> Artificial sequenc e
<220>
   <223> Oligonucleotide 5H -B
   <400> 46
   acctgtcttg aattctccat ggccgaggtg cagctggtgg agtctg 46
<210> 47
   <211> 65
   <212> DNA
   <213> Artificial sequence
<220>
   <223> oligonucleotide 5H -B**65
   <400> 47
   acctgtcttg aattctccat ggccgaggtg cagctggtgg agtctggggg aggcttggta 60
   catcc 65
<210> 48
   <211> 47
   <212> DNA
   <213> Artificial sequence
<220>
   <223> Oligonucleotide 5H-A
   <400> 48
   acctgtcttg aattctccat ggcccaggtg cagctggtgc agtctgg 47
<210> 49
   <211> 68
   <212> DNA
   <213> Artificial sequence
<220>
   <223> Oligonucleotide 115int68
   <400> 49
   caccagggtg ccctggcccc agtagtcaaa gtaactcggc atctgcgacc ttgcacagta 60
   atacacgg 68
<210> 50
   <211> 1341
   <212> DNA
   <213> Artificial sequence
<220>
   <223> Heavy chain of 02 -113
   <220>
   <221> CDS
   <222> (1)..(1341)
   <223>
<400> 50
   cag gtg cag ctg cag gag tcc ggc gca gga ctg ttg aag cct tcg gag      48
   Gln Val Gln Leu Gln Glu Ser Gly Ala Gly Leu Leu Lys Pro Ser Glu
   1               5                   10                  15
   acc ctg tcc ctc acc tgc gct gtc tat ggt ggg tct ttc agt ggc tac      96
   Thr Leu Ser Leu Thr Cys Ala Val Tyr Gly Gly Ser Phe Ser Gly Tyr
               20                  25                  30
   tac tgg agc tgg atc cgg cag ccc cca ggg aag gga ctg gag tgg att      144
   Tyr Trp Ser Trp Ile Arg Gln Pro Pro Gly Lys Gly Leu Glu Trp Ile
           35                  40                  45
   ggg tat atc tat tac agt ggg agc acc aac tac aac ccc tcc ctc aag      192
   Gly Tyr Ile Tyr Tyr Ser Gly Ser Thr Asn Tyr Asn Pro Ser Leu Lys
       50                  55                  60
   agt cga gtc acc ata tca gta gac acg tcc aag aac cag ttc tcc ctg      240
   Ser Arg Val Thr Ile Ser Val Asp Thr Ser Lys Asn Gln Phe Ser Leu
   65                  70                  75                  80
   aag ctg agc tct gtg acc gct gcg gac acg gcc gtg tat tac tgt gca      288
   Lys Leu Ser Ser Val Thr Ala Ala Asp Thr Ala Val Tyr Tyr Cys Ala
                   85                  90                  95
   aag gcc cct tat atg atg tat ttt gac tcc tgg ggc cag ggc acc ctg      336
   Lys Ala Pro Tyr Met Met Tyr Phe Asp Ser Trp Gly Gln Gly Thr Leu
               100                 105                 110
   gtg acc gtc tcc agc gct agc acc aag ggc ccc agc gtg ttc ccc ctg      384
   Val Thr Val Ser Ser Ala Ser Thr Lys Gly Pro Ser Val Phe Pro Leu
           115                 120                 125
   gcc ccc agc agc aag agc acc agc ggc ggc aca gcc gcc ctg ggc tgc      432
   Ala Pro Ser Ser Lys Ser Thr Ser Gly Gly Thr Ala Ala Leu Gly Cys
       130                 135                 140
   ctg gtg aag gac tac ttc ccc gag ccc gtg acc gtg agc tgg aac agc      480
   Leu Val Lys Asp Tyr Phe Pro Glu Pro Val Thr Val Ser Trp Asn Ser
   145                 150                 155                 160
   ggc gcc ttg acc agc ggc gtg cac acc ttc ccc gcc gtg ctg cag agc      528
   Gly Ala Leu Thr Ser Gly Val His Thr Phe Pro Ala Val Leu Gln Ser
                   165                 170                 175
   agc ggc ctg tac agc ctg agc agc gtg gtg acc gtg ccc agc agc agc      576
   Ser Gly Leu Tyr Ser Leu Ser Ser Val Val Thr Val Pro Ser Ser Ser
               180                 185                 190
   ctg ggc acc cag acc tac atc tgc aac gtg aac cac aag ccc agc aac      624
   Leu Gly Thr Gln Thr Tyr Ile Cys Asn Val Asn His Lys Pro Ser Asn
           195                 200                 205
   acc aag gtg gac aaa cgc gtg gag ccc aag agc tgc gac aag acc cac      672
   Thr Lys Val Asp Lys Arg Val Glu Pro Lys Ser Cys Asp Lys Thr His
       210                 215                 220
   acc tgc ccc ccc tgc cct gcc ccc gag ctg ctg ggc gga ccc tcc gtg      720
   Thr Cys Pro Pro Cys Pro Ala Pro Glu Leu Leu Gly Gly Pro Ser Val
   225                 230                 235                 240
   ttc ctg ttc ccc ccc aag ccc aag gac acc ctc atg atc  agc cgg acc      768
   Phe Leu Phe Pro Pro Lys Pro Lys Asp Thr Leu Met Ile Ser Arg Thr
                   245                 250                 255
   ccc gag gtg acc tgc gtg gtg gtg gac gtg agc cac gag gac ccc gag      816
   Pro Glu Val Thr  Cys Val Val Val Asp Val Ser His Glu Asp Pro Glu
               260                 265                 270
   gtg aag ttc aac tgg tac gtg gac ggc gtg gag gtg cac aac gcc aag      864
   Val Lys Phe Asn Trp Tyr Val Asp Gly Val Glu Val His  Asn Ala Lys
           275                 280                 285
   acc aag ccc cgg gag gag cag tac aac agc acc tac cgg gtg gtg agc      912
   Thr Lys Pro Arg Glu Glu Gln Tyr Asn Ser Thr Tyr Arg Val Val Ser
       290                 295                 300
   gtg ctc acc gtg ctg cac cag gac tgg ctg aac ggc aag gag tac aag      960
   Val Leu Thr Val Leu His Gln Asp Trp Leu Asn Gly Lys Glu Tyr Lys
   305                 310                 315                 320
   tgc aag gtg agc aac aag gcc ctg cct gcc ccc atc gag aag acc atc     1008
   Cys Lys Val Ser Asn Lys Ala Leu Pro Ala Pro Ile Glu Lys Thr Ile
                   325                 330                 335
   agc aag gcc  aag ggc cag ccc cgg gag ccc cag gtg tac acc ctg ccc    1056
   Ser Lys Ala Lys Gly Gln Pro Arg Glu Pro Gln Val Tyr Thr Leu Pro
               340                 345                 350
   ccc agc cgg gag gag atg acc aag aac cag gtg tcc  ctc acc tgt ctg    1104
   Pro Ser Arg Glu Glu Met Thr Lys Asn Gln Val Ser Leu Thr Cys Leu
           355                 360                 365
   gtg aag ggc ttc tac ccc agc gac atc gcc gtg gag tgg gag agc aac     1152
   Val Lys Gly Phe Tyr Pro Ser Asp Ile Ala Val Glu Trp Glu Ser Asn
       370                 375                 380
   ggc cag ccc gag aac aac tac aag acc acc ccc cct gtg ctg gac agc     1200
   Gly Gln Pro Glu Asn Asn Tyr Lys Thr Thr Pro Pro  Val Leu Asp Ser
   385                 390                 395                 400
   gac ggc agc ttc ttc ctg tac agc aag ctc acc gtg gac aag agc cgg     1248
   Asp Gly Ser Phe Phe Leu Tyr Ser Lys Leu Thr Val Asp Lys Ser Arg
                   405                 410                 415
   tgg cag cag ggc aac gtg ttc agc tgc agc gtg atg cac gag gcc ctg     1296
   Trp Gln Gln Gly Asn Val Phe Ser Cys Ser Val Met His Glu Ala Leu
               420                 425                 430
   cac aac cac tac acc cag aag agc ctg agc ctg agc ccc ggc aag         1341
   His Asn His Tyr Thr Gln Lys Ser Leu Ser Leu Ser Pro Gly Lys
           435                 440                 445
<210> 51
   <211> 447
   <212> PRT
   <213> Artificial sequence
<220>
   <223> Heavy chain of 02 -113
   <400> 51
<210> 52
   <211> 1344
   <212> DNA
   <213> Artificial sequence
<220>
   <223> Heavy chain of 02 -114
   <220>
   <221> CDS
   <222> (1)..(1344)
   <223>
<400> 52
<210> 53
   <211> 448
   <212> PRT
   <213> Artificial sequence
<220>
   <223> Heavy chain of 02 -114
   <400> 53
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ser Met Asn Trp Val Arg Gln Ala Pro Gly Lys Gly Leu Glu Trp Val
           35                  40                  45
   Ser Tyr Ile Ser Ser Ser Ser Ser Thr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Asp Glu Asp Thr Ala Val Tyr Tyr Cys
                   85                  90                  95
   Ala Lys Glu Thr Trp Trp Gln Ser Phe Asp Tyr Trp Gly Gln Gly Thr
               100                 105                 110
   Leu Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Arg Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Arg Glu Glu Met Thr Lys Asn Gln Val Ser Leu Thr Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
           435                 440                 445
<210> 54
   <211> 1344
   <212> DNA
   <213> Artificial sequence
<220>
   <223> Heavy chain of 02 -115
   <220>
   <221> CDS
   <222> (1)..(1349)

   <223>
<400> 54
<210> 55
   <211> 448
   <212> PRT
   <213> Artificial sequence
<220>
   <223> Heavy chain of 02 -115
   <400> 55
   Gln Val Gln Leu Val Gln Ser Gly Ala Glu Ala Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Ser Ala Tyr Asn Gly Asn Thr Asn Tyr Ala Gln Lys Leu
   50 55 60
   Gln Gly Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Ser Gln Met Pro Ser Tyr Phe Asp Tyr Trp Gly Gln Gly Thr
   100 105 110
   Leu Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Arg Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Arg Glu Glu Met Thr Lys Asn Gln Val Ser Leu Thr Cys
           355                 360                 365
   Leu Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly Lys
   435 440 445
<210> 56
   <211> 1344
   <212> DNA
   <213> Artificial sequence
<220>
   <223> Heavy chain of 02 -116
   <220>
   <221> CDS
   <222> (1)..(1344)
   <223>
<400> 56
   gag gtg cag ctg gtg gag tct ggg gga ggc ttg gta cag cct ggg ggg 48
   Glu Val Gln Leu Val Glu Ser Gly Gly Gly Leu Val Gln Pro Gly Gly
   1 5 10 15
   tcc ctg aga ctc tcc tgt gca gcc tct gga ttc acc ttt agc agc tat 96
   Ser Leu Arg Leu Ser Cys Ala Ala Ser Gly Phe Thr Phe Ser Ser Tyr
   20 25 30
   gcc atg agc tgg gtc cgc cag gct cca ggg aag ggg ctg gag tgg gtc 144
   Ala Met Ser Trp Val Arg Gln Ala Pro Gly Lys Gly Leu Glu Trp Val
   35 40 45
   tca gct att agt ggt agt ggt ggt agc aca tac tac gca gac tcc gtg 192
   Ser Ala Ile Ser Gly Ser Gly Gly Ser Thr Tyr Tyr Ala Asp Ser Val
   50 55 60
   aag ggc cgg ttc acc atc tcc aga gac aat tcc aag aac acg ctg tat 240
   Lys Gly Arg Phe Thr Ile Ser Arg Asp Asn Ser Lys Asn Thr Leu Tyr
   65 70 75 80
   ctg caa atg aac agc cta aga gcc gag gac acg gcc gtg tat tac tgt 288
   Leu Gln Met Asn Ser Leu Arg Ala Glu Asp Thr Ala Val Tyr Tyr Cys
   85 90 95
   gca aag gac gct ctt tgg ctg gct ttt gac tac tgg ggc cag g gc acc 336
   Ala Lys Asp Ala Leu Trp Leu Ala Phe Asp Tyr Trp Gly Gln Gly Thr
   100 105 110
   ctg gtg acc gtc tcc agc gct agc acc aag ggc ccc agc gtg ttc ccc 384
   Leu Val Thr Val Ser Ser Ala Ser Thr Lys Gly Pro Ser Val Phe Pro
   115 120 125
   ctg gcc ccc agc agc aag agc acc agc ggc ggc aca gcc gcc ctg ggc 432
   Leu Ala Pro Ser Ser Lys Ser Thr Ser Gly Gly Thr Ala Ala Leu Gly
   130 135 140
   tgc ctg gtg aag gac tac ttc ccc gag ccc gtg acc gtg agc tgg aac 480
   Cys Leu Val Lys Asp Tyr Phe Pro Glu Pro Val Thr Val Ser Trp Asn
   145 150 155 160
   agc ggc gcc ttg acc agc ggc gtg cac acc ttc ccc gcc gtg ctg cag 528
   Ser Gly Ala Leu Thr Ser Gly Val His Thr Phe Pro Ala Val Leu Gln
   165 170 175
   agc agc ggc ctg tac agc ctg agc agc gtg gtg acc gtg ccc agc agc 576
   Ser Ser Gly Leu Tyr Ser Leu Ser Ser Val Val Thr Val Pro Ser Ser
   180 185 190
   agc ctg ggc acc cag acc tac atc tgc aac gtg aac cac aag ccc agc 624
   Ser Leu Gly Thr Gln Thr Tyr Ile Cys Asn Val Asn His Lys Pro Ser
   195 200 205
   aac acc aag gtg gac aaa cgc gtg gag ccc aag agc tgc g ac aag acc 672
   Asn Thr Lys Val Asp Lys Arg Val Glu Pro Lys Ser Cys Asp Lys Thr
   210 215 220
   cac acc tgc ccc ccc tgc cct gcc ccc gag ctg ctg ggc gga ccc tcc 720
   His Thr Cys Pro Pro Cys Pro Ala Pro Glu Leu Leu Gly Gly Pro Ser
   225 230 235 240
   gtg ttc ctg ttc ccc ccc aag ccc aag gac acc ctc atg atc agc cgg 768
   Val Phe Leu Phe Pro Pro Lys Pro Lys Asp Thr Leu Met Ile Ser Arg
   245 250 255
   acc ccc gag gtg acc tgc gtg gtg gtg gac gtg agc cac gag gac ccc 816
   Thr Pro Glu Val Thr Cys Val Val Val Asp Val Ser His Glu Asp Pro
   260 265 270
   gag gtg aag ttc aac tgg tac gtg gac ggc gtg gag gtg cac aac gcc 864
   Glu Val Lys Phe Asn Trp Tyr Val Asp Gly Val Glu Val His Asn Ala
   275 280 285
   aag acc aag ccc cgg gag gag cag tac aac agc acc tac cgg gtg gtg 912
   Lys Thr Lys Pro Arg Glu Glu Gln Tyr Asn Ser Thr Tyr Arg Val Val
   290 295 300
   agc gtg ctc acc gtg ctg cac cag gac tgg ctg aac ggc aag gag tac 960
   Ser Val Leu Thr Val Leu His Gln Asp Trp Leu Asn Gly Lys Glu Tyr
   305 310 315 320
   aag tgc aag gtg agc aac aag gcc ctg cct gcc ccc a tc gag aag acc 1008
   Lys Cys Lys Val Ser Asn Lys Ala Leu Pro Ala Pro Ile Glu Lys Thr
                   325                 330                 335
   atc agc aag gcc aag ggc cag ccc cgg gag ccc cag gtg tac acc ctg      1056
   Ile Ser Lys Ala Lys Gly Gln Pro Arg Glu Pro Gln Val Tyr Thr Leu
               340                 345                 350
   ccc ccc agc cgg gag gag atg acc aag aac cag gtg tcc ctc acc tgt      1104
   Pro Pro Ser Arg Glu Glu Met Thr Lys Asn Gln Val  Ser Leu Thr Cys
           355                 360                 365
   ctg gtg aag ggc ttc tac ccc agc gac atc gcc gtg gag tgg gag agc      1152
   Leu Val Lys Gly Phe Tyr Pro Ser Asp Ile Ala Val Glu Trp Glu Ser
       370                 375                 380
   aac ggc cag ccc gag aac aac tac aag acc acc ccc cct gtg ctg gac      1200
   Asn Gly Gln Pro Glu Asn Asn Tyr Lys Thr Thr Pro Pro Val Leu Asp
   385                 390                 395                 400
   agc gac ggc agc ttc ttc ctg tac agc aag ctc acc gtg gac aag agc      1248
   Ser Asp Gly Ser Phe Phe Leu Tyr Ser Lys Leu Thr Val Asp Lys Ser
                   405                 410                 415
   cgg tgg cag cag ggc aac gtg ttc agc tgc agc gtg atg cac gag gcc      1296
   Arg Trp Gln Gln Gly Asn Val Phe Ser Cys Ser Val Met His Glu Ala
               420                 425                 430
   ctg cac aac cac tac acc cag aag agc ctg agc c tg agc ccc ggc aag      1344
   Leu His Asn His Tyr Thr Gln Lys Ser Leu Ser Leu Ser Pro Gly Lys
           435                 440                 445
<210> 57
   <211> 448
   <212> PRT
   <213> Artificial sequence
<220>
   <223> Heavy chain of 02-116
<400> 57
<210> 58
   <211> 1341
   <212> DNA
   <213> Artificial sequence
<220>
   <223> Heavy chain of 02-117
   <220>
   <221> CDS
   <222> (1)..(1341)
   <223>
<400> 58
   gag gtg cag ctg gtg gag tct ggg gga ggc ttg gta cat cct ggg ggg 48
   Glu Val Gln Leu Val Glu Ser Gly Gly Gly Leu Val His Pro Gly Gly
   1 5 10 15
   tcc ctg aga ctc tcc tgt gca ggc tct gga ttc acc ttc agt agc tat       96
   Ser Leu Arg Leu Ser Cys Ala Gly Ser Gly Phe Thr Phe Ser Ser Tyr
           20                  25                  30
   gct atg cac tgg gtt cgc cag gct cca gga aaa ggt ctg gag tgg gta      144
   Ala Met His Trp Val Arg Gln Ala Pro Gly Lys Gly Leu Glu Trp Val
       35                  40                  45
   tca gct att ggt act ggt ggt ggc aca tac tat gca gac tcc gtg aag      192
   Ser Ala Ile Gly Thr Gly Gly Gly Thr Tyr Tyr Ala Asp Ser Val Lys
       50                  55                  60
   ggc cga ttc acc atc tcc aga gac aat gcc aag aac tcc ttg tat ctt      240
   Gly Arg Phe Thr Ile Ser Arg Asp Asn Ala Lys Asn Ser Leu Tyr Leu
   65                  70                  75                  80
   caa atg aac agc ctg aga gcc gag gac acg gcc gtg tat tac tgt gca      288
   Gln Met Asn Ser Leu Arg Ala Glu Asp Thr Ala Val Tyr Tyr Cys Ala
                   85                  90                  95
   agg tct acg cct tgg ttt tcc ttt gac tac tgg ggc cag ggc acc ctg      336
   Arg Ser Thr Pro Trp Phe Ser Phe Asp Tyr Trp Gly Gln Gly Thr Leu
               100                 105                 110
   gtg acc gtc tcc agc gct agc acc aag ggc ccc agc gtg ttc ccc ctg      384
   Val Thr Val Ser Ser Ala Ser Thr Lys Gly Pro Ser Val Phe Pro Leu
           115                 120                 125
   gcc ccc agc agc aag agc acc agc ggc ggc aca gcc gcc ctg ggc tgc      432
   Ala Pro Ser Ser Lys Ser Thr Ser Gly Gly Thr Ala Ala Leu Gly Cys
       130                 135                 140
   ctg gtg aag gac tac ttc ccc gag ccc gtg acc gtg agc tgg aac agc      480
   Leu Val Lys Asp Tyr Phe Pro Glu Pro Val Thr Val Ser Trp Asn Ser
   145                 150                 155                 160
   ggc gcc ttg acc agc ggc gtg cac acc ttc ccc gcc gtg ctg cag agc      528
   Gly Ala Leu Thr Ser Gly Val His Thr Phe Pro Ala Val Leu Gln Ser
                   165                 170                 175
   agc ggc ctg tac agc ctg agc agc gtg gtg acc gtg ccc agc agc agc      576
   Ser Gly Leu Tyr Ser Leu Ser Ser Val Val Thr Val Pro Ser Ser Ser
               180                 185                 190
   ctg ggc acc cag acc tac atc tgc aac gtg aac cac aag ccc agc aac      624
   Leu Gly Thr Gln Thr Tyr Ile Cys Asn Val Asn His Lys Pro Ser Asn
           195                 200                 205
   acc aag gtg gac aaa cgc gtg gag ccc aag agc tgc gac aag acc cac      672
   Thr Lys Val Asp Lys Arg Val Glu Pro Lys Ser Cys Asp Lys Thr His
       210                 215                 220
   acc tgc ccc ccc tgc cct gcc ccc gag ctg ctg ggc gga ccc tcc gtg      720
   Thr Cys Pro Pro Cys Pro Ala Pro Glu Leu Leu Gly Gly Pro Ser Val
   225                 230                 235                 240
   ttc ctg ttc ccc ccc aag ccc aag gac acc ctc atg atc agc cgg acc      768
   Phe Leu Phe Pro Pro Lys Pro Lys Asp Thr Leu Met Ile Ser Arg Thr
                   245                 250                 255
   ccc gag gtg acc tgc gtg gtg gtg gac gtg agc cac gag gac ccc gag      816
   Pro Glu Val Thr Cys Val Val Val Asp Val Ser His Glu Asp Pro Glu
               260                 265                 270
   gtg aag ttc aac tgg tac gtg gac ggc gtg gag gtg  cac aac gcc aag       864
   Val Lys Phe Asn Trp Tyr Val Asp Gly Val Glu Val His Asn Ala Lys
           275                 280                 285
   acc aag ccc cgg gag gag cag tac aac agc acc tac cgg gtg gtg agc       912
   Thr Lys Pro Arg Glu Glu Gln Tyr Asn Ser Thr Tyr Arg Val Val Ser
       290                 295                 300
   gtg ctc acc gtg ctg cac cag gac tgg ctg aac ggc aag gag tac aag       960
   Val Leu Thr Val Leu His Gln Asp Trp Leu Asn Gly  Lys Glu Tyr Lys
   305                 310                 315                 320
   tgc aag gtg agc aac aag gcc ctg cct gcc ccc atc gag aag acc atc      1008
   Cys Lys Val Ser Asn Lys Ala Leu Pro Ala Pro Ile Glu Lys Thr Ile
                   325                 330                 335
   agc aag gcc aag ggc cag ccc cgg gag ccc cag gtg tac acc ctg ccc      1056
   Ser Lys Ala Lys Gly Gln Pro Arg Glu Pro Gln Val Tyr Thr Leu Pro
               340                 345                 350
   ccc agc cgg gag gag atg acc aag aac cag gtg tcc ctc acc tgt ctg      1104
   Pro Ser Arg Glu Glu Met Thr Lys Asn Gln Val Ser Leu Thr Cys Leu
           355                 360                 365
   gtg aag ggc ttc tac ccc agc gac atc gcc gtg gag tgg gag agc aac      1152
   Val Lys Gly Phe Tyr Pro Ser Asp Ile Ala Val Glu Trp Glu Ser Asn
       370                 375                 380
   ggc cag ccc gag aac aac tac aag acc acc ccc  cct gtg ctg gac agc      1200
   Gly Gln Pro Glu Asn Asn Tyr Lys Thr Thr Pro Pro Val Leu Asp Ser
   385                 390                 395                 400
   gac ggc agc ttc ttc ctg tac agc aag ctc acc gtg gac aag agc cgg      1248
   Asp Gly Ser Phe Phe Leu Tyr Ser Lys Leu Thr Val Asp Lys Ser Arg
                   405                 410                 415
   tgg cag cag ggc aac gtg ttc agc tgc agc gtg atg cac gag gcc ctg      1296
   Trp Gln Gln Gly Asn Val Phe Ser Cys Ser Val  Met His Glu Ala Leu
               420                 425                 430
   cac aac cac tac acc cag aag agc ctg agc ctg agc ccc ggc aag          1341
   His Asn His Tyr Thr Gln Lys Ser Leu Ser Leu Ser Pro Gly Lys
           435                 440                 445
<210> 59
   <211> 447
   <212> PRT
   <213> Artificial sequence
<220>
   <223> Heavy chain of 02 -117
   <400> 59
<210> 60
   <211> 1341
   <212> DNA
   <213> Artificial sequence
<220>
   <223> Heavy chain of 02 -118 <220>
   <221> CDS
   <222> (1)..(1341)
   <223>
<400> 60
   gag gtg cag ctg gtg gag tct ggg gga ggc ttg gta cat cct ggg ggg       48
   Glu Val Gln Leu Val Glu Ser Gly Gly Gly Leu Val His Pro Gly Gly
   1               5                   10                  15
   tcc ctg aga ctc tcc tgt gca ggc tct gga ttc acc ttc agt agc tat      96
   Ser Leu Arg Leu Ser Cys Ala Gly Ser Gly Phe Thr Phe Ser Ser Tyr
               20                  25                  30
   gct atg cac tgg gtt cgc cag gct cca gga aaa ggt ctg gag tgg gta     144
   Ala Met His Trp Val Arg Gln Ala Pro Gly Lys Gly Leu Glu Trp Val
           35                  40                  45
   tca gct att agt act ggt ggt ggc aca tac tat gca gac tcc gtg aag      192
   Ser Ala Ile Ser Thr Gly Gly Gly Thr Tyr Tyr Ala Asp Ser Val Lys
       50                  5 5                 60
   ggc cga ttc acc atc tcc aga gac aat gcc aag aac tcc ttg tat ctt     240
   Gly Arg Phe Thr Ile Ser Arg Asp Asn Ala Lys Asn Ser Leu Tyr Leu
   65                  70                  75                  80
   caa atg aac agc ctg aga gcc gag gac acg gcc gtg tat tac tgt gca      288
   Gln Met Asn Ser Leu Arg Ala Glu Asp Thr Ala Val Tyr Tyr Cys Ala
                   85                  90                  95
   agg agt gct tgg tgg ctg tcc ttt gac tcc tgg ggc cag ggc acc ctg      336
   Arg Ser Ala Trp Trp Leu Ser Phe Asp Ser Trp Gly Gln Gly Thr Leu
               100                 105                 110
   gtg acc gtc tcc agc gct agc acc aag ggc ccc agc gtg ttc ccc ctg      384
   Val Thr Val Ser Ser Ala Ser Thr Lys Gly Pro Ser Val Phe Pro Leu
           115                 120                 125
   gcc ccc agc agc aag agc acc agc ggc ggc aca gcc gcc ctg ggc tgc     432
   Ala Pro Ser Ser Lys Ser Thr Ser Gly Gly Thr Ala Ala Leu Gly Cys
       130                 135                 140
   ctg gtg aag gac tac ttc ccc gag ccc gtg acc gtg agc tgg aac agc     480
   Leu Val Lys Asp Tyr Phe Pro Glu Pro Val Thr Val Ser Trp Asn Ser
   145                 150                 155                 160
   ggc gcc ttg acc agc ggc gtg cac acc ttc ccc gcc gtg ctg cag agc      528
   Gly Ala Leu Thr Ser Gly Val His Thr Phe Pro Ala Val Leu Gln Ser
                   165                 170                 175
   agc ggc ctg tac agc ctg agc agc gtg gtg acc gtg ccc agc agc agc      576
   Ser Gly Leu Tyr Ser Leu Ser Ser Val Val Thr Val Pro Ser Ser Ser
               180                 185                 190
   ctg ggc acc cag acc tac atc tgc aac gtg aac cac aag ccc agc aac      624
   Leu Gly Thr Gln Thr Tyr Ile Cys Asn Val Asn His Lys Pro Ser Asn
           195                 200                 205
   acc aag gtg gac aaa cgc gtg gag ccc aag agc tgc gac aag acc cac      672
   Thr Lys Val Asp Lys Arg Val Glu Pro Lys Ser Cys Asp Lys Thr His
       210                 215                 220
   acc tgc ccc ccc tgc cct gcc ccc gag ctg ctg ggc gga ccc tcc gtg      720
   Thr Cys Pro Pro Cys Pro Ala Pro Glu Leu Leu Gly Gly Pro Ser Val
   225                 230                 235                 240
   ttc ctg ttc ccc ccc aag ccc aag gac acc ctc atg atc agc cgg acc      768
   Phe Leu Phe Pro Pro Lys Pro Lys Asp Thr Leu Met Ile Ser Arg Thr
                   245                 250                 255
   ccc gag gtg acc tgc gtg gtg gtg gac gtg agc cac gag gac ccc gag      816
   Pro Glu Val Thr Cys Val Val Val Asp Val Ser His Glu Asp Pro Glu
               260                 265                 270
   gtg aag ttc aac tgg tac gtg gac ggc gtg gag gtg cac aac gcc aag      864
   Val Lys Phe Asn Trp Tyr Val Asp Gly Val Glu Val His Asn Ala Lys
           275                 280                 285
   acc aag ccc cgg gag gag cag tac aac agc acc tac cgg gtg gtg agc      912
   Thr Lys Pro Arg Glu Glu Gln Tyr Asn Ser Thr Tyr Arg Val Val Ser
       290                 295                 300
   gtg ctc acc gtg ctg cac cag gac tgg ctg aac ggc aag gag tac aag      960
   Val Leu Thr Val Leu His Gln Asp Trp Leu Asn Gly Lys Glu Tyr Lys
   305                 310                 315                 320
   tgc aag gtg agc aac aag gcc ctg cct gcc ccc atc gag aag acc atc      1008
   Cys Lys Val Ser Asn Lys Ala Leu Pro Ala Pro Ile Glu Lys Thr Ile
                   325                 330                 335
   agc aag gcc aag ggc cag ccc cgg gag ccc cag gtg tac acc ctg ccc      1056
   Ser Lys Ala Lys Gly Gln Pro Arg Glu Pro Gln Val Tyr Thr Leu Pro
               340                 345                 350
   ccc agc cgg gag gag atg acc aag aac cag gtg tcc ctc acc tgt ctg      1104
   Pro Ser Arg Glu Glu Met Thr Lys Asn Gln Val Ser Leu Thr Cys Leu
           355                 360                 365
   gtg aag ggc ttc tac ccc agc gac atc gcc gtg gag tgg gag agc aac      1152
   Val Lys Gly Phe Tyr Pro Ser Asp Ile Ala Val Glu Trp Glu Ser Asn
       370                 375                 380
   ggc cag ccc gag aac aac tac aag acc acc ccc cct gtg ctg gac agc      1200
   Gly Gln Pro Glu Asn Asn Tyr Lys Thr Thr Pro Pro Val Leu Asp Ser
   385                 390                 395                 400
   gac ggc agc ttc ttc ctg tac agc aag ctc acc gtg gac aag agc cgg      1248
   Asp Gly Ser Phe Phe Leu Tyr Ser Lys Leu Thr Val Asp Lys Ser Arg
                   405                 410                 415
   tgg cag cag ggc aac gtg ttc agc tgc agc gtg atg cac gag gcc ctg      1296
   Trp Gln Gln Gly Asn Val Phe Ser Cys Ser Val Met His Glu Ala Leu
               420                 425                 430
   cac aac cac tac acc cag aag agc ctg agc ctg agc ccc ggc aag          1341
   His Asn His Tyr Thr Gln Lys Ser Leu Ser Leu Ser Pro Gly Lys
           435                 440                 445
<210> 61 <211> 447
   <212> PRT
   <213> Artificial sequence
<220>
   <223> Heavy chain of 02 -118
<400> 61
   Glu Val Gln Leu Val Glu Ser Gly Gly Gly Leu Val His Pro Gly Gly
   1 5 10 15
   Ser Leu Arg Leu Ser Cys Ala Gly Ser Gly Phe Thr Phe Ser Ser Tyr
   20 25 30
   Ala Met His Trp Val Arg Gln Ala Pro Gly Lys Gly Leu Glu Trp Val
   35 40 45
   Ser Ala Ile Ser Thr Gly Gly Gly Thr Tyr Tyr Ala Asp Ser Val Lys
   50 55 60
   Gly Arg Phe Thr Ile Ser Arg Asp Asn Ala Lys Asn Ser Leu Tyr Leu
   65 70 75 80
   Gln Met Asn Ser Leu Arg Ala Glu Asp Thr Ala Val Tyr Tyr Cys Ala
   85 90 95
   Arg Ser Ala Trp Trp Leu Ser Phe Asp Ser Trp Gly Gln Gly Thr Leu
   100 105 110
   Val Thr Val Ser Ser Ala Ser Thr Lys Gly Pro Ser Val Phe Pro Leu
   115 120 125
   Ala Pro Ser Ser Lys Ser Thr Ser Gly Gly Thr Ala Ala Leu Gly Cys
   130 135 140
   Leu Val Lys Asp Tyr Phe Pro Glu Pro Val Thr Val Ser Trp Asn Ser
   145 150 155 160
   Gly Ala Leu Thr Ser Gly Val His Thr Phe Pro Ala Val Leu Gln Ser
   165 170 175
   Ser Gly Leu Tyr Ser Leu Ser Ser Val Val Thr Val Pro Ser Ser Ser
   180 185 190
   Leu Gly Thr Gln Thr Tyr Ile Cys Asn Val Asn His Lys Pro Ser Asn
   195 200 205
   Thr Lys Val Asp Lys Arg Val Glu Pro Lys Ser Cys Asp Lys Thr His
   210 215 220
   Thr Cys Pro Pro Cys Pro Ala Pro Glu Leu Leu Gly Gly Pro Ser Val
   225 230 235 240
   Phe Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 62
   <211> 642
   <212> DNA
   <213> Artificial sequence
<220>
   <223> Light chain of 02-113, 02-114, 02-116, 02-117 and 02-118
   <220>
   <221> CDS
   <222> (1)..(642)
   <223>
<400> 62
   gac att cag atg acc cag tct cca tcc tcc ctg tct gca tct gta gga       48
   Asp Ile Gln Met Thr Gln Ser Pro Ser Ser Leu Ser Ala Ser Val Gly
   1               5                   10                  15
   gac aga gtc acc atc act tgc cgg gca agt cag agc att agc agc tac       96
   Asp Arg Val Thr Ile Thr Cys Arg Ala Ser Gln Ser Ile Ser Ser Tyr
               20                  25                  30
   tta aat tgg tat cag cag aaa cca ggg aaa gcc cct aag ctc ctg atc      144
   Leu Asn Trp Tyr Gln Gln Lys Pro Gly Lys Ala Pro Lys Leu Leu Ile
           35                  40                  45
   tat gct gca tcc agt ttg caa agt ggg gtc cca tca agg ttc agt ggc      192
   Tyr Ala Ala Ser Ser Leu Gln Ser Gly Val Pro Ser Arg Phe Ser Gly
       50                  55                  60
   agt gga tct ggg aca gat ttc act c tc acc atc agc agt ctg caa cct     240
   Ser Gly Ser Gly Thr Asp Phe Thr Leu Thr Ile Ser Ser Leu Gln Pro
   65                  70                  75                  80
   gaa gat ttt gca act tac tac tgt caa cag agt tac agt acc cct cca      288
   Glu Asp Phe Ala Thr Tyr Tyr Cys Gln Gln Ser Tyr Ser Thr Pro Pro
                   85                  90                  95
   acg ttc ggc caa ggg acc aag gtg gag atc aaa cgg acc gtg gcc gct      336
   Thr Phe Gly Gln Gly Thr Lys Val  Glu Ile Lys Arg Thr Val Ala Ala
               100                 105                 110
   ccc agc gtg ttc atc ttc ccc ccc tcc gac gag cag ctg aag agc ggc      384
   Pro Ser Val Phe Ile Phe Pro Pro Ser Asp Glu Gln Leu Lys Ser Gly
           115                 120                 125
   acc gcc agc gtg gtg tgc ctg ctg aac aac ttc tac ccc cgg gag gcc      432
   Thr Ala Ser Val Val Cys Leu Leu Asn Asn Phe Tyr Pro Arg Glu Ala
       130                 135                 140
   aag gtg cag tgg aag gtg gac aac gcc ctg cag agc ggc aac agc cag      480
   Lys Val Gln Trp Lys Val Asp Asn Ala Leu Gln Ser Gly Asn Ser Gln
   145                 150                 155                 160
   gag agc gtg acc gag cag gac agc aag gac tcc acc tac agc ctg agc      528
   Glu Ser Val Thr Glu Gln Asp Ser Lys Asp Ser Thr Tyr Ser Leu Ser
                   165                 170                 175
   agc acc ctc acc ctg agc aag g cc gac tac gag aag cac aag gtg tac     576
   Ser Thr Leu Thr Leu Ser Lys Ala Asp Tyr Glu Lys His Lys Val Tyr
               180                 185                 190
   gcc tgc gag gtg acc cac cag ggc ctg agc agc ccc gtg acc aag agc      624
   Ala Cys Glu Val Thr His Gln Gly Leu Ser Ser Pro Val Thr Lys Ser
           195                 200                 205
   ttc aac cgg ggc gag tgt                                              642
   Phe Asn Arg Gly Glu Cys
       210
<210> 63
   <211> 214
   <212> PRT
   <213> Artificial sequence
<220>
   <223> Light chain of 02-113, 02-119, 02-116, 02-117 and 02-118
   <400> 63
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Pro
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 64
   <211> 696
   <212> DNA
   <213> Artificial sequence
<220>
   <223> Light chain of 02-115
   <220>
   <221> CDS
   <222> (1)..(696)
   <223>
<400> 64
<210> 65
   <211> 232
   <212> PRT
   <213> Artificial sequence
<220>
   <223> Light chain of 02 -115
   <400> 65
   Ser Ser Glu Leu Thr Gln Asp Pro Ala Val Ser Val Ala Leu Gly Gln
   1 5 10 15
   Thr Val Arg Ile Thr Cys Gln Gly Asp Ser Leu Arg Ser Tyr Tyr Ala
   20 25 30
   Ser Trp Tyr Gln Gln Lys Pro Gly Gln Ala Pro Val Leu Val Ile Tyr
   35 40 45
   Gly Lys Asn Asn Arg Pro Ser Gly Ile Pro Asp Arg Phe Ser Gly Ser
   50 55 60
   Ser Ser Gly Asn Thr Ala Ser Leu Thr Ile Thr Gly Ala Gln Ala Glu
   65 70 75 80
   Asp Glu Ala Asp Tyr Tyr Cys Asn Ser Arg Asp Ser Ser Gly Asn His
   85 90 95
   Val Val Phe Gly Gly Gly Thr Lys Leu Thr Val Leu Gly Glu Ser Arg
   100 105 110
   Pro Gln Ala Tyr Arg Ala Gly Pro Ala Gln Gly Arg Ser Gln Arg Asp
   115 120 125
   Pro Val Pro Pro Leu Leu Arg Gly Ala Ala Gly Gln Gln Gly His Pro
   130 135 140
   Gly Val Pro His Gln Arg Leu Leu Pro Trp Arg Arg Asp Arg Gly Leu
   145 150 155 160
   Glu Gly Arg Gln Gln Pro Arg Glu Gly Arg Arg Gly Asp His His Pro
   165 170 175
   Gln Gln Ala Glu Gln Gln Gln Val Arg Arg Gln Gln Leu Pro Glu Pro
   180 185 190
   His Pro Arg Ala Val Glu Glu Pro Pro Glu Leu Gln Leu Pro Gly Asp
   195 200 205
   Pro Arg Gly Gln His Arg Gly Glu Asp Arg Gly Pro His Arg Val Gln
   210 215 220
   Leu Ile Asp Leu Ser Leu Asn Arg
   225 230

## Description

### FIELD OF THE INVENTION

The invention relates to the identification of human binding molecules capable of specifically binding to CD1a, to immunoconjugates comprising these binding molecules and to methods of obtaining the binding molecules. The invention further encompasses the use of the human binding molecules in medicine, in particular for the diagnosis, prevention and/or treatment of neoplastic diseases and Langerhans Cell Histiocytosis.

### BACKGROUND OF THE INVENTION

CD1 molecules are a family of molecules that are expressed on the surfaces of dendritic cells, monocytes, and some thymocytes. CD1 molecules are similar to MHC Class I molecules in that they are involved in antigen presentation. Five CD1 genes have thus far been identified in humans: CD1a, CD1b, CD1c, CD1d and CD1e. Four of these five CD1 gene products have been defined serologically. They are referred to as CD1a, CD1b, CD1c and CD1d and are distinguished by unique heavy chains with approximate molecular weights of 49kDa, 45kDa, 43kDa and 48kDa, respectively.

The fact that CD1a molecules are expressed by acute and chronic leukaemic cells of the pre-B, B, T and non-lymphoid lineages renders CD1a in principle a target to detect or attack these disorders (Salamone et al. (1990a), Salamone (1990b), Merle-Beral *et al.* (1989)).

Furthermore, CD1a molecules are present on Langerhans cells (which are the major dendritic antigen-presenting cells in the skin) (Teunissen (1992)). This renders CD1a in principle a target to detect or treat Langerhans Cell Histiocytosis (LCH), a clonal proliferative neoplasm with variable clinical manifestations, ranging from solitary, self-limiting lesions to multisystem disease that can be life threatening.

Binding molecule that specifically bind to CD1a might be very useful in diagnosis and treatment of the above-mentioned disorders. Several murine monoclonal antibodies directed against CD1a are known in the art (Kelly (1994), Amiot *et al.* (1986), Furue et al. (1992) In addition, Moulon et al. (J. Invest. Dermatol. 97:524-528, 1991) have disclosed different murine anti-CD1a monoclonal antibodies recognizing different CD1a epitopes on the surface of Lngerhans cells. Murray et al. (J. Invest. Dermatol. 114:127-134, 2001) have investigated the potential of one of these murine anti-CD1a antibodies in diagnosing and treatment in a model of Langerhans cell histiocytosis. However, murine antibodies, in naked or immunoconjugated format, are limited for their use *in vivo* due to problems associated with administration of murine antibodies to humans, such as short serum half life, an inability to trigger certain human effector functions and elicitation of an unwanted dramatic immune response against the murine antibody in a human (the "human antimouse antibody" (HAMA) reaction) (Van Kroonenburgh and Pauwels (1988)).

In general, attempts to overcome the problems associated with use of fully murine antibodies in humans, have involved genetically engineering the antibodies to be more "human-like". A first stage in the humanization process was preparing chimeric antibodies, *i.e.* antibodies in which the variable regions of the antibody chains are murine-derived and the constant regions of the antibody chains are human-derived. Subsequently, domains between the variable domains which specify the antigen binding were replaced by their human counterparts leading to so-called humanized antibodies. A disadvantage of these chimeric and humanized antibodies is that they still retain some murine sequences and therefore still elicit an unwanted immune reaction, especially when administered for prolonged periods.

In view of their benefit in therapy there is still a need for human binding molecules against CD1a.

The present invention provides human binding molecules against CD1a that can be used in medicine, in particular for diagnosis, prevention and/or treatment of CD1a-associated disorders.

### DESCRIPTION OF THE FIGURES

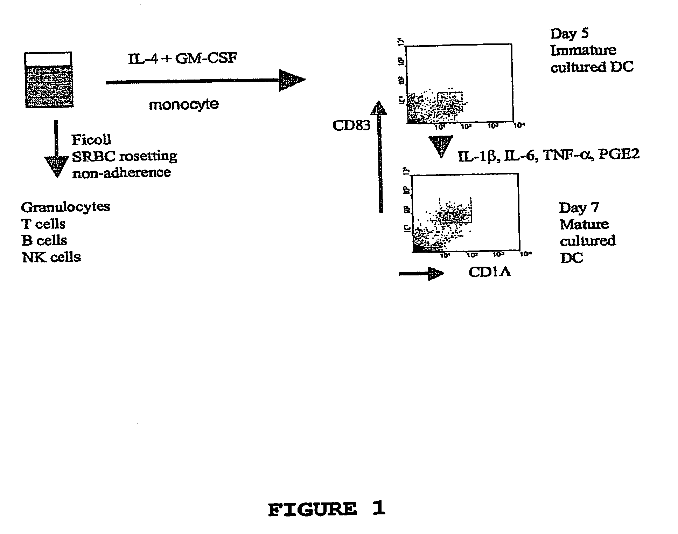

Figure 1. Isolation of monocytes and differentiation of monocytes to immature dendritic cells (DCs) by IL-4 and GM-CSF and to mature DCs by an additional cocktail containing IL-1β, IL-6, TNF-α, PGE2. Indicated in the FACS pictures are a mixture of PBLs, used as subtractor cells during phage selection, and cultured DCs (boxed), wherein the amount of PBLs is about 10 times the amount of DCs.

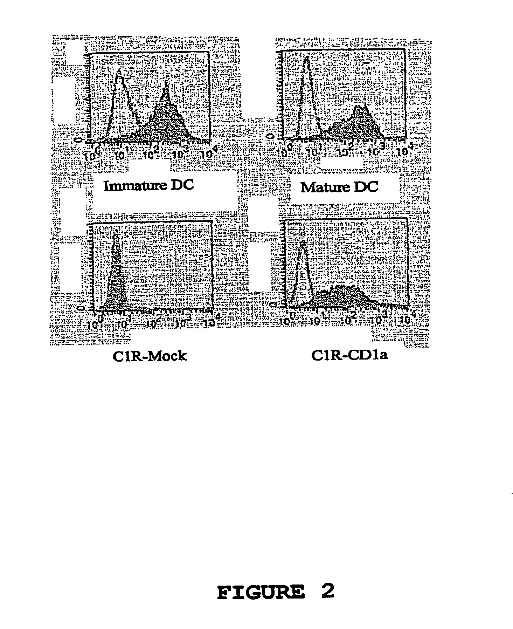

Figure 2. FACS analysis of the binding of a representative monoclonal phage antibody (called SC02-113) to cultured immature (top, left) and mature (top, right) DCs and a CD1a transfectant in C1R cells (bottom, right), compared with the C1R mock-transfected negative control (bottom, left).

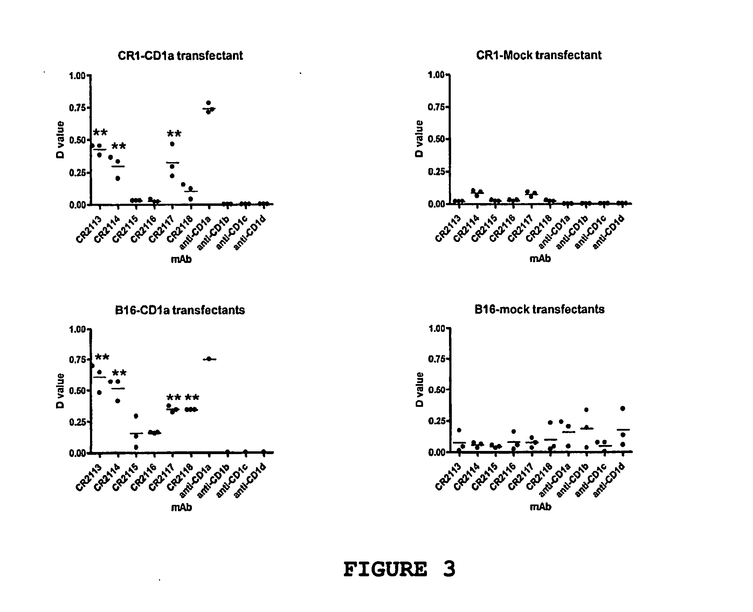

Figure 3. Binding of the human anti-human CD1a monoclonal antibodies called CR2113, CR2114, CR2115, CR2116, CR2117 and CR2118 and commercially available mouse anti-human CD1a antibodies, mouse anti-human CDlb antibodies, mouse anti-human CD1c antibodies and mouse anti-human CD1d antibodies to CR1-CD1a and B16-CDla transfectants and CR1 and B16 mock transfectants. Significant binding of an human anti-human CD1a monoclonal antibodies to CD1a transfectants compared to mock transfectants is indicated with **. Significance was tested with a unpaired two-tailed t-test (p<0.05).

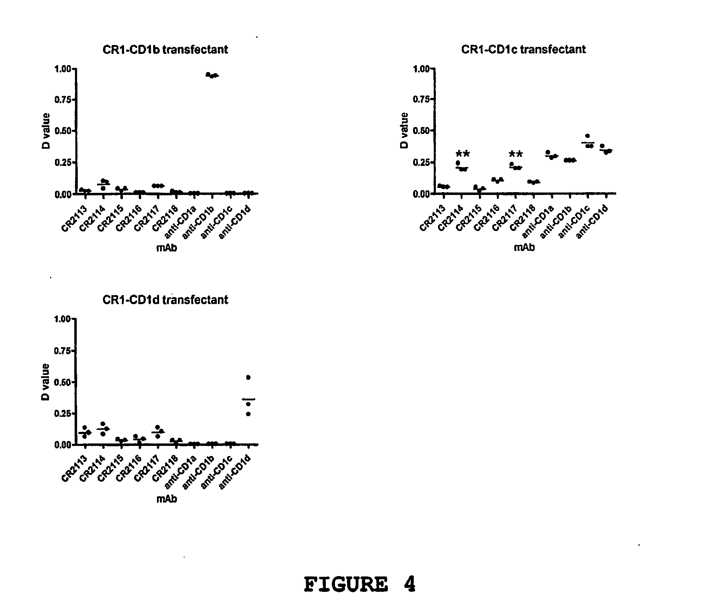

Figure 4. Binding of the human anti-human CD1a monoclonal antibodies called CR2113, CR2114, CR2115, CR2116, CR2117 and CR2118 and commercially available mouse anti-human CD1a antibodies, mouse anti-human CDlb antibodies, mouse anti-human CD1c antibodies and mouse anti-human CD1d antibodies to CR1-CD1b, CR1-CD1c and CR1-CD1d transfectants. Significant binding of an human anti-human CD1a monoclonal antibodies to the CR1-CD1b, CR1-CD1c or CR1-CD1d transfectants compared to CR1 mock transfectants is indicated with **. Significance was tested with a unpaired two-tailed t-test (p<0.05).

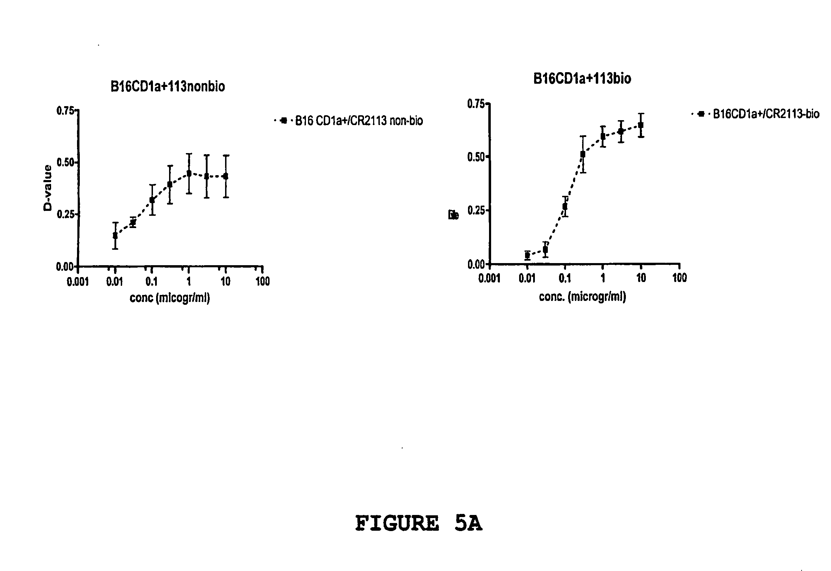

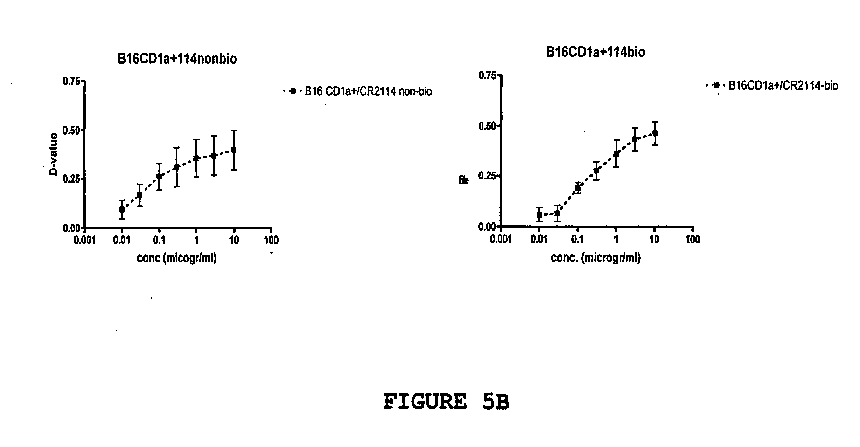

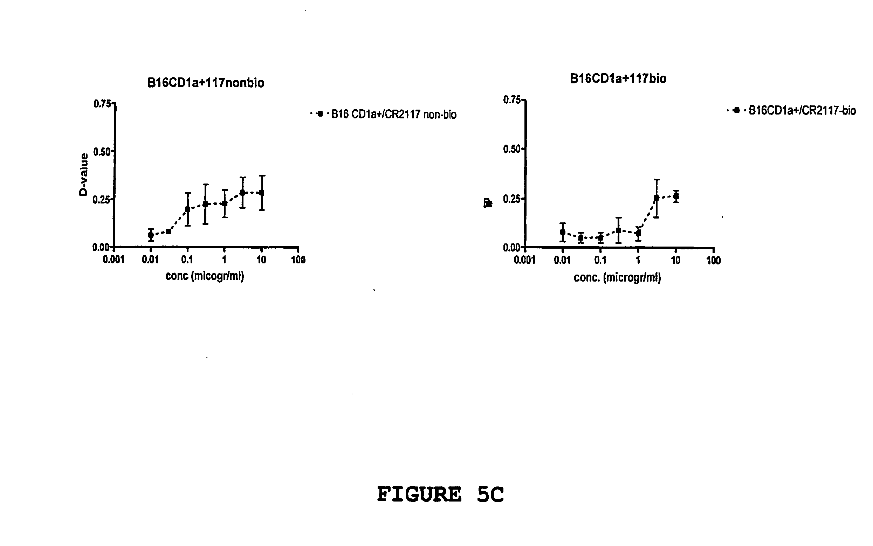

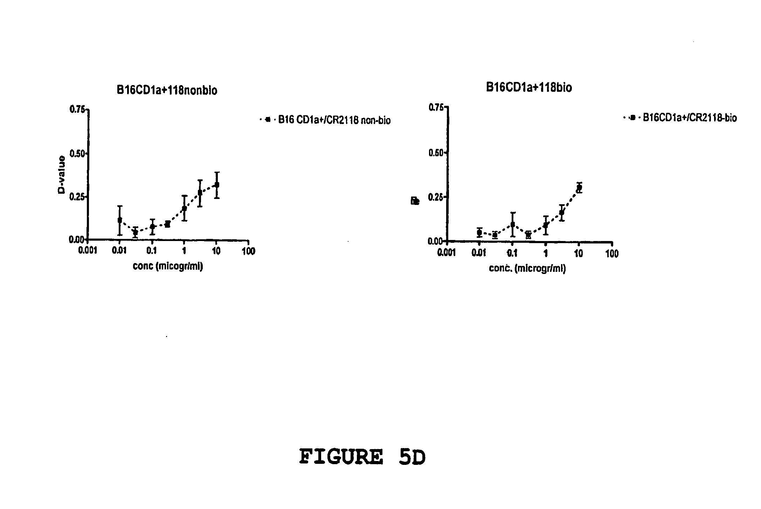

Figure 5. Figures 5A-D show binding curves of unlabeled and biotin labeled human anti-human CD1a monoclonal antibodies called CR2113, CR2114, CR2117 and CR2118 on B16-CD1a transfectants.

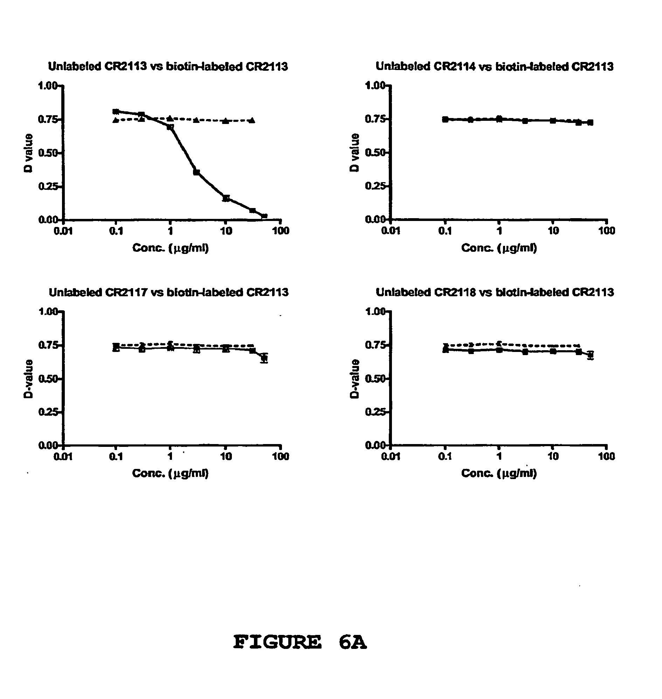

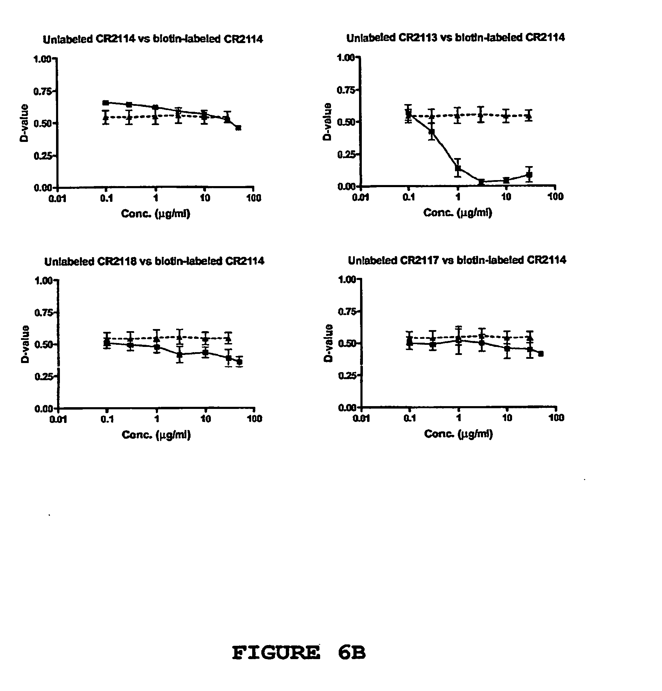

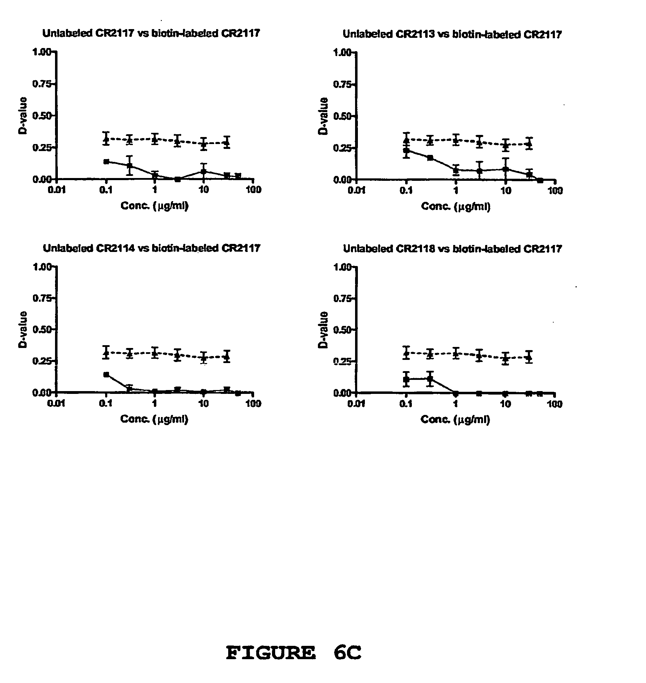

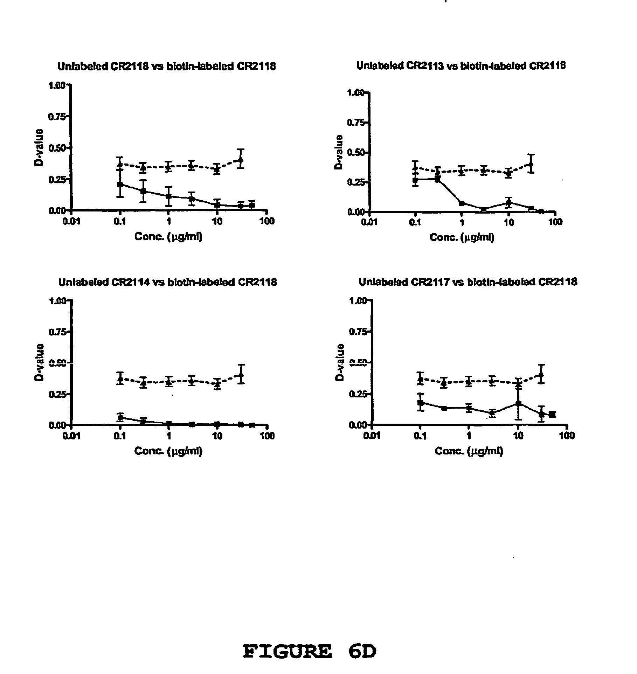

Figure 6. Figures 6A-D show competition curves for CR2113, CR2114, CR2117, and CR2118 against the human anti-human CD1a monoclonal antibodies.

### DESCRIPTION OF THE INVENTION

Herebelow follow definitions of terms as used in the invention

### DEFINITIONS

### Acute myeloid leukemia

As used herein the term "acute myeloid leukemia" is characterized by an uncontrolled proliferation of progenitor cells of myeloid origin including, but not limited to, myeloid progenitor cells, myelomonocytic progenitor cells, immature megakaryoblasts. Subtypes of acute myeloid leukemia (AML) include according to the FAB classification FAB-M0, FAB-M1, FAB-M2, FAB-M3, FAB-M4, FAB-M5, FAB-M6 and FAB-M7.

### Amino acid sequence

The term "amino acid sequence" as used herein refers to naturally occuring or synthetic molecules and to a peptide, oligopeptide, polypeptide or protein sequence.

### Apoptosis

As used herein, the term "apoptosis" refers to any cell death, orderly or controlled that results from the complex cascade of cellular events that occur at specific stages of cellular differentiation and in response to specific stimuli. Apoptosis is characterized and/or accompanied by one or more characteristic cell changes, including, but not limited to, condensation of cytoplasm, loss of plasma membrane microvilli, segmentation of the nucleus, degradation of chromosomal DNA or loss of mitochondrial function. Apoptosis can be determined and measured, for instance, by cell viability assays, FACS analysis or DNA electrophoresis, all of which are known in the art.

### Binding molecule

As used herein the term "binding molecule" refers to an intact immunoglobulin including monoclonal antibodies, such as chimeric, humanized or human monoclonal antibodies, or to an antigen-binding and/or variable domain comprising fragment of an immunoglobulin that competes with the intact immunoglobulin for specific binding to the binding partner of the immunoglobulin, e.g. CD1a. Regardless of structure, the antigen-binding fragment binds with the same antigen that is recognised by the intact immunoglobulin. The term "binding molecule", as used herein includes immunoglobulins from classes and subclasses of intact antibodies. These include IgA, IgD, IgE, IgG, and IgM, and several of these may be further divided into subclasses (isotypes), *e.g.*, IgA1, IgA2, IgG1, IgG2, IgG3 and IgG4 as well as antigen-binding fragments, thereof.

Antigen-binding fragments includes, *inter alia*, Fab, F(ab'), F(ab')₂, Fv, dAb, Fd, complementarity determining region (CDR) fragments, single-chain antibodies (scFv), bivalent single-chain antibodies, diabodies, triabodies, tetrabodies, (poly)peptides that contain at least a fragment of an immunoglobulin that is sufficient to confer specific antigen binding to the (poly)peptide, etc. The above fragments may be produced synthetically or by enzymatic or chemical cleavage of intact immunoglobulins or they may be genetically engineered by recombinant DNA techniques. The methods of production are well known in the art and are described, for example, in Antibodies: A Laboratory Manual, Edited by: E. Harlow and D, Lane (1988), Cold Spring Harbor Laboratory, Cold Spring Harbor, New York . A binding molecule or antigen-binding fragment thereof may have one or more binding sites. If there is more than one binding site, the binding sites may be identical to one another or they may be different.

The binding molecule can be a naked or unconjugated binding molecule. A naked or unconjugated binding molecule is intended to refer to a binding molecule that is not conjugated, operatively linked or otherwise physically or functionally associated with an effector moiety or tag, such as *inter alia* a toxic substance, a radioactive substance, a liposome, an enzyme. It will be understood that naked or unconjugated binding molecules do not exclude binding molecules that have been stabilized, multimerized, humanized or in any other way manipulated, other than by the attachment of an effector moiety or tag. Accordingly, all post-translationally modified naked and unconjugated binding molecules are included herewith, including where the modifications are made in the natural binding molecule-producing cell environment, by a recombinant binding molecule-producing cell, and are introduced by the hand of man after initial binding molecule preparation. Of course, the term naked or unconjugated binding molecule includes binding molecules having the ability to form functional associations with effector cells and/or molecules after administration to the body, as some such interactions are necessary in order to exert a biological effect.

### Biological sample

As used herein, the term "biological sample" encompasses a variety of sample types, including blood and other liquid samples of biological origin, solid tissue samples such as a biopsy specimen or tissue cultures, or cells derived therefrom and the progeny thereof. The term also includes samples that have been manipulated in any way after their procurement, such as by treatment with reagents, solubilization, or enrichment for certain components, such as proteins or polynucleotides. The term encompasses various kinds of clinical samples obtained from any species, and also includes cells in culture, cell supernatants and cell lysates.

### Chronic myeloid leukemia

The term "chronic myeloid leukemia" as used herein is characterized by an uncontrolled proliferation of myelopoietic cells in the bone marrow and extramedullary sites in which the malignant myeloblast is able to differentiate and give rise to myelocytes, metamyelocytes, band cells and granulocytes.

### Complementary determining regions (CDR)

The term "complementary determining regions" as used herein means sequences within the variable regions of binding molecules, such as immunoglobulins, that generate the antigen binding site which is complementary in shape and charge distribution to the epitope recognised on the antigen. The CDR regions can be specific for linear epitopes, discontinuous epitopes, or conformational epitopes of proteins or protein fragments, either as present on the protein in its native conformation or, in some cases, as present on the proteins as denatured, e.g., by solubilization in SDS. Epitopes may also consist of post translational modifications of proteins.

### Deletion

The term "deletion", as used herein, denotes a change in either amino acid or nucleotide sequence in which one or more amino acid or nucleotide residues, respectively, are absent as compared to the parent, often the naturally occurring, molecule.

### Expression-regulating nucleic acid sequence

The term "expression-regulating nucleic acid sequence" as used herein refers to polynucleotide sequences necessary for and/or affecting the expression of an operably linked coding sequence in a particular host organism. When two nucleic acid sequences are operably linked, they usually will be in the same orientation and also in the same reading frame. They usually will be essentially contiguous, although this may not be required. The expression-regulating nucleic acid sequences, such as inter alia appropriate transcription initiation, termination, promoter, enhancer sequences; repressor or activator sequences; efficient RNA processing signals such as splicing and polyadenylation signals; sequences that stabilize cytoplasmic mRNA; sequences that enhance translation efficiency (e.g., ribosome binding sites); sequences that enhance protein stability; and when desired, sequences that enhance protein secretion, can be any nucleic acid sequence showing activity in the host organism of choice and can be derived from genes encoding proteins, which are either homologous or heterologous to the host organism.

### Functional variant

The term "functional variant", as used herein, refers to a binding molecule that comprises a nucleotide and/or amino acid sequence that is altered by one or more nucleotides and/or amino acids compared to the nucleotide and/or amino acid sequences of the parent binding molecule and that is still capable of competing for binding to the binding partner, e.g. CD1a, with the parent binding molecule. In other words, the modifications in the amino acid and/or nucleotide sequence of the parent binding molecule do not significantly affect or alter the binding characteristics of the binding molecule encoded by the nucleotide sequence or containing the amino acid sequence, i.e. the binding molecule is still able to recognize and bind its target. The functional variant may have conservative sequence modifications including nucleotide and amino acid substitutions, additions and deletions. These modifications can be introduced by standard techniques known in the art, such as site-directed mutagenesis and random PCR-mediated mutagenesis.

Conservative amino acid substitutions include for instance the ones in which the amino acid residue is replaced with an amino acid residue having similar structural or chemical properties. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (*e.g.*, threonine, valine, isoleucine) and aromatic side chains *(e.g.*, tyrosine, phenylalanine, tryptophan, histidine). Furthermore, a variant may have non-conservative amino acid substitutions, *e.g.*, replacement of an amino acid with an amino acid residue having different structural or chemical properties. Similar minor variations may also include amino acid deletions or insertions, or both. Guidance in determining which amino acid residues may be substituted, inserted, or deleted without abolishing immunological activity may be found using computer programs well known in the art.

A mutation in a nucleotide sequence can be a single alteration made at a locus (a point mutation), such as transition or transversion mutations, or alternatively, multiple nucleotides may be inserted, deleted or changed at a single locus. In addition, one or more alterations may be made at any number of loci within a nucleotide sequence. The mutations may be performed by any suitable method known in the art.

### Host

The term "host", as used herein, is intended to refer to an organism or a cell into which a vector such as a cloning vector or an expression vector has been introduced. The organism or cell can be prokaryotic or eukaryotic. It should be understood that this term is intended to refer not only to the particular subject organism or cell, but to the progeny of such an organism or cell as well. Because certain modifications may occur in succeeding generations due to either mutation or environmental influences, such progeny may not, in fact, be identical to the parent organism or cell, but are still included within the scope of the term "host" as used herein.

### Human

The term "human", when applied to binding molecules as defined herein, refers to molecules that are either directly derived from a human or based upon a human sequence. When a binding molecule is derived from or based on a human sequence and subsequently modified, it is still to be considered human as used throughout the specification. In other words, the term "human", when applied to binding molecules is intended to include binding molecules having variable and constant regions derived from human germline immunoglobulin sequences based on variable or constant regions either or not occuring in a human or human lymphocyte or in modified form. Thus, the human binding molecules may include amino acid residues not encoded by human germline immunoglobulin sequences, comprise substitutions and/or deletions (*e.g.*, mutations introduced by for instance random or site-specific mutagenesis *in vitro* or by somatic mutation *in vivo*). "Based on" as used herein also refers to the situation that a nucleic acid sequence may be exactly copied from a template, or with minor mutations, such as by error-prone PCR methods, or synthetically made matching the template exactly or with minor modifications. Semi-synthetic molecules based on human sequences are also considered to be human as used herein.

### Immunoliposome

The term "immunoliposome" refers to a liposome bearing a binding molecule, as defined herein, that acts as a targeting moiety enabling the liposome to specifically bind to the binding partner of the binding molecule. The binding partner may be present in solution or may be bound to the surface of a cell.

### Insertion

The term "insertion", also known as the term "addition", denotes a change in an amino acid or nucleotide sequence resulting in the addition of one or more amino acid or nucleotide residues, respectively, as compared to the parent, often the naturally occurring, molecule.

### Internalising binding molecule

The term "internalising binding molecule" as used herein means a binding molecule as defined herein that is capable of being internalised within the target cells to which it binds. In other words, the binding molecule is taken up, *i.e.* transported from the outside (cell surface) of a target cell to the inside, *e.g.* into the endosomal compartment or other compartment or into the cytoplasm of the cell, by the target cells upon binding to the binding partner of the binding molecule.

### Isolated

The term "isolated", when applied to binding molecules as defined herein, refers to binding molecules that are substantially free of other proteins or polypeptides, particularly free of other binding molecules having different antigenic specificities, and are also substantially free of other cellular material and/or chemicals. For example, when the binding molecules are recombinantly produced, they are preferably substantially free of culture medium, and when the binding molecules are produced by chemical synthesis, they are preferably substantially free of chemical precursors or other chemicals, *i.e.*, they are separated from chemical precursors or other chemicals which are involved in the synthesis of the protein. The term "isolated" when applied to nucleic acid molecules encoding binding molecules as defined herein, is intended to refer to nucleic acid molecules in which the nucleotide sequences encoding the binding molecules are free of other nucleotide sequences, particularly nucleotide sequences encoding binding molecules that bind binding partners other than CD1a. Furthermore, the term "isolated" refers to nucleic acid molecules that are substantially separated from other cellular components that naturally accompany the native nucleic acid molecule in its natural host, *e.g.*, ribosomes, polymerases, or genomic sequences with which it is naturally associated. Moreover, "isolated" nucleic acid molecules, such as a cDNA molecules, can be substantially free of other cellular material, or culture medium when produced by recombinant techniques, or substantially free of chemical precursors or other chemicals when chemically synthesized.

### Langerhans Cell Histiocytosis (LCH)

The term "Langerhans Cell Histiocytosis (LCH)" refers to a disorder which is characterized by localized or generalized proliferation of dendritic-like cells, resembling Langerhans cells. These cells have their origin in the bone-marrow and are normally mainly present in the skin. In LCH these cells proliferate and may or may not affect many other organs like among others bone, liver, lungs and brain. LCH is the most frequent among the histiocytic disorders. The disease occurs in persons of all ages, but the peak incidence is in children between 0 and 4 years. Clinical symptoms vary widely from limited skin rash or single bone lesions, which may heal spontaneously, to extensive visceral organ involvement and dysfunction, eventually leading to patient death.

### Liposome

The term "liposome" as used herein refers to a small vesicle bounded by a layer composed of various types of lipids, preferably amphipathic lipids, phospholipids and/or surfactants and made artificially from these molecules by techniques known in the art such as sonication or removal of detergent from phospholipid-detergent complexes. The layer typically is a bilayer formed by molecules that comprise a hydrophobic portion and a hydrophilic portion, wherein hydrophobic portions associate in an aqueous medium to form an internal part of the layer, whereas hydrophilic portions remain in contact with the medium. The layer surrounds and encloses an interior, which may contain, wholly or partially, an aqueous phase, a solid, a gel, a gas phase, or a non-aqueous fluid. Liposomes are useful for delivery of one or more molecules such as nucleic acid molecules, binding molecules, proteins, toxic substances and other material or compounds into cells such as animal cells by liposome fusion with the plasma membrane, a process also called lipofection. The molecules may be contained within the interior of the liposome, in the lipid layer, or attached to the outer surface of the lipid layer.

### Monoclonal antibody

The term "monoclonal antibody" as used herein refers to a monoclonal antibody displaying a single binding specificity and affinity for a particular epitope. Accordingly, the term "human monoclonal antibody" refers to an antibody displaying a single binding specificity which has variable and constant regions derived from human germline immunoglobulin sequences or derived from completely synthetic or semi-synthetic sequences.

### Myelodysplastic syndrome

The term "myelodysplastic syndrome" as used herein encompasses a heterogeneous group of closely related clonal hematopoietic disorders that originate in an early blood-forming cell in the marrow. All disorders are characterized by a cellular marrow with impaired morphology and maturation (dysmyelopoiesis) and peripheral blood cytopenias, resulting from ineffective blood cell production. In other words, the maturing blood cells often die in the marrow before they reach full maturity and enter the blood, accounting for the low blood cell concentrations. In patients suffering from myelodysplastic syndrome there may also be an accumulation of very immature marrow cells, called leukemic blast cells.

### Naturally occurring

The term "naturally-occurring" as used herein as applied to an object refers to the fact that an object can be found in nature. For example, a polypeptide or polynucleotide sequence that is present in an organism that can be isolated from a source in nature and which has not been modified by man in the laboratory is naturally-occurring.

### Neoplastic cells

The term "neoplastic cells" as used herein refers to cells that result from undesired autonomous new growth which has no apparent physiological function. A neoplastic cell further includes transformed cells and cancer cells including blood cancers (benign and malignant).

### Nucleic acid molecule

The term "nucleic acid molecule" as used in the present invention refers to a polymeric form of nucleotides and includes both sense and antisense strands of RNA, cDNA, genomic DNA, and synthetic forms and mixed polymers of the above. A nucleotide refers to a ribonucleotide, deoxynucleotide or a modified form of either type of nucleotide. The term also includes single- and double-stranded forms of DNA. In addition, a polynucleotide may include either or both naturally-occurring and modified nucleotides linked together by naturally-occurring and/or non-naturally occurring nucleotide linkages. The nucleic acid molecules may be modified chemically or biochemically or may contain non-natural or derivatized nucleotide bases, as will be readily appreciated by those of skill in the art. Such modifications include, for example, labels, methylation, substitution of one or more of the naturally occurring nucleotides with an analog, internucleotide modifications such as uncharged linkages (*e.g.,* methyl phosphonates, phosphotriesters, phosphoramidates, carbamates, etc.), charged linkages (*e.g.*, phosphorothioates, phosphorodithioates, etc.), pendent moieties (*e.g.*, polypeptides), intercalators (*e.g.*, acridine, psoralen, etc.), chelators, alkylators, and modified linkages (*e.g.*, alpha anomeric nucleic acids, etc.) The above term is also intended to include any topological conformation, including single-stranded, double-stranded, partially duplexed, triplex, hairpinned, circular and padlocked conformations. Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule. A reference to a nucleic acid sequence encompasses its complement unless otherwise specified. Thus, a reference to a nucleic acid molecule having a particular sequence should be understood to encompass its complementary strand, with its complementary sequence. The complementary strand is also useful, *e.g.*, for antisense therapy, hybridization probes and PCR primers.

### Operably linked

The term "operably linked" refers to two or more nucleic acid sequence elements that are physically linked and are in a functional relationship with each other. For instance, a promoter is operably linked to a coding sequence if the promoter is able to initiate or regulate the transcription or expression of a coding sequence, in which case the coding sequence should be understood as being "under the control of" the promoter. Generally, when two nucleic acid sequences are operably linked, they will be in the same orientation and usually also in the same reading frame. They usually will be essentially contiguous, although this may not be required.

### Pharmaceutically acceptable excipient

By "pharmaceutically acceptable excipient" is meant any inert substance that is combined with an active molecule such as a drug, agent, or binding molecule for preparing an agreeable or convenient dosage form. The "pharmaceutically acceptable excipient" is an excipient that is non-toxic to recipients at the dosages and concentrations employed and is compatible with other ingredients of the formulation comprising the drug, agent, or binding molecule.

### Specifically Binding

The term "specifically binding", as used herein, in reference to the interaction of a binding molecule, *e.g.* an antibody, and its binding partner, *e.g.* an antigen, means that the interaction is dependent upon the presence of a particular structure, *e.g.* an antigenic determinant or epitope, on the binding partner. In other words, the antibody preferentially binds or recognizes the binding partner even when the binding partner is present in a mixture of other molecules. The binding may be mediated by covalent or non-covalent interactions or a combination of both.

### Substitutions

A "substitution", as used herein, denotes the replacement of one or more amino acids or nucleotides by different amino acids or nucleotides, respectively.

### Therapeutically effective amount

The term "therapeutically effective amount" refers to an amount of the binding molecule as defined herein that is effective for preventing and/or treating a disorder or disease wherein CD1a molecules play a role or are associated with, or ameliorating a condition associated with the disease or disorder.

### Treatment

The term "treatment" refers to therapeutic treatment as well as prophylactic or preventative measures. Those in need of treatment include those already with the disease or disorder wherein CD1a molecules play a role or are associated with as well as those in which the disease or disorder is to be prevented.

### Vector

The term "vector" denotes a nucleic acid molecule into which a second nucleic acid molecule can be inserted for introduction into a host where it will be replicated, and in some cases expressed. In other words, a vector is capable of carrying a second nucleic acid molecule to which it has been linked. Cloning as well as expression vectors are contemplated by the term "vector", as used herein. Vectors include, but are not limited to, plasmids, cosmids, bacterial artificial chromosomes (BAC) and yeast artificial chromosomes (YAC) and vectors derived from bacteriophages or plant or animal (including human) viruses. Vectors comprise an origin of replication recognised by the proposed host and in case of expression vectors, promoter and other regulatory regions recognised by the host. A vector containing a second nucleic acid molecule may be introduced into a cell by transformation, transfection, or by making use of viral entry mechanisms. Certain vectors are capable of autonomous replication in a host into which they are introduced (*e.g.*, bacterial vectors having a bacterial origin of replication). Other vectors can be integrated into the genome of a host upon introduction into the host, and thereby are replicated along with the host genome.

### SUMMARY OF THE INVENTION

In the present invention several human binding molecules capable of binding to a human CD1a have been identified and obtained by using phage display technology. Furthermore, methods of producing these human binding molecules and the use of the human binding molecules in diagnosis, prevention and treatment of *inter alia* neoplastic disorders and diseases have been described.

### DETAILED DESCRIPTION OF THE INVENTION

The present invention encompasses human binding molecules capable of specifically binding to human CD1a. The binding molecules are also capable of binding, particularly specifically binding, to a fragment of human CD1a, the fragment at least comprising the antigenic determinant of CD1a that is recognised by the human binding molecules of the invention. Human binding molecules capable of specifically binding naturally-occurring truncated or secreted forms, naturally-occurring variant forms (*e.g.*, alternatively spliced forms) and naturally-occurring allelic variants of CD1a are also a part of the present invention. Binding molecules of the invention may also be capable of specifically binding to non-naturally occuring variants or analogues of CD1a as long as the modifications do not abolish the binding of the binding molecules to the CD1a molecules.

The human binding molecules according to the invention can be intact immunoglobulin molecules such as polyclonal or monoclonal antibodies, or the binding molecules can be antigen-binding fragments including, but not limited to, Fab, F(ab'), F(ab')₂, Fv, dAb, Fd, complementarity determining region (CDR) fragments, single-chain antibodies (scFv), bivalent single-chain antibodies, diabodies, triabodies, tetrabodies, and (poly)peptides that contain at least a fragment of an immunoglobulin that is sufficient to confer specific antigen binding to the (poly)peptides. The human binding molecules of the invention can be used in non-isolated or isolated form. Furthermore, the human binding molecules of the invention can be used alone or in a mixture comprising at least one human binding molecule (or variant or fragment thereof). In other words, the human binding molecules can be used in combination, *e.g.*, as a pharmaceutical composition comprising two or more human binding molecules or fragments thereof. For example, human binding molecules having different, but complementary activities can be combined in a single therapy to achieve a desired therapeutic or diagnostic effect, but alternatively, human binding molecules having identical activities can also be combined in a single therapy to achieve a desired therapeutic or diagnostic effect. The mixture may further comprise at least one other therapeutic agent. Typically, human binding molecules according to the invention can bind to their binding partners, *i.e.* human CD1a, with an affinity constant (K_{d}-value) that is lower than 0.2*10⁻⁴ M, 1.0*10⁻⁵ M, 1.0*10⁻⁶ M, 1.0*10⁻⁷ M, preferably lower than 1.0*10⁻⁸ M, more preferably lower than 1.0*10⁻⁹ M, more preferably lower than 1.0*10⁻¹⁰ M, even more preferably lower than 2.0*10⁻¹¹ M, and in particular lower than 1.0*10⁻¹² M. The affinity constants can vary for antibody isotypes. For example, affinity binding for an IgM isotype refers to a binding affinity of at least about 1.0*10⁻⁷ M. Affinity constants can for instance be measured using surface plasmon resonance, *i.e.* an optical phenomenon that allows for the analysis of real-time biospecific interactions by detection of alterations in protein concentrations within a biosensor matrix, for example using the BIAcore system (Pharmacia Biosensor AB, Uppsala, Sweden).

The human binding molecules according to the invention may bind to human CD1a in soluble form or may bind to human CD1a bound or attached to a carrier or substrate, *e.g.*, microtiter plates, membranes and beads, etc. Carriers or substrates may be made of glass, plastic (*e.g.* polystyrene), polysaccharides, nylon, nitrocellulose, or teflon, etc. The surface of such supports may be solid or porous and of any convenient shape. Furthermore, the human binding molecules may bind to the human CD1a in purified or non-purified form. Preferably, the human binding molecules are capable of specifically binding to human CD1a molecules associated with cells, such as a human CD1a positive cells or portions or parts of these cells comprising human CD1a or a fragment thereof.

In an embodiment of the invention, the human binding molecules of the invention which stay bound to the surface upon binding to human CD1a present on the surface of target cells may be used in the format of naked binding molecules to support possible effector functions of antibody-dependent cellular cytotoxicity (ADCC) and complement-dependent cytotoxicity (CDC).

ADCC refers to a cell-mediated reaction in which nonspecific cytotoxic cells that express Fc receptors (FcRs) (e.g. Natural Killer (NK) cells, neutrophils, and macrophages) recognize the so-called Fc portion of binding molecules while the latter bind to a target cell and subsequently cause lysis of the target cell. CDC refers to the ability of a molecule to lyse a target in the presence of complement. The complement activation pathway is initiated by the binding of the first component of the complement system (Clq) to a binding molecule complexed with a cognate antigen. To distinguish cell death induced by antibody-dependent cell-mediated/cellular cytotoxicity (ADCC) or complement-dependent cytotoxicity (CDC), cell death may be determined *in vitro* in the absence of complement and immune effector cells. The assay for cell death may for instance be performed using heat inactivated serum (*i.e.* in the absence of complement) and in the absence of immune effector cells. To determine whether the binding molecule is able to induce cell death, loss of membrane integrity as evaluated by uptake of propidium iodide (PI), trypan blue or 7AAD can be assessed relative to untreated cells.

Naked antibodies according to the invention may also induce apoptosis of target cells in another way than by means of ADCC or CDC. Target cells include, but are not limited to, CD1a positive cells such as neoplastic cells. Methods of measuring apoptosis are known to a skilled artisan and include, but are not limited to, FACS analysis using Annexin V staining, DNA electrophoresis, uptake of propidium iodide (PI), trypan blue or 7AAD.

Naked binding molecules according to the invention may also be used to inhibit or block the binding of another molecule, such as a ligand, normally binding to CD1a. This way the human binding molecules could interfere with one or more possible downstream processes which are triggered/activated by the binding/interaction of the molecule to CD1a.

Alternatively, upon binding to CD1a molecules present on the surface of target cells, the human binding molecules as defined herein may internalise. Internalisation of binding molecules can be assayed by known techniques that include, but are not limited to, specifically tracing internalised binding molecules capable of binding CD1a molecules. The binding molecules may be labelled with a fluorochrome and internalisation may be measured by means of flow cytometry or confocal scanning laser microscopy.

In case the human binding molecules as defined in the present invention are slowly internalising and, before internalisation, stay bound to the surface of target cells for a prolonged period of time, they may be useful, similarly as binding molecules that do not internalise at all, in therapies which make use of ADCC, CDC, apoptosis or antibody-directed enzyme-prodrug therapy (ADEPT). ADCC, CDC and apoptosis are discussed above, while ADEPT is discussed below.

The human binding molecules according to the invention comprise a variable heavy chain comprising the amino acid sequence shown in SEQ ID NO:8 and a variable light chain comprising the amino acid sequence of SEQ ID NO:20. . Plasmids comprising DNA encoding the heavy chain or light chain of human IgG1 antibodies directed against human CD1a, said antibodies being called 02-113, 02-114, 02-115, 02-116, 02-117 and 02-118, have been deposited. The plasmids comprising DNA encoding the anti-CD1a human IgG1 heavy chains were called pgG102-113C03, pgG102-114C03, pgG102-115C03, pgG102-116C03 pgG102-117C03 and pgG102-118C03 and were deposited at the European collection of Cell Cultures (ECACC), CAMR, Salisbury, Wiltshire SP4 OJG, Great Britain on 28 October 2003, under accession numbers 03102801, 03102802, 03102803, 03102804, 03102805 and 03102806, respectively. The plasmid comprising DNA encoding the light chain of the anti-CD1a human IgG1's called 02-113, 02-114, 02-116, 02-117 and 02-118 was called pSyn-C05-VkI and was deposited at the European Collection of Cell Cultures (ECACC), CAMR, Salisbury, Wiltshire SP4 OJG, Great Britain on 28 October 2003, under accession number 03102807. The plasmid comprising DNA encoding the light chain of the anti-CD1a human IgG1's called 02-115 was called pSyn-C04-V13 and was deposited at the European Collection of Cell Cultures (ECACC), CAMR, Salisbury, Wiltshire SP4 OJG, Great Britain on 28 October 2003, under accession number 03102808.

Another aspect of the invention includes functional variants of binding molecules or fragments thereof as defined herein. Molecules are considered to be functional variants of a binding molecule according to the invention, if the variants are capable of competing for specifically binding to human CD1a, preferably competing for the same binding site on the human CD1a, with the parent binding molecules. In other words, when the functional variants are still capable of binding to human CD1a or a portion thereof. Functional variants include, but are not limited to, derivatives that are substantially similar in primary structural sequence, but which contain *e.g. in vitro* or *in vivo* modifications, chemical and/or biochemical, that are not found in the parent binding molecule. Such modifications include *inter alia* acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma-carboxylation, glycosylation, GPI-anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, ubiquitination, and the like.

Alternatively, functional variants can be binding molecules as defined in the present invention comprising an amino acid sequence containing substitutions, insertions, deletions or combinations thereof of one or more amino acids compared to the amino acid sequences of the parent binding molecules. Furthermore, functional variants can comprise truncations of the amino acid sequence at either or both the amino or carboxy termini. Functional variants according to the invention may have the same or different, either higher or lower, binding affinities compared to the parent binding molecule but are still capable of binding to human CD1a molecules present on *e.g.* a cell. For instance, functional variants according to the invention may have increased or decreased binding affinities for human CD1a compared to the parent binding molecules. Preferably, the amino acid sequences of the variable regions, including, but not limited to, framework regions, hypervariable regions, in particular the CDR3 regions, are modified. Generally, the light chain and the heavy chain variable regions comprise three hypervariable regions, comprising three CDRs, and more conserved regions, the so-called framework regions (FRs). The hypervariable regions comprise amino acid residues from CDRs and amino acid residues from hypervariable loops. Functional variants intended to fall within the scope of the present invention have at least about 50% to about 99%, preferably at least about 60% to about 99%, more preferably at least about 70% to about 99%, even more preferably at least about 80% to about 99%, most preferably at least about 90% to about 99%, in particular at least about 95% to about 99%, and in particular at least about 97% to about 99% amino acid sequence homology with the parent binding molecules as defined herein. Computer algorithms such as *inter alia* Gap or Bestfit known to a person skilled in the art can be used to optimally align amino acid sequences to be compared and to define similar or identical amino acid residues.

Functional variants can be obtained by altering the parent binding molecules or parts thereof by general molecular biology methods known in the art including, but not limited to, error-prone PCR, oligonucleotide-directed mutagenesis and site-directed mutagenesis.

In yet a further aspect; the invention includes immunoconjugates, *i.e.* molecules comprising at least one human binding molecule as defined herein and further comprising at least one tag, such as a therapeutic moiety that inhibits or prevents the function of cells and/or causes destruction of cells. Also contemplated in the present invention are mixtures of immunoconjugates according to the invention or mixtures of at least one immunoconjugates according to the invention and another molecule, such as a therapeutic agent or another binding molecule or immunoconjugate. In a further embodiment, the immunoconjugates of the invention may comprise more than one tag. These tags can be the same or distinct from each other and can be joined/conjugated non-covalently to the binding molecules. The tags can also be joined/conjugated directly to the binding molecules through covalent bonding, including, but not limited to, disulfide bonding, hydrogen bonding, electrostatic bonding, recombinant fusion and conformational bonding. Alternatively, the tags can be joined/conjugated to the binding molecules by means of one or more linking compounds. Techniques for conjugating tags to binding molecules are well known, see *e.g.* Arnon et al., Monoclonal Antibodies For Immunotargeting Of Drugs In Cancer Therapy, p. 243-256 in Monoclonal Antibodies And Cancer Therapy (1985), Edited by: Reisfeld et al., A. R. Liss, Inc.; Hellstrom et al., Antibodies For Drug Delivery, p. 623-653 in Controlled Drug Delivery, 2nd edition (1987), Edited by: Robinson et al., Marcel Dekker, Inc.; Thorpe, Antibody Carriers Of Cytotoxic Agents, p. 475-506 In Cancer Therapy: A Review, in Monoclonal Antibodies'84 : Biological And Clinical Applications (1985), Edited by: Pinchera *et al.*; Analysis, Results, And Future Prospective Of The Therapeutic Use Of Radiolabeled Antibody In Cancer Therapy, p. 303-316 in Monoclonal Antibodies For Cancer Detection And Therapy (1985), Edited by: Baldwin et al., Academic Press.

Tags according to the invention include, but are not limited to, toxic substances, radioactive substances, liposomes, enzymes, polynucleotide sequences, plasmids, proteins, peptides or combinations thereof. Toxic substances include, but are not limited to, cytotoxic agents, such as small molecule toxins or chemotherapeutic agents, or enzymatically active toxins of bacterial, fungal, plant or animal origin, or fragments thereof. Examples of cytotoxic agents include, but are not limited to, alkylating agents such as thiotepa and cyclophosphamide; alkyl sulfonate such as busulfan, improsulfan and piposulfan; aziridines such as benzodopa, carboquone, meturedopa, and uredopa; ethylenimines and methylamelamines such as altretamine, triethylenemelamine, trietylenephosphoramide, triethylenethiophosphoramide and trimethylolomelamine; nitrogen mustards such as chlorambucil, chlornaphazine, cholophosphamide, estramustine, ifosfamide, mechlorethamine, mechlorethamine oxide hydrochloride, melphalan, novembiehin, phenesterine, prednimustine, trofosfamide, uracil mustard; nitrosoureas such as carmustine, chlorozotocin, fotemustine, lomustine, nimustine, ranimustine; antibiotics such as aclacinomysins, actinomycin, authramycin, azaserine, bleomycin, cactinomycin, calicheamicin, carabicin, carminomycin, carzinophilin, chromoinycins, dactinomycin, daunorubicin, detorubicin, 6-diazo-5-oxo-L-norleucine, doxorubicin, epirubicin, esorubicin, idarubicin, marcellomycin, mitomycin, mycophenolic acid, nogalamycin, olivomycin, peplomycin, potfiromycin, puromycin, quelamycin, rodorubicin, streptonigrin, streptozocin, tubercidin, ubenimex, zinostatin, zorubicin; macrolide antibiotics such as geldanamicin and maytansin, anti-metabolites such as methotrexate and 5-fluorouracil; folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; platinum analogs such as cisplatin and carboplatin; triazenes; epipodophyllotoxins; platinum coordination complexes; maytansinoids; and taxoids, such as paclitaxel and doxetaxel. Pharmaceutically acceptable salts, acids or derivatives of any of the above are also included in the present invention. In general, suitable chemotherapeutic agents are described in Remington's Pharmaceutical Sciences, 18th edition (1990), Edited by: A.R. Gennaro, Mack Publishing Co., Philadelphia and in Goodman and Gilman's The Pharmacological Basis of Therapeutics, 7th edition (1985), Edited by: A.G. Gilman, L.S. Goodman, T.W. Rall and F. Murad. MacMillan Publishing Co., New York. Suitable chemotherapeutic agents that are still in the experimental phase are known to those of skill in the art and might also be used as toxic substances in the present invention.

Examples of enzymatically active toxins of bacterial, fungal, plant or animal origin include, but are not limited to, ricin A chain, modeccin A chain, abrin A chain, *Pseudomonas* exotoxin and endotoxin A chain, shiga toxin A, anthrax toxin lethal factor, diphteria A chain, nonbinding active fragments of diphtheria toxin, staphylococcal enterotoxin A, the human ribonuclease angiogenin, *Aleurites* fordii proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, tricothecenes, saporin, alpha-sarcin, and fragments or derivatives thereof.

Fusion proteins comprising enzymatically active toxins and binding molecules of the immunoconjugate of the invention can be produced by methods known in the art such as, e.g., recombinantly by constructing nucleic acid molecules comprising nucleotide sequences encoding the human binding molecules in frame with nucleotide sequences encoding the enzymatically active toxin and then expressing the nucleic acid molecules. Alternatively, fusion proteins can be produced chemically by conjugating, directly or indirectly via for instance a linker, binding molecules as defined herein to enzymatically active toxins.

Immunoconjugates comprising enzymes may be useful in antibody-directed enzyme-prodrug therapy (ADEPT). In this technique enzymes are conjugated to binding molecules. This conjugation converts the enzymes into inactive prodrugs. The binding molecule-enzyme conjugates are then administered and bind to the binding partner of the binding molecule. After clearance of the conjugates from the circulation, prodrugs are administered, which are converted into active drugs by the enzyme of the conjugates. Passive uptake of the active drugs into the target cells will then occur.

Also contemplated within the present invention are binding molecules of the immunoconjugate of the invention that are labeled with radionuclides. Suitable radionuclides include, but are not limited to, radionuclides that emit alpha radiation such as *inter alia* ²¹²bismuth, ²¹³bismuth and ²¹¹astatine; radionuclides that emit beta radiation such as *inter alia* ¹³¹iodine, ⁹⁰yttrium, ¹⁸⁶rhodium and ¹⁸⁸rhodium; and radionuclides that emit gamma radiation such as *inter alia* ¹³¹iodine, ¹⁸⁶rhodium and ¹⁸⁸rhodium. Suitable radionuclides further include, but are not limited to, Auger-electron-emitting radionuclides such as *inter alia* ¹²³iodine, ¹²⁴iodine, ¹²⁵iodine, ¹²⁹iodine, ¹³¹iodine, ¹¹¹indium, ⁷⁷bromine, and other radiolabeled halogens. The skilled man will appreciate that other suitable radionuclides can also be identified as suitable in the present invention. The choice of radionuclide will be dependent on many factors such as, *e.g.*, the type of disease to be treated, the stage of the disease to be treated, the patient to be treated and the like. Binding molecules can be attached to radionuclides directly or indirectly via a chelating agent by methods well known in the art.

In another embodiment, the binding molecules of the immunoconjugate of the invention can be conjugated to liposomes to produce so-called immunoliposomes. A liposome may be conjugated to one or more binding molecules, the binding molecules being either the same or different. A variety of methods are available for preparing liposomes. These methods are well known in the art and include, but are not limited to, sonication, extrusion, high pressure/homogenization, microfluidisation, detergent dialysis, calcium-induced fusion of small liposome vesicles, and ether-infusion methods. The liposomes may be multilamellar vesicles, but preferably the liposomes are unilamellar vesicles such as small unilamellar (200 - 500 Å) or large unilamellar vesicles (500 - 5000 Å). After preparation, the liposomes which have not been sized during formation may be sized by methods known in the art to achieve a desired size range and relatively narrow distribution of liposome sizes. The methods of loading drugs into liposomes are well known to those of skill in the art. The most common methods include the encapsulation technique and the transmembrane potential loading method. In the encapsulation technique, the drugs and liposome components are dissolved in an organic solvent or mixture of solvents in which all species are miscible, and then concentrated to a dry film. A buffer is then added to the dried film and liposomes are formed having the drugs incorporated into the vesicle walls. This method has been described in detail in U.S. Patent Numbers 4,885,172, 5,059,421, and 5,171,578 . The transmembrane potential loading method has been described in detail in U.S. Patent Numbers 4,885,172, 5,059,421, 5,171, 578, 5,316,771 and 5,380,531 . As will be understood, the loading techniques are not limited to these two general loading techniques.

The drugs that can be loaded into liposomes include, but are not limited to, the toxic substances mentioned above. Liposomes having loaded different drugs and different liposomes, each liposome having loaded one kind of drug, may be alternative embodiments of liposomes that can be used and these embodiments are therefore also contemplated in the present invention. Human banding molecules of the invention may be attached at the surface of the liposomes or to the terminus of polymers such as polyethylene glycol that are grafted at the surface of the liposomes using conventional chemical-coupling techniques. An advantage of immunoliposomes is the ability to deliver several tens of thousands of drug molecules with a few tens of binding molecules per liposome resulting in high drug to binding molecule ratios. Following binding of the immunoliposomes to the target cells the drug can either, in case of binding molecules that are slowly internalised or not internalised at all, be gradually released from the immunoliposomes and taken up by the cells as a free drug using standard uptake mechanisms or, in case of binding molecules that are rapidly internalised, the immunoliposomes themselves are taken up by the target cells by receptor-mediated endocytosis and the drugs are gradually released within the cells.

In yet another embodiment, the human binding molecules of the invention may be linked to water-soluble, biodegradable polymers, such as for instance polymers of hydroxypropylmethacrylamine (HPMA). The polymers have toxic substances linked on separate sites of the polymers with the use of appropriate degradable spacers to allow for release of the toxic substances. The above described polymers are also called immunopolymers.

In another aspect the human binding molecules of the invention may be conjugated/attached to one or more antigens. Preferably, these antigens are antigens which are recognised by the immune system of a subject to which the binding molecule-antigen conjugate is administered. The antigens may be identical but may also be different. Conjugation methods for attaching the antigens and binding molecules are well known in the art and include, but are not limited to, the use of cross-linking agents. The human binding molecules will bind to the cells comprising human CD1a and the antigens attached to the binding molecules will initiate a powerful T-cell attack on the conjugate which will eventually lead to the destruction of the cell.

Alternatively, the human binding molecules as described in the present invention can be conjugated to tags and be used for detection and/or analytical and/or diagnostic purposes. The tags used to label the binding molecules for those purposes depend on the specific detection/analysis/diagnosis techniques and/or methods used such as *inter alia* immunohistochemical staining of tissue samples, flow cytometric detection, scanning laser cytometric detection, fluorescent immunoassays, enzyme-linked immunosorbent assays (ELISA's), radioimmunoassays (RIA's), bioassays (e.g., growth inhibition assays), Western blotting applications, etc. For immunohistochemical staining of tissue samples preferred labels are enzymes that catalyze production and local deposition of a detectable product. Enzymes typically conjugated to binding molecules to permit their immunohistochemical visualization are well-known and include, but are not limited to, alkaline phosphatase, P-galactosidase, glucose oxidase, horseradish peroxidase, and urease. Typical substrates for production and deposition of visually detectable products include, but are not limited to, o-nitrophenyl-beta-D-galactopyranoside (ONPG), o-phenylenediamine dihydrochloride (OPD), p-nitrophenyl phosphate (PNPP), p-nitrophenyl-beta-D-galactopryanoside (PNPG), 3', 3'diaminobenzidine (DAB), 3-amino-9-ethylcarbazole (AEC), 4-chloro-1-naphthol (CN), 5-bromo-4-chloro-3-indolyl-phosphate (BCIP), ABTS, BluoGal, iodonitrotetrazolium (INT), nitroblue tetrazolium chloride (NBT), phenazine methosulfate (PMS), phenolphthalein monophosphate (PMP), tetramethyl benzidine (TMB), tetranitroblue tetrazolium (TNBT), X-Gal, X-Gluc, and X-glucoside. Other substrates that can be used to produce products for local deposition are luminescent substrates. For example, in the presence of hydrogen peroxide, horseradish peroxidase can catalyze the oxidation of cyclic diacylhydrazides such as luminol. Next to that, binding molecules of the immunoconjugate of the invention can also be labeled using colloidal gold or they can be labeled with radioisotopes, such as ³³p, ³²p, ³⁵S, ³H, and ¹²⁵I. When the binding molecules of the present invention are used for flow cytometric detections, scanning laser cytometric detections, or fluorescent immunoassays, they can usefully be labeled with fluorophores. A wide variety of fluorophores useful for fluorescently labeling the binding molecules of the present invention include, but are not limited to, Alexa Fluor and Alexa Fluor&commat dyes, BODIPY dyes, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethylrhodamine, Cy2, Cy3, Cy3.5, Cy5, Cy5.5, Cy7, fluorescein isothiocyanate (FITC), allophycocyanin (APC), R-phycoerythrin (PE), peridinin chlorophyll protein (PerCP), Texas Red, fluorescence resonance energy tandem fluorophores such as PerCP-Cy5.5, PE-Cy5, PE-Cy5.5, PE-Cy7, PE-Texas Red, and APC-Cy7. When the binding molecules of the present invention are used for secondary detection using labeled avidin, streptavidin, captavidin or neutravidin, the binding molecules may be labeled with biotin.

Next to that, the human binding molecules of the invention may be conjugated to photoactive agents or dyes such as fluorescent and other chromogens or dyes to use the so obtained immunoconjugates in photoradiation, phototherapy, or photodynamic therapy. The photoactive agents or dyes include, but are not limited to, photofrin.RTM, synthetic diporphyrins and dichlorins, phthalocyanines with or without metal substituents, chloroaluminum phthalocyanine with or without varying substituents, O-substituted tetraphenyl porphyrins, 3,1-meso tetrakis (o-propionamido phenyl) porphyrin, verdins, purpurins, tin and zinc derivatives of octaethylpurpurin, etiopurpurin, hydroporphyrins, bacteriochlorins of the tetra(hydroxyphenyl) porphyrin series, chlorins, chlorin e₆, mono-1-aspartyl derivative of chlorin e₆, di-1-aspartyl derivative of chlorin e₆, tin(IV) chlorin e₆, metatetrahydroxyphenylchlor- in, benzoporphyrin derivatives, benzoporphyrin monoacid derivatives, tetracyanoethylene adducts of benzoporphyrin, dimethyl acetylenedicarboxylate adducts of benzoporphyrin, Diels-Adler adducts, monoacid ring "a" derivative of benzoporphyrin, sulfonated aluminum PC, sulfonated AlPc, disulfonated, tetrasulfonated derivative, sulfonated aluminum naphthalocyanines, naphthalocyanines with or without metal substituents and with or without varying substituents, anthracenediones, anthrapyrazoles, aminoanthraquinone, phenoxazine dyes, phenothiazine derivatives, chalcogenapyrylium dyes, cationic selena and tellurapyrylium derivatives, ring-substituted cationic PC, pheophorbide derivative, naturally occurring porphyrins, hematoporphyrin, ALA-induced protoporphyrin IX, endogenous metabolic precursors, 5-aminolevulinic acid benzonaphthoporphyrazines, cationic imminium salts, tetracyclines, lutetium texaphyrin, tin-etio-purpurin, porphycenes, benzophenothiazinium and combinations thereof.

When the immunoconjugates of the invention are used for *in vivo* diagnostic use, the human binding molecules can also be made detectable by conjugation to *e.g.* magnetic resonance imaging (MRI) contrast agents, such as gadolinium diethylenetriaminepentaacetic acid, to ultrasound contrast agents or to X-ray contrast agents, or by radioisotopic labeling.

Furthermore, the human binding molecules or immunoconjugates of the invention can also be attached to solid supports, which are particularly useful for immunoassays or purification of the binding partner. Such solid supports might be porous or nonporous, planar or nonplanar and include, but are not limited to, glass, cellulose, polyacrylamide, nylon, polystyrene, polyvinyl chloride or polypropylene supports. The binding molecules can also for example usefully be conjugated to filtration media, such as NHS-activated Sepharose or CNBr-activated Sepharose for purposes of immunoaffinity chromatography. They can also usefully be attached to paramagnetic microspheres, typically by biotinstreptavidin interaction. The microspheres can be used for isolation of cells that express or display human CD1a or fragments thereof. As another example, the human binding molecules of the present invention can usefully be attached to the surface of a microtiter plate for ELISA.

Another aspect of the present invention concerns nucleic acid molecules as defined herein encoding human binding molecules of the present invention. In yet another aspect, the invention provides nucleic acid molecules encoding at least the human binding molecules specifically binding to human CD1a. In a preferred embodiment, the nucleic acid molecules are isolated or purified.

The skilled man well appreciate that functional variants of these nucleic acid molecules are also intended to be a part of the present invention. Functional variants are nucleic acid sequences that can be directly translated, using the standard genetic code, to provide an amino acid sequence identical to that translated from the parent nucleic acid molecules,

Preferably the nucleic acid molecules encode binding molecules comprising a Variable heavy chain comprising the amino acid sequence of SEQ ID NO:8 and a variable light chain comprising the amino acid sequence of SEQ ID NO:20.

In a specific embodiment of the invention the nucleic acid molecules encoding the binding molecules of the invention comprise a nucleotide sequence selected from the group consisting of SEQ ID NO:7, SEQ ID NO:9, SEQ ID NO:11, SEQ ID NO:13, SEQ ID NO:15 and SEQ ID NO:17.

In a specific embodiment of the present invention, the nucleic acid molecules encoding the binding molecules of the invention comprise the nucleotide sequence of SEQ ID NO:7 and SEQ ID NO:19

It is another aspect of the invention to provide vectors, *i.e*. nucleic acid constructs, comprising one or more nucleic acid molecules according to the present invention. Vectors can be derived from plasmids such as *inter alia* F, R1, RP1, Col, pBR322, TOL, Ti, etc; cosmids; phages such as lambda, lambdoid, M13, Mu, P1, P22, Qβ, T-even, T-odd, T2, T4, T7, etc; plant viruses such as *inter alia* alfalfa mosaic virus, bromovirua, capillavirus, carlavirus, carmovirus, caulivirus, clostervirus, comovirus, cryptovirus, cucumovirus, dianthovirus, fabavirus, fijivirus, furovirus, geminivirus, hordeivirus, ilarvirus, luteovirus, machlovirus, marafivirus, necrovirus, nepovirus, phytorepvirus, plant rhabdovirus, potexvirus, potyvirus, sebemovirus, tenuivirus, tobamovirus, tobravirus, tomato spotted wilt virus, tombusvirus, tymovirus, etc; or animal viruses such as i*nter alia* adenovirus, arenaviridae, baculoviridae, birnaviridae, bunyaviridae, calciviridae, cardioviruses, coronaviridae, corticoviridae, cystoviridae, Epstein-Barr virus, enteroviruses, filoviridae, flaviviridae, Foot-and-Mouth disease virus, hepadnaviridae, hepatitis viruses, herpesviridae, immunodeficiency viruses, influenza virus, inoviridae, iridoviridae, orthomyxoviridae, papovaviruses, paramyroviridae, parvoviridae, picornaviridae, poliovirus, polydnaviridae, poxviridae, reoviridae, retroviruses, rhabdoviridae, rhinoviruses, Semliki Forest virus, tetraviridae, togaviridae, toroviridae, vaccinia virus, vascular atomatitis virus, etc. Vectors can be used for cloning and/or for expression of the binding molecules of the invention and might even be used for gene therapy purposes. Vectors comprising one or more nucleic acid molecules according to the invention operably linked to one or more expression-regulating nucleic acid molecules are also covered by the present invention. The choice of the vector is dependent on the recombinant procedures followed and the host used. Introduction of vectors in host cells can be effected by *inter alia* calcium phosphate transfection, virus infection, DEAE-dextran mediated transfection, lipofectamin transfection or electroporation. Vectors may be autonomously replicating or may replicate together with the chromosome into which they have been integrated. Preferably, the vectors contain one or more selection markers. The choice of the markers may depend on the host cells of choice, although this is not critical to the invention as is well known to persons skilled in the art. They include, but are not limited to, kanamycin, neomycin, puromycin, hygromycin, zeocin, thymidine kinase gene from Herpes simplex virus (HSV-TK), dihydrofolate reductase gene from mouse (dhfr). Vectors comprising one or more nucleic acid molecules encoding the binding molecules as described above operably linked to one or more nucleic acid molecules encoding proteins or peptides that can be used to isolate the binding molecules are also covered by the invention. These proteins or peptides include, but are not limited to, glutathione-S-transferase, maltose binding protein, metal-binding polyhistidine, green fluorescent protein, luciferase and beta-galactosidase.

Hosts containing one or more copies of the vectors mentioned above are an additional subject of the present invention. Preferably, the hosts are host cells. Host cells include, but are not limited to, cells of mammalian, plant, insect, fungal or bacterial origin. Bacterial cells include, but are not limited to, cells from Gram positive bacteria such as several species of the genera *Bacillus, Streptomyces* and *Staphylococcus* or cells of Gram negative bacteria such as several species of the genera *Escherichia* and *Pseudomonas.* In the group of fungal cells preferably yeast cells are used. Expression in yeast can be achieved by using yeast strains such as *inter alia Pichia pastoris, Saccharomyces cerevisiae* and *Hansenula polymorpha.* Furthermore, inspect cells such as cells from Drosophila and Sf9 can be used as host cells. Besides that, the host cells can be plant cells such as *inter alia* cells from crop plants such as forestry plants, or cells from plants providing food and raw materials such as cereal plants, or medicinal plants, or cells from ornamentals, or cells from flower bulb crops. Transformed (transgenic) plants or plant cells are produced by known methods, for example, Agrobacterium-mediated gene transfer, transformation of leaf discs, protoplast transformation by polyethylene glycol-induced DNA transfer, electroporation, sonication, microinjection or bolistic gene transfer. Additionally, a suitable expression system can be a baculovirus system. Expression systems using mammalian cells such as Chinese Hamster Ovary (CHO) cells, COS cells, BHK cells or Bowes melanoma cells are preferred in the present invention. Mammalian cells provide expressed proteins with posttranslational modifications that are most similar to natural molecules of mammalian origin. Since the present invention deals with molecules that may have to be administered to humans, a completely human expression system would be particularly preferred. Therefore, even more preferably, the host cells are human cells. Examples of human cells are *inter alia* HeLa, 911, AT1080, A549, 293 and HEK293T cells. Preferred mammalian cells are human embryonal retinoblasts such as 911 cells or the cell line deposited at the European Collection of Cell Cultures (ECACC), CAMR, Salisbury, Wiltshire SP4 OJG, Great Britain on 29 February 1996 under number 96022940 and marketed under the trademark PER.C6 (PER.C6 is a pending trademark of Crucell Holland B.V.). In preferred embodiments, the human producer cells comprise at least a functional part of a nucleic acid sequence encoding an adenovirus E1 region in expressible format. In even more preferred embodiments, said host cells are derived from a human retina and immortalised with nucleic acids comprising adenoviral E1 sequences, such as the cell line deposited at the European Collection of Cell Cultures (ECACC), CAMR, Salisbury, Wiltshire SP4 OJG, Great Britain on 29 February 1996 under number 96022940 and marketed under the trademark PER.C6™, and derivatives thereof. Production of recombinant proteins in host cells can be performed according to methods well known in the art. The use of the cells marketed under the trademark PER.C6™ as a production platform for proteins of interest has been described in WO 00/63403 .

In yet another embodiment, human binding molecules of the present invention can also be produced in transgenic, non-human, mammals such as *inter alia* rabbits, goats or cows, and secreted into for instance the milk thereof.

It is another aspect of the invention to provide a method of producing human binding molecules or functional variants thereof according to the present invention. The method comprises the steps of a) culturing a host as described in the present invention under conditions conducive to the expression of the human binding molecules, and optionally, b) recovering the expressed human binding molecules. The expressed human binding molecules can be recovered from the cell free extract, but preferably they are recovered from the culture medium. Methods to recover proteins, such as binding molecules, from cell free extracts or culture medium are well known to the man skilled in the art. Human binding molecules as obtainable by the above described method are also a part of the present invention.

Alternatively, next to the expression in hosts, such as host cells, the human binding molecules of the invention can be produced synthetically by conventional peptide synthesizers or in cell-free translation systems using RNA's derived from DNA molecules according to the invention. Human binding molecule as obtainable by the above described synthetic production methods or cell-free translation systems are also a part of the present invention.

In yet another alternative embodiment, human binding molecules according to the present invention may be generated by transgenic non-human mammals, such as for instance transgenic mice or rabbits, that express human immunaglobulin genes. Preferably, the transgenic non-human mammals have a genome comprising a human heavy chain transgene and a human light chain transgene encoding all or a portion of the human binding molecules as described above. The transgenic non-human mammals can be immunized with a purified or enriched preparation of human CD1a or a fragment thereof and/or cells expressing human CD1a molecules. Protocols for immunizing non-human mammals are well established in the art. See Using Antibodies: A Laboratory Manual, Edited by: E. Harlow, D. Lane (1998), Cold Spring Harbor Laboratory, Cold Spring Harbor, New York and Current Protocols in Immunology, Edited by: J.E. Coligan, A.M. Kruisbeek, D.H. Margulies, E.M. Shevach, W. Strober (2001), John Wiley & Sons Inc., New York, Immunization protocols often include multiple immunizations, either with or without adjuvants such as Freund's complete adjuvant and Freund's incomplete adjuvant, but may also include naked DNA immunizations. In another embodiment, the human binding molecules are produced by B cells or plasma cells derived from the transgenic animals. In yet another embodiment, the human binding molecules are produced by hybridomas which are prepared by fusion of B cells obtained from the above described transgenic non-human mammals to immortalized cells. B cells, plasma cells and hybridomas as obtainable from the above describes transgenic non-human mammals and human binding molecules as obtainable from the above described transgenic non-human mammals, B cells, plasma cells and hybridomas are also a part of the present invention.

In a further aspect, the invention provides a method of identifying human binding molecules according to the invention or nucleic acid molecules according to the invention and comprises the steps of a) contacting a phage library of human binding molecules with material comprising human CD1a or a part thereof, b) selecting at least once for a phage binding to the material comprising human CD1a or a part thereof, and c) separating and recovering the phage binding to the material comprising human CD1a or a part thereof. Material comprising human CD1a can be, *e.g.* cells transfected with human CD1a expression plasmids, isolated human CP1a, the extracellular part of human CD1a, fusion proteins comprising human CD1a or a part thereof, and the like. Phage display methods for identifying and obtaining binding molecules, *e.g.* antibodies, are by now well-established methods known by the person skilled in the art. They are *e.g.* described in US Patent Number 5,696,108; Burton and Barbas (1994); and de Kruif *et al*. (1995b). For the construction of phage display libraries, collections of human monoclonal antibody heavy and light chain variable region genes are expressed on the surface of bacteriophage, preferably filamentous bacteriophage, particles, in for example single chain Fv (scFv) or in Fab format (de Kruif *et al*., 1995b). Large libraries of antibody fragment-expressing phages typically contain more than 1.0*10⁹ antibody specificities and may be assembled from the immunoglobulin V regions expressed in the B lymphocytes of immunized- or non-immunized individuals. Alternatively, phage display libraries may be constructed from immunoglobulin variable regions that have been partially assembled *in vitro* to introduce additional antibody diversity in the library (semi-synthetic libraries). For example, *in vitro* assembled variable regions contain stretches of synthetically produced, randomized or partially randomized DNA in those regions of the molecules that are important for antibody specificity, *e.g.* CDR regions. Antigen specific phage antibodies can be selected from the library by immobilising target antigens such as human CD1a molecules or fragments thereof on a solid phase and subsequently exposing the target antigens to a phage library to allow binding of phages expressing antibody fragments specific for the solid phase-bound antigen. Non-bound phages are removed by washing and bound phages eluted from the solid phase for infection of *Escherichia coli* (*E. coli*) bacteria and subsequent propagation. Multiple rounds of selection and propagation are usually required to sufficiently enrich for phages binding specifically to the target antigen. Phages may also be selected for binding to complex antigens such as complex mixtures of proteins or whole cells such as cells transfected with human CD1a expression plasmids or cells naturally expressing human CD1a. Selection of antibodies on whole cells has the advantage that target antigens are presented in their native configuration, *i.e.* unperturbed by possible conformational changes that might have been introduced in the case where an antigen is immobilized to a solid phase. Antigen specific phage antibodies can be selected from the library by incubating a cell population of interest, expressing known and unknown antigens on their surface, with the phage antibody library to let for example the scFv or Fab part of the phage bind to the antigens on the cell surface. After incubation and several washes to remove unbound and loosely attached phages, the cells of interest are stained with specific fluorescent labeled antibodies and separated on a Fluorescent Activated Cell Sorter (FACS). Phages that have bound with their scFv or Fab part to these cells are eluted and used to infect *Eacherichia coli* to allow amplification of the new specificity. Generally, one or more selection rounds are required to separated the phages of interest from the large excess of non-binding phages. Monoclonal phage preparation can be analyzed for their specific staining patterns and allowing identification of the antigen being recognized (de Kruif *et al*., 1995a). The phage display method can be extended and improved by subtracting non-relevant binders during screening by addition of an excess of non-target molecules that are similar, but not identical, to the target, and thereby strongly enhance the chance of finding relevant binding molecules (This process is referred to as the Mabstract^{™} process. Mabstract^{™} is a pending trademark application of Crucell Holland B.V., see also US Patent Number 6, 265, 150). Alternatively, one or more subtraction steps can be performed before or after screening.

In yet a further aspect, the invention provides a method of obtaining a human binding molecule or a nucleic acid molecule according to the invention, wherein the method comprises the steps of a) performing the above described method of identifying human binding molecules according to the invention or nucleic acid molecules according to the invention, and b) isolating from the recovered phage the human binding molecule and/or the nucleic acid encoding the human binding molecule. Once a new monoclonal phage antibody has been established or identified with the above mentioned method of identifying human binding molecules or nucleic acid molecules encoding the human binding molecules, the DNA encoding the scFv or Fab can be isolated from the bacteria or phages and combined with standard molecular biological techniques to make constructs encoding bivalent scFv's or complete human immunoglobulins of a desired specificity (*e.g*. IgG, IgA or IgM). These constructs can be transfected into suitable cell lines and complete human monoclonal antibodies can be produced (Huls *et al.*, 1999; Boel *et al.*, 2000).

In a further aspect, the invention provides compositions comprising at least one human binding molecule, at least one functional variant or fragment thereof, at least one immunoconjugate according to the invention or a combination thereof. In addition to that, the compositions may comprise *inter alia* stabilising molecules, such as albumin or polyethylene glycol, or salts. Preferably, the salts used are salts that retain the desired biological activity of the human binding molecules and do not impart any undesired toxicological effects. Examples of such salts include, but are not limited to, acid addition salts and base addition salts. Acid addition salts include, but are not limited to, those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include, but are not limited to, those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like. If necessary, the binding molecules of the invention may be coated in or on a material to protect them from the action of acids or other natural or non-natural conditions that may inactivate the human binding molecules.

In yet a further aspect, the invention provides compositions comprising at least one nucleic acid molecule as defined in the present invention. The compositions may comprise aqueous solutions such as aqueous solutions containing salts (*e.g*., NaCl or salsts as described above), detergents (*e.g.,* SDS) and/or other suitable components.

Furthermore, the present invention pertains to pharmaceutical compositions comprising at least one human binding molecule according to the invention, at least one functional variant or fragment thereof, at least one immunoconjugate according to the invention, at least one composition according to the invention, or combinations thereof. The pharmaceutical composition of the invention further comprises at least one pharmaceutically acceptable excipient. A pharmaceutical composition according to the invention can further comprise at least one other therapeutic, prophylactic and/or diagnostic agent.

Typically, pharmaceutical compositions must be sterile and stable under the conditions of manufacture and storage. The human binding molecules, variant or fragments thereof, immunoconjugates, nucleic acid molecules or compositions of the present invention can be in powder form for reconstitution in the appropriate pharmaceutically acceptable excipient before or at the time of delivery. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Alternatively, the human binding molecules, variant or fragments thereof, immunoconjugates, nucleic acid molecules or compositions of the present invention can be in solution and the appropriate pharmaceutically acceptable excipient can be added and/or mixed before or at the time of delivery to provide a unit dosage injectable form. Preferably, the pharmaceutically acceptable excipient used in the present invention is suitable to high drug concentration, can maintain proper fluidity and, if necessary, can delay absorption.

The choice of the optimal route of administration of the pharmaceutical compositions will be influenced by several factors including the physico-chemical properties of the active molecules within the compositions, the urgency of the clinical situation and the relationship of the plasma concentrations of the active molecules to the desired therapeutic effect. For instance, if necessary, the human binding molecules of the invention can be prepared with carriers that will protect them against rapid release, such as a controlled release formulation, including implants, transdermal patches, and microencapsulated delivery systems. Biodegradable, biocompatible polymers can *inter alia* be used, such as ethylene vinyl acetate, polyanhydrides, polyglycolic acid, collagen, polyorthoesters, and polylactic acid. Furthermore, it may be necessary to coat the human binding molecules with, or co-administer the human binding molecules with, a material or compound that prevents the inactivation of the human binding molecules. For example, the human binding molecules may be administered to a subject in an appropriate carrier, for example, liposomes, or a diluent.

The routes of administration can be divided into two main categories, oral and parenteral administration. These two categories include, but are not limited to, bolus, buccal, epidermal, epidural, inhalation, intra-abdominal, intraarterial, intra-articular, intrabronchial, intracapsular, intracardiac, intracartilaginous, intracavitary, intracelial, intracelebellar, intracerebronventricular, intracolic, intracervical, intradermal, intragastric, intrahepatic, intramedullary, intramuscular, intramyocardial, intranasal, intra-ocular intra-orbital, intra-osteal, intrapelvic, intrapericardiac, intraperitoneal, intraplaque, intrapleural, intraprostatic, intrapulmonary, intrarectal, intrarenal, intraretinal, intraspinal, intrasternal, intrasynovial, intrathecal, intrathoracic, intratumoral, intra-uterine, intravenous, intraventricular, intravesical, rectal, spinal, subarachnoid, subcapsular, subcutaneous, subcuticular, sublingual, topical, transdermal, transmucosal, transtracheal, and vaginal administration. The preferred administration route is intravenous.

Oral dosage forms can be formulated *inter alia* as tablets, troches, lozenges, aqueous or oily suspensions, dispersable powders or granules, emulsions, hard capsules, soft gelatin capsules, syrups or elixirs, pills, dragees, liquids, gels, or slurries. These formulations can contain pharmaceutically excipients including, but not limited to, inert diluents such as calcium carbonate, sodium carbonate, lactose, calcium phosphate or sodium phosphate; granulating and disintegrating agents such as corn starch or alginic acid; binding agents such as starch, gelatin or acacia; lubricating agents such as calcium stearate, glyceryl behenate, hydrogenated vegatable oils, magnesium stearate, mineral oil, polyethylene glycol, sodium stearyl, fumarate, stearic acid, talc, zinc stearate; preservatives such as n-propyl-p-hydroxybenzoate; colouring, flavouring or sweetening agents such as sucrose, saccharine, glycerol, propylene glycol or sorbitol; vegetable oils such as arachis oil, olive oil, sesame oil or coconut oil; mineral oils such as liquid parrafin; wetting agents such as benzalkonium chloride, docusate sodium, lecithin, poloxamer, sodium lauryl sulfate, sorbitan esters; and thickening agents such as agar, alginic acid, beeswax, carboxymethyl cellulose calcium, carageenan, dextrin or gelatin.

The pharmaceutical compositions of the present invention can also be formulated for parenteral administration. Formulations for parenteral administration can be *inter alia* in the form of aqueous or non-aqueous isotonic sterile non-toxic injection or infusion solutions or suspensions. Preferred parenteral administration routes include intravenous, intraperitoneal, epidural, intramuscular and intratumoral injection or infusion. The solutions or suspensions may comprise agents that are non-toxic to recipients at the dosages and concentrations employed such as 1,3-butanediol, Ringer's solution, Hank's solution, isotonic sodium chloride solution, oils such as synthetic mono- or diglycerides or fatty acids such as oleic acid, local anaesthetic agents, preservatives, buffers, viscosity or solubility increasing agents, water-soluble antioxidants such as ascorbic acid, cysteine hydrochloride, sodium bisulfate, sodium metabisulfite, sodium sulfite and the like, oil-soluble antioxidants such as ascorbyl palmitate, butylated hydroxyanisole (BHA), butylated hydroxytoluene (BHT), lecithin, propyl gallate, alpha-tocopherol, and the like, and metal chelating agents, such as citric acid, ethylenediamine tetraacetic acid (EDTA), sorbitol, tartaric acid, phosphoric acid, and the like.

The human binding molecules according to the invention, the variants or fragments thereof, the immunoconjugates according to the invention, the nucleic acid molecules according to the invention, the compositions according to the invention or the pharmaceutical compositions according to the invention can be used as medicaments. They can *inter alia* be used in the diagnosis, prevention, treatment, or combination thereof, of a neoplastic disorder or disease. Preferably, the neoplastic disorder or disease is selected from the group consisting of acute myeloid leukemia (AML), chronic myeloid leukemia (CML), chronic myelogenous leukemia in blast crisis (CML-BC), chronic myelomonocytic leukemia, acute promyelocytic leukemia, myelodysplastic syndrome, juvenile myelomonocytic leukemia, acute lymphoblastic leukemia (ALL), acute non-lymphocytic leukemia (ANLL), T-cell acute lymphoblastic leukemia (T-ALL), large granular lymphocytic leukemia (LGLL), B-cell chronic lymphocytic leukemia (B-CLL) and Langerhans cell histiocytosis. The human binding molecules of the invention are suitable for treatment of yet untreated patients suffering from any of the above disorders and diseases, patients who have been or are treated and are in remission or are not in remission, and patients with a recurrent/refractory diseases or disorders.

The above mentioned molecules or compositions may be employed in conjunction with other molecules useful in diagnosis, prevention and/or treatment. They can be used in *vitro, ex vivo* or *in vivo.* The molecules are typically formulated in the compositions and pharmaceutical compositions of the invention in a therapeutically or diagnostically effective amount. Dosage regimens can be adjusted to provide the optimum desired response (*e.g*., a therapeutic response). For example, a single bolus may be administered, several divided doses may be administered over time or the dose may be proportionally reduced or increased as indicated by the exigencies of the therapeutic situation. The molecules and compositions according to the present invention are preferably sterile. Methods to render these molecules and compositions sterile are well known in the art. The other molecules useful in diagnosis, prevention and/or treatment can be administered in a similar dosage regimen as proposed for the human binding molecules of the invention. If the other molecules are administered separately, they may be administered to a subject with a neoplastic disorder or disease prior (*e.g*., 2 minutes, 5 minutes, 10 minutes, 15 minutes, 30 minutes, 45 minutes, 60 minutes, 2 hours, 4 hours, 6 hours, 8 hours, 10 hours, 12 hours, 14 hours, 16 hours, 18 hours, 20 hours, 22 hours, 24 hours, 2 days, 3 days, 4 days, 5 days, 7 days, 2 weeks, 4 weeks or 6 weeks before) to, concomitantly with, or subsequent (*e.g*., 2 minutes, 5 minutes, 10 minutes, 15 minutes, 30 minutes, 45 minutes, 60 minutes, 2 hours, 4 hours, 6 hours, 8 hours, 10 hours, 12 hours, 14 hours, 16 hours, 18 hours, 20 hours, 22 hours, 24 hours, 2 days, 3 days, 4 days, 5 days, 7 days, 2 weeks, 4 weeks or 6 weeks after) to the administration of one or more of the human binding molecules or pharmaceutical compositions of the invention. The dosing regimen is usually sorted out during clinical trials in human patients.

Human binding molecules and pharmaceutical compositions comprising the human binding molecules are particularly useful, and often preferred, when to be administered to human beings as *in vivo* diagnostic or therapeutic agents, since recipient.immune response to the administered antibody will often be substantially less than that occasioned by administration of a monoclonal murine, chimeric or humanised binding molecule.

Alternatively, cells that are genetically engineered to express the human binding molecules of the invention are administered to patients *in vivo.* Such cells may be obtained from an animal or patient or an MHC compatible donor and can include, but are not limited to fibroblasts, bone marrow cells, blood cells (*e.g*., lymphocytes), adipocytes, muscle cells, endothelial cells, etc. The cells are genetically engineered *in vitro* using recombinant DNA techniques to introduce the nucleic acid molecules of the invention into the cells. Preferably, the human binding molecules are secreted from the cells. The engineered cells which express and preferably secrete the human binding molecules as described herein can be introduced into the patient for example systemically, *e.g*., in the circulation, or intraperitoneally. In other embodiments, the cells can be incorporated into a matrix or can be encapsulated and implanted in the body. In a gene therapy setting the human binding molecules may be administered in the form of a vector capable of infecting cells of the host, coding for a human binding molecule according to the invention.

In another aspect, the invention concerns the use of human binding molecules, fragments or variants thereof, immunoconjugates according to the invention, nucleic acid molecules according to the invention, compositions or pharmaceutical compositions according to the invention in the preparation of a medicament for the diagnosis, prophylaxis, treatment, or combination thereof, of a neoplastic disorder or disease. The neoplastic disease or disorder is preferably selected from the group described above.

Next to that, kits comprising at least one human binding molecule according to the invention, at least one variant or fragment thereof, at least one immunoconjugate according to the invention, at least one nucleic acid molecule according to the invention, at least one composition according to the invention, at least one pharmaceutical composition according to the invention, at least one vector according to the invention, at least one host according to the invention or a combination thereof are also a part of the present invention. Optionally, the above described components of the kits of the invention are packed in suitable containers and labeled for diagnosis, prevention and/or treatment of the indicated conditions. The above-mentioned components may be stored in unit or multi-dose containers, for example, sealed ampoules, vials, bottles, syringes, and test tubes, as an aqueous, preferably sterile, solution or as a lyophilized, preferably sterile, formulation for reconstitution. The containers may be formed from a variety of materials such as glass or plastic and may have a sterile access port (for example the container may be an intravenous solution bag or a vial having a stopper pierceable by a hypodermic injection needle). The kit may further comprise more containers comprising a pharmaceutically acceptable buffer, such as phosphate-buffered saline, Ringer's solution and dextrose solution. It may further include other materials desirable from a commercial and user standpoint, including other buffers, diluents, filters, needles, syringes, culture medium for one or more of the suitable hosts. Associated with the kits can be instructions customarily included in commercial packages of therapeutic, prophylactic and/or diagnostic products, that contain information about for example the indications, usage, dosage, manufacture, administration, contraindications and/or warnings concerning the use of such therapeutic, prophylactic and/or diagnostic products.

In a further aspect, the invention provides a method of detecting human CD1a or a variant or part thereof, wherein the method comprises the steps of a) contacting a sample with a diagnostically effective amount of a human binding molecule of the invention or a functional variant or fragment thereof or an immunoconjugate according to the invention, and b) determining whether the human binding molecule or functional variant or fragment thereof or immunoconjugate specifically binds to a compound of the sample. Methods of detection are known in the art and include, but are not limited to, Western Blot, RIA, ELISA, and immunohistochemical methods.

### EXAMPLES

### Example 1

### In vitro generation of dendritic cells

Cultured dendritic cells (DCs) were obtained by the isolation of monocytes from buffycoats, using Ficoll density gradient centrifugation for granulocyte removal, sheep red blood cell-rosetting (SRBC-rosetting) for T-cell removal, and plastic adherence for 2 hours at 37°C to remove other cells and obtain monocytes. The obtained monocytes were cultured for five days in the presence of GM-CSF and IL-4 to differentiate them into immature DCs (mainly characterized by a CD1a⁺/CD83⁻phenotype). A further incubation of two days in the presence of a cocktail of GM-CSF, IL-4, PGE2, IL-1ß, IL-6 and TNF-α resulted in the development of mature DCs (mainly characterized by a CD1a⁺/CD83⁺ phenotype) (Jonuleit *et al.,* 1997)(See Figure 1).

### Example 2

### Selection of phage antibodies carrying single chain Fv fragments on dendritic cells by cell sorting

After five and seven days of culture, about 1.5*10⁷ immature and mature monocyte derived DCs, respectively, were obtained from the tissue culture flasks and mixed with 0.5 ml of a semi-synthetic phage scFv antibody display library. This library of human single chain antibody fragments was constructed as described in de Kruif *et al.* (1995b). In short, the library consisted of a combination of 49 germline VH genes fused with ∼10⁸ synthetic heavy chain CDR3 regions and 7 light chains. The CDR3 regions varied in length between 6 and 15 amino_acid. The light chains were encoded by members of the Vκ1 to Vκ4 and Vλ1 to Vλ3 families. The final library comprised about 4x10⁸ individual clones.

0.5 ml of the above library containing approximately 10¹³ phage particles per ml was blocked at 4°C for 15 minutes in RPMI-medium containing 20% fetal calf serum and 5 mM EDTA. After that, about 1.5*10⁷ immature DCs or about 7.3*10⁶ mature DCs were added to the blocked library in the presence of about 7.5*10⁷ fresh peripheral blood leucocytes (PBL) acting as subtractor cells in a final volume was 5 ml. This mixture was slowly rotated overnight at 4°C. The next day, the cells were washed twice with 50 ml ice-cold RPMI-medium containing 20% fetal calf serum and 5 mM EDTA and were stained with 100 µl PE-conjugated anti-CD83 antibody (BD PharMingen) and 100 µl FITC-conjugated anti-CD1a antibody (BD PharMingen) to visualize the dendritic cell populations on a flow cytometer. These antibodies did not recognize PBL and therefore a pure population of DC was obtained. After an incubation of 1 hour on ice, the cells were washed once with 15 ml of the above medium and resuspended in 4 to 6 ml of the medium. Just before sorting, cells were separated with a cell strainer (BD). Cell sorting was performed on a FACStar^{PLUS} fluorescence activated cell sorter with the gates set around the CD83⁻CD1a⁺ immature or CD83⁺CD1a⁺ mature DCs. For each cell population, 10⁴ to 10⁵ cells with phages still attached were sorted.

To elute specifically bound phages, the cells were pelleted for 10 minutes at 1200 rpm. Thereafter, they were resuspended in a volume of 100 µl of the obtained supernatant and brought in a 50 ml tube containing 150 µl of 76 mM sodium citrate. After 10 minutes at room temperature, the pH was neutralized by adding 200 µl of 1 M Tris-HCl buffer (pH 7.4). Finally, 1 ml of 2TY medium and 1 ml of log phase *Escherichia coli* XL-1 blue were added. Infection was allowed to proceed for 30 minutes at 37°C. Next, the bacteria were centrifuged at 2800 rpm for 10 minutes, suspended in 0.5 ml of 2TY, and plated on agar plates containing 25 µg/ml tetracycline, 100 µg/ml ampicillin, and 5% glucose. After overnight culture at 37°C, the plates were scraped and bacteria were frozen in stock vials or used to prepare the next restricted library, using VCSM13 helper phage. After the first round of selection 7.6*10⁴ and 2.5*10⁴ colonies were obtained from the selection with mature and immature DCs, respectively.

The selection round described above was repeated two more times, with the proviso that after both the second and the third round bacteria were seeded in the proper dilution allowing isolation of single colonies. The twenty single clones obtained were individually grown and rescued with VCSM13 helper phage to prepare phage antibody solutions. Next, each of the twenty clones obtained was tested for specific binding to the immature and mature DC population (see upper panels of Figure 2 for binding of the representative single chain phage antibody called SC02-113 to immature and mature DCs) .

### Example 3

### Identification of CD1a as the antigen recognised by the selected scFv phage antibodies

In total, three selection rounds were performed and after both the second and third round, phage antibodies were tested for specific binding on a variety of cell-types, *i.e.* cultured DCs, PBLs, tonsil and synovial fluid cell suspensions, several cell lines including K562, U266, IM9, U937, THP, CEM, Fravel, HL60, Raji, RPMI8226, HepgII, HELA, HT29 and Jurkatt, a C1R cell line transfected with the molecule CD1a, and A431 cell lines transfected with the molecules CD80, CD83, and CD86. For staining of cells, 100 µl of the phage antibodies were blocked by adding 50 µl of PBS containing 4% milk powder for 15 minutes at room temperature. Next, 5x10⁵ cells in 50 µl of PBS containing 1% BSA were added to the blocked phage antibodies and the mixture obtained was incubated on ice for 1 hour. After that, the obtained mixture was washed three times with 200 µl PBS containing 1% BSA. To detect cell-bound phage antibodies, the cells were incubated with 20 µl of a 1:800 dilution of sheep anti-M13 polyclonal antibody for 20 minutes on ice. Thereafter, the cells were washed with PBS containing 1% BSA and incubated with 20 µl of a 1:700 dilution of PE-conjugated donkey anti-sheep polyclonal antibody for 20 minutes on ice.

To visualize DCs the cells were washed again with PBS containing 1% BSA, stained with 10 µl 1:10 diluted FITC-conjugated anti-CD1a antibody (BD PharMingen) and suspended in 200 µl of PBS containing 1% BSA. Flow cytometric analyses were performed using a FACScan (Becton Dickinson).

The twenty scFv phage antibodies derived from the selections described above bound exclusively to the cell line transfected with CD1a (see lower right panel of Figure 2 for binding of the representative scFv phage antibody called SC02-113 to the CD1a transfected cell line). The C1R cells transfected with vector containing a CD1a insert were clearly positive, whereas the C1R cell line transfected with vector without CD1a insert (mock-transfected) was negative after staining with the phage antibodies (see lower left panel of Figure 2 for binding of the representative scFv phage antibody called SC02-113 to the mock-transfected cell line). The A431 cell lines transfected with CD80, CD83 and CD86 were negative after staining with the phage antibodies (data not shown).

### Example 4

### Characterization of the human CDla specific scFv's

From the twenty phage antibody (scFv) clones that bound exclusively to the cell line transfected with human CD1a, plasmid DNA was obtained and nucleotide sequences were determined according to standard techniques. In short, the nucleotide sequence of the VH and VL of all twenty clones was determined using primer M13REV (5'-AACAGCTATGACCATG (SEQ ID NO:23)) and fdSeq (5'-GAATTTTCTGTATGAGG (SEQ ID NO:24)) in a sequence reaction with the Taq sequencing kit with the following cycling protocol (25 cycles): 94°C for 10 seconds (denaturing), 50°C for 5 seconds (annealing) and 60°C for 4 minutes (extension). Precipitated DNA was dissolved in sample buffer, run and analyzed on an ABIPRISM automated fluorescent sequencer. The obtained nucleotide sequences were compared to the VBASE database which is well known to the average skilled person the the art of antibodies and the gene family of each individual chain was determined. The twenty clones turned out to contain six different scFv's. These were named SC02-113, SC02-114, SC02-115, SC02-116, SC02-117 and SC02-118 respectively (see Table 1). The nucleotide sequences of the scFv's called SC02-113, SC02-114, SC02-115, SC02-116, SC02-117 and SC02-118 are shown in SEQ ID NO:25, SEQ ID NO:27, SEQ ID NO:29, SEQ ID NO:31, SEQ ID NO:33 and SEQ ID NO:35, respectively (see Table 1). The amino acid sequences of the scFv's called SC02-113, SC02-114, SC02-115, SC02-116, SC02-117 and SC02-118 are shown in SEQ ID NO:26, SEQ ID NO:28, SEQ ID NO:30, SEQ ID NO:32, SEQ ID NO:34 and SEQ ID NO:36, respectively (see Table 1). The VH and VL gene identity and heavy chain CDR3 sequences of the human CD1a specific scFv's are also depicted in Table 1.

### Example 5

### Construction of fully human immunoglobulin molecules (human monoclonal anti-CDla antibodies) from the selected anti-CD1a single chain Fv's

Heavy and light chain variable regions of the scFv's called SC02-113, SC02-114, SC02-115, SC02-116, SC02-117 and SC02-118 were PCR-amplified using oligonucleotides to append restriction sites and/or sequences for expression in the IgG expression vectors pSyn-C03-HCγ1 (see SEQ ID No:37) pSyn-C05-Cκ (see SEQ ID No:38) and pSyn-C04-Cλ (see SEQ ID No:39). The heavy chain variable regions of the scFv's called SC02-113, SC02-114, SC02-115, SC02-116, SC02-117 and SC02-118 were cloned into the vector pSyn-C03-HCγ1; the shared light chain variable region of the scFv's called SC02-113, SC02-114, SC02-116, SC02-117 and SC02-118 was cloned into the vector pSyn-C05-Cκ; the light chain variable region of the scFv called SC02-115 was cloned into the vector pSyn-C04-Cλ. The VL gene shared between scFv's SC02-113, SC02-114, SC02 -116, SC02-117 and SC02-118 was first amplified using oligonucleotides 5K-I (SEQ ID NO:40) and sy3K-C (SEQ ID NO:41) (see below) and the PCR products cloned into vector pSyn-C05-Cκ, The VL gene for scFv SC02-115 was first amplified using oligonucleotides sy5L-A (SEQ ID NO:42) and 3L-B (SEQ ID NO:43) (see below) and the PCR product cloned into vector pSyn-C04-Cλ. Nucleotide sequences for all constructs were verified according to standard techniques known to the skilled artisan.

VH genes were first amplified using the following oligonucleotide sets: SC02-113, 5H-Fshort (SEQ ID NO:44) and sy3H-A (SEQ ID NO:45); SC02-114, SC02-116 and SC02-117, 5H-B (SEQ ID NO:46) and sy3H-A (SEQ ID NO:45); SC02-118, 5H-B**65 (SEQ ID NO:47) and sy3H-A (SEQ ID NO:45); SC02-115; First 5H-A (SEQ ID NO:48) and 115int68 (SEQ ID NO:49), and then the PCR product obtained was re-amplified with the primers 5H-A (SEQ ID NO:48) and sy3H-A (SEQ ID NO:45). Thereafter, the PCR products were cloned into vector pSyn-C03-HCγ1 and nucleotide sequences were verified according to standard techniques known to the skilled person in the art.
5H-B**65 5H-B
acctgtcttgaattctccatggccgaggtgcagctggtggagtctg
115int68 5H-A
acctgtcttgaattctccatggcccaggtgcagctggtgcagtctgg
5H-Fshort
acctgtcttgaattctccatggcccaggtgcagctgcaggagtccggcc
sy3H-A gcccttggtgctagcgctggagacggtcaccagggtgccctggcccc 5K-I
acctgtctcgagttttccatggctgacatccagatgacccagtctccatcctcc
sy3K-C
gggaccaaggtggagatcaaacggaccgtggccgcccccagc
sy5L-A
acctgtctcgagttttccatggcttcctccgagctgacccaggaccctgctg
3L-B
ttttccttagcggccgcgactcacctaggacggtcagcttggtc

The resulting expression constructs pgG102-113C03, pgG102-114C03, pgG102-115C03, pgGl02-116C03 , pgG102-117C03 and pgG102-118C03 (the deposits of which having accession numbers 03102801, 03102802, 03102803, 03102804, 03102805 and 03102806) encoding the anti-CD1a human IgG1 heavy chains were transiently expressed in combination with the pSyn-CO5-VkI construct (the deposit of which has accession number 03102807), except for construct pgG102-115C03 which was transfected with the pSyn-C04-V13 construct (the deposit of which has accession number 03102808), encoding the light chains in 293T cells and supernatants containing IgG1 antibodies were obtained. The nucleotide sequences of the heavy chains of the antibodies called 02-113, 02-114, 02-115, 02-116, 02-117 and 02-118 (the antibodies are herein also called CR2113, CR2114, CR2115, CR2116, CR2117 and CR2118, respectively) are shown in SEQ ID NOs 50, 52, 54, 56, 58 and 60, respectively. The amino acid sequences of the heavy chains of the antibodies called 02-113, 02-114, 02-115, 02-116, 02-117 and 02-118 (the antibodies are herein also called CR2113, CR2114, CR2115, CR2116, CR2117 and CR2118, respectively) are shown in SEQ ID Nos 51, 53, 55, 57, 59 and 61, respectively.

The nucleotide sequences of the light chain of antibodies 02-113, 02-114, 02-116, 02-117 and 02-118 is shown in SEQ ID NO:62 and of antibody 02-115 in SEQ ID NO:64. The amino acid sequences of the light chain of antibodies 02-113, 02-114, 02-116, 02-117 and 02-118 is shown in SEQ ID NO:63 and of antibody 02-115 in SEQ ID NO:65. Subsequently, the antibodies were purified over protein-A columns and size-exclusion columns using standard purification methods used generally for immunoglobulins (see for instance WO 00/63403).

The human anti-CD1a IgG1 antibodies were validated for their ability to bind to CD1a transfected cell lines essentially as described for scFv's (see Example 3). The purified anti-CD1a antibodies were diluted to various concentrations (depicted in the figures) and detected with mouse anti-human IgG_{κ} labelled with FITC or FITC-labeled streptavidin in the case of biotinylated human anti-CD1a antibodies. In addition to the cell lines C1R-CD1a and C1R mock transfected described in Example 3, the cell lines C1R-CD1b, C1R-CD1c, C1R-CD1d and B16-CD1a and B16 mock transfected were tested. Each staining was repeated three times and compared to the negative control irrelevant isotype control antibody called CR2027 using the Kolmogorov - Smirnov test. To calculate K-S statistics, an overlay of histograms of isotype control antibody and anti-CD1a antibody was made. The log shift between the peaks of the histograms was determined and used to calculate the D-value. The greater the log shift of the anti-CD1a antibody response peak from the isotype control antibody response peak, the larger the D-value.

After conversion to full IgG all six antibodies bound to the B16-CDla transfectant at a concentration of 10 µg/ml, however only four of the six, i.e. the antibodies called CR2113, CR2114, CR2117, CR2118 bound to the C1R-CD1a transfectant (see Figure 3, figures on the left side). None of the antibodies bound significantly to the C1R or B16 mock transfected (see Figure 3, figures on the right side).

No specific staining of any of the six human anti-CD1a monoclonal antibodies was detected on the C1R-CD1b or C1R-CD1d transfectants (see Figure 4). Slight, but significant, staining (p<0.05) was detected for the two antibodies CR2114 and CR2117 on C1R-CD1c transfectants (see Figure 4). The results demonstrate the binding specificity of the antibodies for human CDla, even among the highly conserved human CD1 family of genes. In addition, staining was also carried out on freshly prepared mouse thymocytes, which express murine CD1a and CD1d at low levels. No specific staining for any of the six human anti-CD1a monoclonal antibodies was observed demonstrating species specificity of the antibodies (see Table 2).

Binding curves were generated for the antibodies CR2113, CR2114, CR2117 and CR2118 by measuring binding on B16-CD1a transfected cells with FACS as above using the concentrations of antibody ranging from 0.01 to 10 µg/ml. Both unlabelled and biotinylated antibodies were used and the pattern of staining was indistinguishable between the two forms (see Figures 5A-D). CR2113 showed saturation of binding at 1 µg/ml, CR2114 was not saturated at 10 µg/ml and CR2117 and CR2118 stained the transfectant less intensely compared to CR2113 and CR2114.

Competition experiments were performed to determine whether the antibodies recognize different epitopes. B16 mouse melanoma cells expressing human CD1a were stained with unlabelled CR2113, CR2114, CR2117, CR2118 or the negative control CR2027 at different concentrations (0.1, 0.3, 1, 3, 10, 30, 50, 100 µg/ml) for 30 minutes and then stained with one of the above antibodies labelled with biotin as described above for 5 minutes at a concentration of 2.5 µg/ml. Detection was by FITC-labelled streptavidin incubated for 20 minutes. Furthermore, staining was performed as described above (see Example 3). Each staining was done three successive times and the D-value calculated with K-S statistics (see above). The staining of unlabeled control antibody CR2027 is shown by the dotted line staining using the test antibody is depicted with a solid line. CR2113 was able to compete with itself in a dose dependent manner, however none of the other antibodies could compete with biotinylated CR2113 for binding (see Figure 6A).

This is likely due to its superior binding affinity for human CD1a compared to the other antibodies. In contrast, unlabelled CR2113 was able to compete for binding of CR2114 (see Figure 6B), CR2117 (see Figure 6C) and CR2118 (see Figure 6D) to B16-CD1a transfectants in a concentration dependent manner. Neither CR2117 or CR2118 was able to compete with CR2114 for binding to CD1a. This could be due to sub-optimal affinity.

In conclusion all four antibodies tested appear to share overlapping epitopes, although in the case of CR2113 and CR2114 these are unlikely to be identical due to the differences in specificity seen in Figure 3.

### Example 6

### Immunohistochemistry with human monoclonal anti-CD1a antibodies

The human monoclonal anti-CD1a antibodies are analyzed for their ability to bind to normal tissues by immunohistochemistry. For this purpose, frozen sections of the following normal tissues: adrenal gland; bladder; brain (cerebellum and cerebrum); blood vessels (aorta and coronary artery); fallopian tube; oesophagus; stomach (antrum and body); duodenum; ileum; colon; heart; kidney; liver; lung; lymphnode; ovary; pancreas; parathyroid; peripheral nerve; pituitary gland; placenta; prostate; salivary gland; skin; spinal cord; spleen; striated muscle; testis; tonsil; thyroid; ureter and uterus (cervix and endometrium) are cut, mounted on glass slides and are dried at room temperature. The sections are blocked for endogenous peroxidase with 50 mM sodium azide containing 0.03% H₂0₂ for 20 minutes, followed by blocking for endogenous biotin according to the provided protocol (X0590, Dako). Subsequently, the sections are blocked with PBS containing 4% BSA and 10% normal human serum prior to incubation with the biotinylated anti-human CD1a human IgGs for 60 minutes at room temperature. To detect bound IgG molecules the sections are incubated with streptavidin coupled-horse radish peroxidase (Dako) followed by incubation with diaminobenzidine (Sigma), resulting in a local deposition of brown crystals. The sections are counterstained with hematoxilin to visualizenucleated cells within the sections. Prior to analysis the sections are dehydrated and the slides are sealed with eukitt (BDH).

### Example 7

### Flow cytometric analysis of expression of CD1a molecules on fresh tumor samples and tumor cell lines with human monoclonal anti-CD1a antibodies

The human anti-CD1a IgG1 antibody panel is used to evaluate CD1a expression on the following fresh tumor samples; acute myeloid leukemia (AML), acute non-lymphocytic leukemia (ANLL), chronic myelogenous leukemia in blast crisis (CML-BC), large granular lymphocytic leukemia (LGLL), B cell chronic lymphocytic leukemia (B-CLL), T-cell acute lymphoblastic leukemia (T-ALL) and cell line SUP-T1. For this purpose 2*10⁵ mononuclear cells prepared by ficoll-paque gradient separation (for fresh samples) or from culture according to suppliers instructions (for cell lines) are stained with the human monoclonal anti-CD1a IgG1 antibodies of the invention at concentrations ranging from 0 to 50 µg/ml at 4°C. Binding of the antibodies called 02-113, 02-114, 02-115, 02-116, 02-117 and 02-118 is visualized using biotinylated goat-anti-human IgG (Fc specific, Caltag) followed by streptavidinphycoerythrin (Caltag). The stained cells are analyzed by flow cytometry. Moderate to high levels of staining are expected in subpoputions of the tumour cells following the phenotype established with anti-CD1a mouse monoclonals. Moderate to high levels of CD1a expression is expected to be necessary for the human anti-CD1a monoclonal antibodies to be useful as therapeutic molecules.

### Example 8

### Internalisation studies with human monoclonal anti -CD1a antibodies

Internalization assays with CD1a expressing cell lines such as Jurkatt cells are performed in order to determine the internalising capacity of the human monoclonal anti-human CD1a IgG1 antibodies. The anti-human CD1a IgG1 antibodies are stained with Oregon Green-480-SE (Molecular Probes) labeling dye as follows. 0.1 mg of dye is dissolved in 10 µl of DMSO, added to 0.4 mg of antibody in a final volume of 0.4 ml PBS, and incubated at room temperature for 1 hour. Subsequently, this mixture is loaded onto a Sephadex G25 column equilibrated in PBS. The labeled antibody is eluted with PBS and the coloured fraction is collected. Aliquots of 5*10⁵ Jurkatt cells are loaded with the appropriate antibody at a saturating concentration on ice for 30 minutes. Unbound antibody is removed by three washes with ice-cold RPMI 1640 medium containing 10% FBS medium. Subsequently, cells are resuspended in 50 µl of the medium and incubated for 1 hour at either 4°C (no internalisation) or 37°C to allow internalisation of the antibodies. Following three washes with ice-cold PBS, cell surface-bound antibodies are stripped off the cells with 2.5 mg/ml subtilisin for 1 hour at 4°C. Cells are washed again with ice-cold PBS-L% BSA and samples are analyzed by flow cytometry. With cells that are incubated at 4°C (a temperature at which no internalization occurs) the cell-bound IgGs can be stripped off the cells as shown by loss of fluorescence. On the other hand, when cells are incubated at 37°C to allow internalization of antibodies, the cells remain fluorescent after eliminating antibodies bound to CD1a molecules at the cell surface by stripping the cells using subtilisin. This indicates that the anti-CD1 antibodies do internalize into cells and have become resistant to protease treatment. In contrast, a negative control antibody, anti-CD20 (which does not internalise, see Ghetie et al. (2001)), can be stripped of the cells at both temperatures indicating that the antibody binds to its target, but does not internalise into the cell.

On the one hand, internalisation of anti-CD1a antibodies will allow better efficacy of immunoconjugates containing certain toxin molecules coupled to the internalizing anti-CD1a antibodies, if such toxin molecules need to cross the cellular membrane for efficient functioning/activity. On the other hand, anti-CD1a antibodies not capable of internalizing might be useful in radioconjugates. Such conjugates do not need to cross the cellular membrane to exert a cytotoxic effect. In addition, antibodies bound to the surface of target cells can recruit cytotoxic leukocytes such as natural killer cells (ADCC) or direct the deposition of complement (CDC). Rapid internalisation of CD1a upon ligation of the antibodies could interfere with these processes.

### Example 9

### Induction of antibody dependent cellular cytotoxicity (ADCC) and complement dependent cytotoxicity (CDC) in tumor cells using human anti-CD1A antibodies

To determine the ability of the human monoclonal human anti-CD1A antibodies to induce antibody dependent cellular cytotoxicity (ADCC) and complement dependent cytotoxicity (CDC) standard ⁵¹Cr release assays are performed. For ADCC experiments, HL60 or U937 tumor target cells are labeled with 100 µCi of ⁵¹Cr (Amersham, Buckinghamshire, UK) for 1 hour at 37°C. After extensive washing, the target cells are plated in U-bottom microtiter plates at a concentration of 5*10³ cells/well. Isolated human peripheral blood mononuclear cells are subsequently added at several effector to target ratios ranging from 80:1 to 10:1. The cell mixtures are incubated in triplicate at 37°C in the presence of various concentrations of the human monoclonal anti-CD1a antibodies in a final volume of 150 µl. After 4 hours of incubation, part of the supernatant is harvested and its ⁵¹Cr content is measured. The percentage of specific lysis is calculated using the formula: % specific lysis = ([experimental cpm - basal cpm] / [maximal cpm - basal cpm]) x 100%. Maximal lysis is determined by lysing the target cells with 1% Triton X-100 while basal release is measured by incubating the target cells with medium alone.

For CDC experiments the same procedure is followed except that the effector cells are replaced by 50 µl human serum as a complement source.

If the specific lysis of target cells due to ADCC and/or CDC is significantly greater when the cells are incubated with the anti-CD1a antibodies than when the cells are incubated with control antibodies, there is a greater likelihood that the anti-CD1a antibodies will be effective as naked antibodies in an *in vivo* setting.

### Example 10

### In vivo anti-tumor efficacy of human anti-CD1a antibodies in a CD1a-transfected mouse tumor model

To analyse the potential anti-tumor efficacy of the human anti-CD1a antibodies *in vivo,* animal experiments employing a human CD1a-transfected mouse B16 tumor model are performed. This tumor has been described extensively in the tumor-immunology literature (see Fidler *et al*. (1976)) and grows progressively in for instance syngeneic C57B1/6 mice. To test the effect of anti-CD1a treatment, C57B1/6, C57B1/6 nude, SCID or NOD-SCID mice are inoculated subcutaneously at day 0 with 1*10⁶ B16 tumor cells. Each mouse receives B16 cells transfected with CD1a cDNA in the sense orientation (*i.e.* normally expressed) in one flank. In the opposite flank mice receive B16 cells transfected with CD1a cDNA in the antisense orientation (*i.e*. not expressed) to act as a negative control. In some experiments CD1a-B16 cells are cotransfected with luciferase for real time detection of tumor growth. The animals are given a dose of 240 µg human anti-CD1a antibody intraperitoneally (i.p.) at day 1, followed by three more doses of 80 µg antibody i.p. at days 3, 6 and 9. The animals are then monitored for tumor growth for over 28 days, animals are sacrificed when tumor sizes exceed 2 cm³ or after ulceration of the tumor. Tumor size can be monitored by means of fluorescent imagery (in case of luciferase transfected B16 cells) or directly by callipers. A potential anti-tumor effect of the antibodies is analysed by comparing the tumor outgrowth in animals receiving antibody treatment as compared to a control groups that are treated with the saline vehicle or with a human IgG1 control antibody.

All anti-CD1a monoclonal antibodies capable of specifically binding to CD1a might be tested in the *in vivo* model. Effectiveness in the ADCC and/or CDC assay is not a criterium for inclusion in the *in vivo* study. Significant inhibition of tumor growth compared to the isotype antibody controls is considered to be a strong indication that the anti-CD1a monoclonal antibodies are efficacious in inhibiting growth of CDla-expressing tumours in humans.

### Example 11

### BIACORE analysis of the anti-CD1a antibodies

The BIACORE 3000, which applies the technique of real-time surface plasmon resonance, is used to perform affinity measurements with the anti-CD1a antibodies prepared as described above and a murine anti-CD1a control antibody. First, the ligand, *i.e*. the CD1a protein (Abnova corporation), is immobilised as a ligand on a research grade CM5 4-flow channel (Fc) sensor chip using amine coupling. Briefly, the dextran matrix of CM5 chip (BIACORE) is activated with a mixture of N-hydroxysuccinimide (NHS) and N-ethyl-N'-(dimethylaminopropyl)carbodiimide (EDC). Next, the CD1A protein (preferably 30 µg/ml) is coupled to the activated carboxyl groups on the matrix. After coupling, 1 M et-hanolamine-HCL (pH 8.5) is added to deactivate remaining active carboxyl groups. One of the four channels is activated and deactivated without CD1A protein coupling and serves as a control flow channel. Thereafter, kinetic analyses are performed. For that purpose association rate (Ka), dissociation rate (Kd) and affinity (KD) of the anti-CD1A antibodies are investigated. A concentration series between 0.01 to 1000 nM is applied at 25°C (dilution factor 2, dilution buffer HBS-EP). One of the four channels is used as reference, flow cell and injection of HBS-EP buffer serves as control injection. Fifty µl of the respective antibodies is injected with a constant flow rate of 20 µl/min. At the end of the injection, HBS-EP buffer is applied for 750 seconds followed by regeneration of the CM5 chip with pulses of regeneration buffer (for instance glycine, pH 2) according to the manufacturer instructions. The experiment is repeated 3 times to determine the assay variation. Then, BIACORE evaluation software is used to fit the association and dissociation curves of the concentrations in the concentration series. Based on these fits affinity is determined. To minimize possible avidity effects of the bivalent antibodies, the following 4 important parameters are included during evaluation; 1) concentration range of 0.1 - 10 X KD, 2) low standard error values of Ka and Kd rates, 3) low residual Chi² values, and 4) good precision (preferably <20%). Anti-CD1a antibodies bind preferentially with high affinity in the 0.1-50 nM range.

**Table 1: Nucleotide and amino acid sequences, heavy chain CDR3 amino acid sequence and gene identity of the VH and VL genes of the CD1a specific scFv's.**

| Name of scFv | Amino acid sequence of HCDR3 | SEQ ID NO of nucleotide sequence of scFv | SEQ ID NO of amino acid sequence of scFv | VH family | VL family |
|---|---|---|---|---|---|
| SC02-113 | APYMMYFDS (SEQ ID NO:1) | SEQ ID NO:25 | SEQ ID NO:26 | VH4:DP-66 | Vκ1:DPK-9 |
| SC02-114 | ETWWQSFDY (SEQ ID NO:2) | SEQ ID NO:27 | SEQ ID NO:28 | VH3:DP-51 | Vκ1:DPK-9 |
| SC02-115 | SQMPSYFDY (SEQ ID NO:3) | SEQ ID NO:29 | SEQ ID NO:30 | VH1:DP-14 | Vλ3:DPL-16 |
| SC02-116 | DALWLAFDY (SEQ ID NO:4) | SEQ ID NO:31 | SEQ ID NO:32 | VH3:DP-47 | Vκ1:DPK-9 |
| SC02-117 | STPWFSFDY (SEQ ID NO:5) | SEQ ID NO:33 | SEQ ID NO:34 | VH3:DP-48 | Vκ1:DPK-9 |
| SC02-118 | SAWWLSFDS (SEQ ID NO:6) | SEQ ID NO:35 | SEQ ID NO:36 | VH3:DP-48 | Vκ1:DPK-9 |

**Table 2: Binding of the human anti-CD1a monoclonal antibodies to mouse thymocytes measured by flow cytometry.**

| Antibody | Mouse Thymocytes (D-value) |
|---|---|
| CR2113 | 0 |
| CR2114 | 0 |
| CR2115 | 0 |
| CR2116 | 0 |
| CR2117 | 0 |
| CR2118 | 0 |
| Positive control (mouse anti-human CD1a-FITC) | 0.03 |
| Negative control (anti-GBSIII antibody) | 0 |
| Negative control (mouse IgG; k-FITC) | 0 |

### REFERENCES

Amiot M., Bernard A., Raynal B., Knapp W., Deschildre C. and Boumsell L. (1986), J. Immunol. 136:1752-1757.
Boel E., Verlaan S., Poppelier M.J., Westerdaal N.A., Van Strijp J.A. and Logtenberg T. (2000), J. Immunol. Methods 239:153-166.
Burton D.R. and Barbas C.F. (1994), Adv. Immunol. 57:191-280.
De Kruif J., Terstappen L., Boel E. and Logtenberg T. (1995a), Proc. Natl. Acad. Sci. USA 92:3938-3942.
De Kruif J., Boel E. and Logtenberg T. (1995b), J. Mol. Biol. 248:97-105.
Fidler I.J., Gersten D.M. and Budmen M. B. (1976), Cancer Research 36:3610-3165.
Furue M., Nindl M., Kawabe K., Nakamura K., Ishibashi Y. and Sagawa K. (1992), J. Am. Acad. Dermatol. 27:419-426.
Ghetie M.A., Bright H. and Vitetta E.S. (2001), Blood 97:1392-1398.
Huls G., Heijnen I.J., Cuomo E., van der Linden J., Boel E., van de Winkel J. and Logtenberg T. (1999), Cancer Res. 59: 5778-5784.
Jonuleit H., Kuhn U., Muller G., Steinbrink K., Paragnik L., Schmitt E., Knop J., Enk A.H. (1997), Eur. J. Immunology 27: 3135-3142.
Kelly K.M., Beverly P.C., Chu A.C., Davenport V., Gordon I., Smith M. and Pritchard J. (1994), J. Pediatr. 125:717-722.
Merle-Beral H., Boumsell L., Michel A. and Debre P. (1989), Br. J. Haematol. 72:209-212.
Salomone M.C., Roisman F.R., Santiago J., Satz M.L. and Fainboim L. (1990a), Dis. Markers 8:265-274.
Salomone M.C., Roisman F.R., Santiago J., Satz M.L. and Fainboim L. (1990b), Dis. Markers 8:275-281.
Teunissen M.B. (1992), Histochem. J. 24:697-716.
Van Kroonenburgh M.J. and Pauwels E.K. (1988), Nucl. Med. Commun. 9:919-930.

## Claims

1. A human monoclonal antibody capable of specifically binding to human CD1a, **characterized in that** the human monoclonal antibody comprises a heavy chain variable region comprising the amino acid sequence of SEQ ID NO: 8 and a light chain variable region comprising SEQ ID NO: 20.

2. A human monoclonal antibody according to claim 1, **characterized in that** the antibody is an IgG1.

3. A humain monoclonal antibody according to any of the claims 1 - 2, **characterized in that** the antibody has at least an activity selected from the group consisting of antibody-dependent cellular cytotoxicity, complement-dependent cytotoxicity and internalization.

4. An immunoconjugate comprising a human monoclonal antibody according to any of the claims 1 - 3, the immunoconjugate further comprising at least one tag.

5. An immunoconjugate according to claim 4, **characterized in that** the tag is selected from the group consisting of a toxic substance, a radioactive substance, a liposome, an enzyme and combinations thereof.

6. A nucleic acid molecule encoding a human monoclonal antibody according to any of the claims 1 - 3.

7. A vector comprising at least one nucleic acid molecule according to claim 6.

8. A host cell comprising at least one vector according to claim 7.

9. A host cell according to claim 8, **characterized in that** the host cell is a human cell.

10. A method of producing a human monoclonal antibody according to any of the claims 1- 3, wherein the method comprises the steps of:
a) culturing a host cell according to claim 8 or 9 under conditions conducive to the expression of the monoclonal antibody, and optionally,
b) recovering the expressed human monoclonal antibody.

11. A composition comprising a human monoclonal antibody according to any of the claims 1- 3, or an immunoconjugate according to claim 4 or 5.

12. A pharmaceutical composition comprising a human monoclonal antibody according to any one of the claims 1 - 3, an immunoconjugate according to claim 4 or 5, or a composition according to claim 11, the pharmaceutical composition further comprising at least one pharmaceutically acceptable excipient.

13. A pharmaceutical composition according to claim 12 further comprising at least one other therapeutic agent.

14. A human monoclonal antibody according to any one of the claims 1 - 3, an immunoconjugate according to claim 4 or 5, a composition according to claim 11, or a pharmaceutical composition according to claim 12 or 13 for use as a medicament.

15. A method of detecting human CD1a, wherein the method comprises the steps of:
a. contacting a sample with a diagnostically effective amount of a human monoclonal antibody according to any of the claims 1- 3, or an immunoconjugate according to claim 4 or 5, and
b. determining whether the human monoclonal antibody or immunoconjugate specifically binds to a compound of the sample.

## Patentansprüche

1. Menschlicher monoklonaler Antikörper, der fähig ist, spezifisch an menschliches CD1a zu binden, **dadurch gekennzeichnet, dass** der menschliche monoklonale Antikörper eine variable Region der schweren Kette umfasst, die die Aminosäuresequenz der SEQ ID NO: 8 umfasst, sowie eine variable Region der leichten Kette, die die SEQ ID NO: 20 umfasst.

2. Menschlicher monoklonaler Antikörper nach Anspruch 1, **dadurch gekennzeichnet, dass** der Antikörper ein IgG1 ist.

3. Menschlicher monoklonaler Antikörper nach einem der Ansprüche 1 - 2, **dadurch gekennzeichnet, dass** der Antikörper mindestens eine Aktivität hat, die aus der Gruppe ausgewählt ist, die aus antikörperabhängiger zellulärer Zytotoxizität, komplementabhängiger Zytotoxizität und Internalisierung besteht.

4. Immunkonjugat, das einen menschlichen monoklonalen Antikörper nach einem der Ansprüche 1 - 3 umfasst, wobei das Immunkonjugat weiterhin mindestens eine Markierung umfasst.

5. Immunkonjugat nach Anspruch 4, **dadurch gekennzeichnet, dass** die Markierung aus der Gruppe ausgewählt ist, die aus einer toxischen Substanz, einer radioaktiven Substanz, einem Liposom, einem Enzym und Kombinationen davon besteht.

6. Nukleinsäuremolekül, das einen menschlichen monoklonalen Antikörper nach einem der Ansprüche 1- 3 kodiert.

7. Vektor, der mindestens ein Nukleinsäuremolekül nach Anspruch 6 umfasst.

8. Wirtszelle, die mindestens einen Vektor nach Anspruch 7 umfasst.

9. Wirtszelle nach Anspruch 8, **dadurch gekennzeichnet, dass** die Wirtszelle eine menschliche Zelle ist.

10. Verfahren zur Herstellung eines menschlichen monoklonalen Antikörpers nach einem der Ansprüche 1 - 3, wobei das Verfahren die Schritte umfasst:
a) Züchtung einer Wirtszelle nach Anspruch 8 oder 9 unter Bedingungen, die der Expression des monoklonalen Antikörpers förderlich sind, und gegebenenfalls
b) Gewinnung des exprimierten menschlichen monoklonalen Antikörpers.

11. Zusammensetzung, die einen menschlichen monoklonalen Antikörper nach einem der Ansprüche 1 - 3 oder ein Immunkonjugat nach Anspruch 4 oder 5 umfasst.

12. Pharmazeutische Zusammensetzung, die einen menschlichen monoklonalen Antikörper nach einem der Ansprüche 1 - 3, ein Immunkonjugat nach Anspruch 4 oder 5 oder eine Zusammensetzung nach Anspruch 11 umfasst, wobei die pharmazeutische Zusammensetzung weiterhin mindestens einen pharmazeutisch verträglichen Exzipienten enthält.

13. Pharmazeutische Zusammensetzung nach Anspruch 12, die weiterhin mindestens ein anderes therapeutisches Agenz umfasst.

14. Menschlicher monoklonaler Antikörper nach einem der Ansprüche 1 - 3, Immunkonjugat nach Anspruch 4 oder 5, Zusammensetzung nach Anspruch 11, oder pharmazeutische Zusammensetzung nach Anspruch 12 oder 13 zur Verwendung als Medikament.

15. Verfahren zum Nachweis von menschlichem CD1a, wobei das Verfahren die Schritte umfasst:
a. Inkontaktbringen einer Probe mit einer diagnostisch wirksamen Menge eines menschlichen monoklonalen Antikörpers nach einem der Ansprüche 1 - 3 oder eines Immunkonjugats nach Anspruch 4 oder 5; und
b. Bestimmen, ob der menschliche monoklonale Antikörper oder das Immunkonjugat spezifisch an einen Stoff aus der Probe bindet.

## Revendications

1. Anticorps monoclonal humain capable de se lier spécifiquement à la CD1a humaine, **caractérisé en ce que** l'anticorps monoclonal humain comprend une région variable de chaîne lourde comprenant la séquence d'acides aminés de SEQ ID NO: 8 et une région variable de chaîne légère comprenant SEQ ID NO: 20.

2. Anticorps monoclonal humain selon la revendication 1, **caractérisé en ce que** l'anticorps est une IgG1.

3. Anticorps monoclonal humain selon l'une quelconque des revendications 1 à 2, **caractérisé en ce que** l'anticorps a au moins une activité choisie dans le groupe constitué par une cytotoxicité cellulaire dépendante d'un anticorps, une cytotoxicité dépendante du complément et une internalisation.

4. Immunoconjugué comprenant un anticorps monoclonal humain selon l'une quelconque des revendications 1 à 3, l'immunoconjugué comprenant en outre au moins une étiquette.

5. Immunoconjugué selon la revendication 4, **caractérisé en ce que** l'étiquette est choisie dans le groupe constitué par une substance toxique, une substance radioactive, un liposome, une enzyme et les combinaisons de ceux-ci.

6. Molécule d'acide nucléique codant pour un anticorps monoclonal humain selon l'une quelconque des revendications 1 à 3.

7. Vecteur comprenant au moins une molécule d'acide nucléique selon la revendication 6.

8. Cellule hôte comprenant au moins un vecteur selon la revendication 7.

9. Cellule hôte selon la revendication 8, **caractérisée en ce que** la cellule hôte est une cellule humaine.

10. Procédé de production d'un anticorps monoclonal humain selon l'une quelconque des revendications 1 à 3, dans lequel le procédé comprend les étapes de :
a) culture d'une cellule hôte selon la revendication 8 ou 9 dans des conditions propices à l'expression de l'anticorps monoclonal, et éventuellement,
b) récupération de l'anticorps monoclonal humain exprimé.

11. Composition comprenant un anticorps monoclonal humain selon l'une quelconque des revendications 1 à 3, ou un immunoconjugué selon la revendication 4 ou 5.

12. Composition pharmaceutique comprenant un anticorps monoclonal humain selon l'une quelconque des revendications 1 à 3, un immunoconjugué selon la revendication 4 ou 5, ou une composition selon la revendication 11, la composition pharmaceutique comprenant en outre au moins un excipient pharmaceutiquement acceptable.

13. Composition pharmaceutique selon la revendication 12 comprenant en outre au moins un autre agent thérapeutique.

14. Anticorps monoclonal humain selon l'une quelconque des revendications 1 à 3, un immunoconjugué selon la revendication 4 ou 5, une composition selon la revendication 11, ou une composition pharmaceutique selon la revendication 12 ou 13 pour une utilisation en tant que médicament.

15. Méthode de détection de la CD1a humaine, dans laquelle la méthode comprend les étapes de :
a. mise en contact d'un échantillon avec une quantité efficace au plan diagnostic d'un anticorps monoclonal humain selon l'une quelconque des revendications 1 à 3, ou un immunoconjugué selon la revendication 4 ou 5, et
b. détermination si l'anticorps monoclonal humain ou l'immunoconjugué se lie spécifiquement à un composé de l'échantillon.
